# EUROPEAN PATENT SPECIFICATION

(11) **EP 3 645 551 B1**
(45) Date of publication and mention of the grant of the patent: **13.03.2024**
(21) Application number: 18749635.1
(22) Date of filing: 27.06.2018
(51) Int. Cl.: C07K 14/015, C12N 15/35, C12N 15/864, A61K 35/76, A61K 48/00, C12N 5/10, C07K 16/18, C07K 16/28

(54) **TROPISM-MODIFIED RECOMBINANT VIRAL VECTORS AND USES THEREOF FOR THE TARGETED INTRODUCTION OF GENETIC MATERIAL INTO HUMAN CELLS**
TROPISMUSMODIFIZIERTE REKOMBINANTE VIRALE VEKTOREN UND DEREN VERWENDUNGEN ZUR GEZIELTEN EINFUHR VON GENETISCHEM MATERIAL IN MENSCHLICHE ZELLEN
VECTEURS VIRAUX RECOMBINANTS AU TROPISME MODIFIÉ ET LEURS UTILISATIONS POUR L'INTRODUCTION CIBLÉE DE MATÉRIEL GÉNÉTIQUE DANS DES CELLULES HUMAINES

(30) Priority: 27.06.2017 US 201762525704 P
(43) Date of publication of application: 06.05.2020
(60) Divisional application: 24154743.9
(73) Proprietor: Regeneron Pharmaceuticals, Inc., Tarrytown, NY 10591 (US)
(72) Inventor: KYRATSOUS, Christos, Tarrytown, New York 10591 (US); MURPHY, Andrew J., Tarrytown, New York 10591 (US); WANG, Cheng, Beijing (CN); SABIN, Leah, Tarrytown, New York 10591 (US)
(74) Representative: J A Kemp LLP
(86) International application number: PCT/US2018/039874
(87) International publication number: WO 2019/006043

(56) References cited:
- EP-A2- 2 158 211
- WO-A2-00/26254
- GRIFMAN M ET AL: "Incorporation of tumor-targeting peptides into recombinant adeno-associated virus capsids", MOLECULAR THERAPY : THE JOURNAL OF THE AMERICAN SOCIETY OF GENE THERAPY, ACADEMIC PRESS ; NATURE PUBLISHING GROUP, US, vol. 3, no. 6, 1 June 2001 (2001-06-01), pages 964-975, XP002235294, ISSN: 1525-0016, DOI: 10.1006/MTHE.2001.0345
- C. L. BELL ET AL: "Identification of the Galactose Binding Domain of the Adeno-Associated Virus Serotype 9 Capsid", JOURNAL OF VIROLOGY, vol. 86, no. 13, 18 April 2012 (2012-04-18), pages 7326-7333, XP055165759, ISSN: 0022-538X, DOI: 10.1128/JVI.00448-12
- WICKHAM T J ET AL: "TARGETED ADENOVIRUS-MEDIATED GENE DELIVERY TO T CELLS VIA CD3", JOURNAL OF VIROLOGY, THE AMERICAN SOCIETY FOR MICROBIOLOGY, US, vol. 71, no. 10, 1 January 1997 (1997-01-01), pages 7663-7669, XP002935954, ISSN: 0022-538X
- MARTIN U RIED: "Adeno-Associated Virus Capsids Displaying Immunoglobulin-Binding Domains Permit Antibody-Mediated Vector Retargeting to Specific Cell Surface Receptors", JOURNAL OF VIROLOGY, vol. 76, 1 May 2002 (2002-05-01), pages 4559-4566, XP055507786,
- BARTLETT J ET AL: "Targeted adeno-associated virus vector transduction of nonpermissive cells mediated by a bispecific F(ab'gamma)2 antibody", NATURE BIOTECHNOLOGY, GALE GROUP INC, vol. 17, 1 February 1999 (1999-02-01), pages 181-186, XP002135910, ISSN: 1087-0156, DOI: 10.1038/6185
- ULRICH BRINKMANN ET AL: "The making of bispecific antibodies", MABS, vol. 9, no. 2, 10 January 2017 (2017-01-10), pages 182-212, XP055374463, US ISSN: 1942-0862, DOI: 10.1080/19420862.2016.1268307

## Description

The Sequence Listing written in file 10335WO01_ST25.txt is 88 kilobytes, was created on June 27, 2018.

### TECHNICAL FIELD

The disclosure herein relates generally to tropism modified recombinant adenovirus-associated virus (AAV) vectors, and compositions comprising the same, which are useful for the targeted introduction of genetic material into cells and/or tissues.

### BACKGROUND OF THE INVENTION

The delivery of genes into particular target cells has become one of the most important technologies in modern medicine for diagnosis and gene therapy of a variety of chronic and genetic diseases. So far, the progress in the clinical application of gene therapy has been limited by the lack of ideal gene delivery vehicles. In order to achieve therapeutic success, gene delivery vehicles must be capable of transducing target cells while avoiding transduction of the non-target cells. Specifically, when the native tropism of the virus does not meet the instant therapeutic needs, there is a need for recombinant viral vectors wherein the natural tropism is ablated or diminished and the desired tropism is successfully engineered. (Buchholz et al.).

In recent years, most progress in vector development has been achieved using naked viruses (e.g., viruses comprising a capsid formed by viral capsid proteins without an envelope (e.g., lipid bilayer)) such as adeno-associated viruses (AAVs) and adenoviruses (Ads), as well as enveloped viruses (e.g., viruses for which the capsid is surrounded by a lipid bilayer) such as retroviruses, lentiviruses, and herpes simplex virus. AAV based vectors have been the focus of much research, since AAVs are non-enveloped viruses that are only mildly immunogenic but capable of transducing a wide range of species and tissues *in vivo* with no evidence of toxicity.

AAVs are small, non-enveloped, single-stranded DNA viruses. The AAV genome is 4.7 kb and is characterized by two inverted terminal repeats (ITR) and two open reading frames which encode the Rep proteins and Cap proteins, respectively. The two ITRs are the only cis elements essential for AAV replication, packaging and integration. The Rep reading frame encodes four proteins of molecular weight 78 kD, 68 kD, 52 kD and 40 kD. These proteins function mainly in regulating AAV replication and integration of the AAV into a host cell's chromosomes. The Cap reading frame encodes three structural (capsid) viral proteins (VP) having molecular weights of 83-85 kD (VP1), 72-73 kD (VP2) and 61-62 kD (VP3). More than 80% of total proteins in AAV virion comprise VP3; in mature virions VP1, VP2 and VP3 are found at relative abundance of approximately 1:1:10. *In vitro,* the three proteins assemble spontaneously into virion-like structures, e.g., viral capsids. It appears, therefore, that viral capsid formation in infected cells proceeds independent of viral DNA synthesis (reviewed by Kotin et al. (1994) Hum. Gene Ther. 5:793).

Among all known AAV serotypes, AAV2 is perhaps the most well-characterized serotype, because its infectious clone was the first made. (Samulski et al. (1982) Proc. Natl. Acad. Sci. USA 79:2077-2081). Subsequently, the full sequences for AAV3A, AAV3B, AAV4 and AAV6 have also been determined. (Rutledge et al. (1998) J. Virol. 72:309-319; Chiorini et al. (1997) J. Virol. 71:6823-6833; S. Muramatsu et al. (1996) Virol. 221:208-217). Generally, all AAVs share more than 80% identity in nucleotide sequence.

AAV is a promising vector for human gene therapy since, unlike other viral vectors, AAVs have not been shown to be associated with any known human disease and are generally not considered pathogenic. (Muzyczka et al. (1992) Current Topics in Microbiology and Immunology 158:97-129). Moreover, AAV safely transduces postmitotic tissues with relatively low immunogenicity, and is capable of integrating into host chromosomes in a site-specific manner, and into tissue cultured cells in chromosome 19 if the Rep proteins are supplied in trans. (Kotin et al. (1990) Proc. Natl. Acad. Sci. USA 87:2211-2215; Samulski et al. (1991) EMBO J. 10(12):3941-3950; Balague et al. (1997) J. Virol. 71:3299-3306; Surosky et al. (1997) J. Virol. 71:7951-7959). The integrated genomes of AAV have been shown to allow long term gene expression in a number of tissues, including, muscle, liver, and brain (Fisher (1997) Nature Med. 3(3):306-312; Snyder et al. (1997) Nature Genetics 16:270-276; Xiao et al. (1997) Experimental Neurology 144:113-124; Xiao et al. (1996) J. Virol. 70(11):8098-8108).

A number of viruses, including AAVs, infect cells via a virus/ligand:cell/receptor interaction ultimately resulting in endocytosis of the virus by the infected cell. This ligand: receptor interaction is the focus of much of the research in viral vectors, e.g., may be manipulated to redirect a virus' natural tropism from a cell naturally permissive to infection by the wildtype virus to a target cell, e.g., via a receptor expressed by the target cell.

Theoretically, retargeting a vector toward any cell surface protein or marker should result in infection by a target cell as most cell surface receptors or markers are involved in pathways of endocytosis, either constitutive (e.g., for recycling) or ligand induced (e.g., receptormediated). These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. As such, platforms for retargeting viral vectors often aim to ablate the natural tropism of the viral vector and redirect the viral vector to a receptor or marker expressed solely or primarily by the target cell. Many of the advances in targeted gene therapy using viral vectors may be summarized as non-recombinatorial (non-genetic) or recombinatorial (genetic) modification of the viral vector, which result in the pseudotyping, expanding, and/or retargeting of the natural tropism of the viral vector. (Reviewed in Nicklin and Baker (2002) Curr. Gene Ther. 2:273-93; Verheiji and Rottier (2012) Advances Virol 2012:1-15).

Non-genetic approaches typically utilize an adaptor, which recognizes both a wildtype (non-modified) virus surface protein and a target cell. Soluble pseudo-receptors (for the wildtype virus), polymers such as polyethylene glycol, and antibodies or portions thereof, have been used as the virus binding domain of the adaptors, while natural peptide or vitamin ligands, and antibodies and portions thereof have been used for the cell binding domain of the adaptors described above. With this approach, retargeting of the viral vector to a target cell may be accomplished upon binding of the vector: adaptor complex to a protein expressed on the surface of the target cell, e.g., a cell surface protein.

Such approach has been used for AAV (Bartlett et al. (1999) Nat. Biotechnol. 74: 2777-2785), adenoviruses (Hemminki et al. (2001) Cancer Res. 61: 6377-81; van Beusechem et al. (2003) Gene Therapy 10:1982-1991; Einfeld, et al. (2001) J. Virol. 75:11284-91; Glasgow et al. (2009) PLOS One 4:e8355), herpesviruses (Nakano et al. (2005) Mol. Ther. 11:617-24), and paramyxoviruses (Bian et al. (2005) Cancer Gene Ther. 12:295-303; Bian et al. (2005) Int. J. Oncol. 29:1359-69), Coronaviruses (Haijema et al. (2003) J. Virol. 77:4528-43]8; Wurdinger et al. (2005) Gene Therapy 12:1394-1404)*.*

A more popular approach has been the recombinatorial genetic modification of viral capsid proteins, and thus, the surface of the viral capsid. In indirect recombinatorial approaches, a viral capsid is modified with a heterologous "scaffold", which then links to an adaptor. The adaptor binds to the scaffold and the target cell. (Arnold et al. (2006) Mol. Ther. 5:125-132; Ponnazhagen et al. (2002) J. Virol. 76:12900-907; *see also* WO 97/05266). Scaffolds such as (1) Fc binding molecules (e.g., Fc receptors, Protein A, etc.), which bind to the Fc of antibody adaptors, (2) (strept)avidin, which binds to biotinylated adaptors, (3) biotin, which binds to adaptors fused with (strept)avidin, and (4) protein:protein binding pairs that form isometric peptide bonds such as SpyCatcher, which binds a SpyTagged adaptor, have been incorporated into Ad (Pereboeva et al. (2007) Gene Therapy 14: 627-637; Park et al. (2008) Biochemical and Biophysical Research Communications 366: 769-774; Henning et al. (2002) Human Gene Therapy 13:1427-1439; Banerjee et al. (2011) Bioorganic and Medicinal Chemistry Letters 21:4985-4988), AAV (Gigout et al. (2005) Molecular Therapy 11:856-865; Stachler et al. (2008) Molecular Therapy 16:1467-1473), and togavirus (Quetglas et al. (2010) Virus Research 153:179-196; Ohno et al. (1997) Nature Biotechnology 15:763-767; Klimstra et al. (2005) Virology 338:9-21).

In a direct recombinatorial targeting approach, a targeting ligand is directly inserted into, or coupled to, a viral capsid, i.e., protein viral capsids are modified to express a heterologous targeting ligand. The ligand than redirects, e.g., binds, a receptor or marker preferentially or exclusively expressed on a target cell. (Stachler et al. (2006) Gene Ther. 13:926-931; White et al. (2004) Circulation 109:513-519.). Direct recombinatorial approaches have been used in AAV (Park et al., (2007) Frontiers in Bioscience 13:2653-59; Girod et al. (1999) Nature Medicine 5:1052-56; Grifman et al. (2001) Molecular Therapy 3:964-75; Shi et al. (2001) Human Gene Therapy 12:1697-1711; Shi and Bartlett (2003) Molecular Therapy 7:515-525), retrovirus (Dalba et al. Current Gene Therapy 5:655-667; Tai and Kasahara (2008) Frontiers in Bioscience 13:3083-3095; Russell and Cosset (1999) Journal of Gene Medicine 1:300-311; Erlwein et al. (2002) Virology 302:333-341; Chadwick et al. (1999) Journal of Molecular Biology 285:485-494; Pizzato et al. (2001) Gene Therapy 8:1088-1096), poxvirus (Guse et al. (2011) Expert Opinion on Biological Therapy 11:595-608; Galmiche et al. (1997) Journal of General Virology 78:3019-3027; Paul et al. (2007) Viral Immunology 20:664-671), paramyxovirus (Nakamura and Russell (2004) Expert Opinion on Biological Therapy 4:1685-1692; Hammond et al. (2001) Journal of Virology 75:2087-2096; Galanis (2010) Clinical Pharmacology and Therapeutics 88:620-625; Blechacz and Russell (2008) Current Gene Therapy 8:162-175; Russell and Peng (2009) Current Topics in Microbiology and Immunology 330:213-241), and herpesvirus (Shah and Breakefield (2006) Current Gene Therapy 6:361-370; Campadelli-Fiume et al. (2011) Reviews in Medical Virology 21:213-226).

Each of the three approaches has advantages and disadvantages. A main advantage of a direct recombinatorial approach is the specificity of the viral vector is inherent in the viral genome, and may be maintained upon replication. However, for this and indirect recombinatorial approaches, the ability to genetically modify the virus requires the structure of the capsid be maintained, and the targeting ligand or scaffold be placed in a position that will tolerate and appropriately display the targeting ligand or scaffold, thus limiting the repertoire of appropriate ligands or scaffolds that can be used. As such, recombinatorial retargeting methods are limited by naturally existing molecules useful as targeting ligands, leading to the incorporation of other binding ligands such as antibodies or portions thereof. Both the non-recombinatorial and recombinatorial adaptor platforms are advantageous in the flexibility in the adaptor used. However, optimal transduction efficiencies are difficult to achieve with these two component systems.

Also mentioned is Wickham et al. (1997) Journal of Virology, 71(10): 7663-7669 which is concerned with targeted-adenovirus-mediated gene delivery to T cells via CD3.

Provided herein is an AAV retargeting strategy that solves the problems inherent in previous recombinatorial retargeting strategies by inserting a heterologous epitope into an AAV capsid. The instant recombinant AAV capsid exhibits reduced to abolished natural tropism, which is restored and redirected upon combination with a multispecific, optionally bispecific, binding molecule comprising an antibody paratope, e.g., Fv, that specifically binds the heterologous epitope and a retargeting ligand that specifically binds a target cell, particularly within certain viral vector: multispecific binding molecule ratios.

### SUMMARY OF THE INVENTION

Disclosed herein are recombinant adeno-associated virus (AAV) capsid proteins, AAV capsids comprising the recombinant AAV capsid proteins, AAV vectors comprising nucleotide of interest encapsulated by the recombinant AAV capsid; wherein said AAV capsid proteins, AAV capsids, and AAV vectors are genetically modified to comprise (display) a heterologous epitope, wherein the heterologous epitope (portion thereof or in combination with the AAV capsid protein) forms a binding pair with an antibody paratope, and wherein the recombinant AAV capsid protein/capsid/vector may further comprise a mutation, insertion or deletion at an amino acid position involved with receptor binding, e.g., the (natural) tropism of the AAV capsid protein/capsid/vector, such that the recombinant AAV capsid protein or an AAV capsid comprising the recombinant AAV protein has a reduced to abolished (natural) tropism (e.g., has, in the absence of a multispecific, optionally bispecific, binding molecule, a transduction efficiency that is less than the transduction efficiency of a reference AAV capsid protein/capsid/vector that lacks the heterologous epitope, or an undetectable transduction efficiency in the absence of a multispecific, optionally bispecific, binding molecule). Such reduced to abolished (natural) tropism of the recombinant AAV capsid protein/capsid/vector may be enhanced or restored in the presence of an appropriate multispecific, optionally bispecific, binding moiety. Accordingly, also described are compositions comprising (1) the recombinant AAV vectors having a capsid comprising a recombinant AAV capsid protein described herein and (2) a multispecific, optionally bispecific, binding molecule comprising the antibody paratope and a targeting ligand, including compositions comprising certain viral vector:multispecific binding molecule ratios; and uses of same to direct and/or introduce genetic material to a target cell are also described herein. Also described are methods of retargeting a recombinant AAV vector, e.g., for the targeted delivery of a nucleotide of interest to a target cell, comprising contacting the recombinant AAV vector with a multispecific, optionally bispecific, binding molecule, and methods of making the recombinant AAV vector, are also described.

Accordingly, the present invention provides a composition comprising:
(i) a recombinant adeno-associated virus (AAV) vector comprising an AAV capsid that encapsulates a nucleotide of interest,
   wherein the AAV capsid comprises a recombinant AAV capsid protein modified to comprise a heterologous epitope, wherein the recombinant AAV capsid protein is derived from an AAV capsid gene modified to express the heterologous epitope;
(ii) a multispecific binding molecule comprising
   (a) an antibody paratope that specifically binds the heterologous epitope; and
   (b) a retargeting ligand that specifically binds a cell surface protein or marker; and optionally
(iii) a pharmaceutically acceptable carrier,
   wherein the AAV capsid exhibits:
   - reduced to abolished tropism in the absence of the multispecific binding molecule; and
   - modified tropism upon combination with the multispecific binding molecule compared to a reference AAV capsid, wherein the reference AAV capsid comprises a reference AAV capsid protein that is identical to the recombinant AAV capsid protein but for the lack of the heterologous epitope.

The present invention further provides a composition of the present invention for use in an *in vivo* method of therapy or diagnosis by delivering the nucleotide of interest to a target cell expressing a cell surface protein comprising contacting the target cell with the composition, wherein the multispecific binding molecule comprises a retargeting ligand that binds the cell surface protein.

The present invention also provides an *in vitro* or *ex vivo* method of delivering a nucleotide of interest to a target cell expressing a cell surface protein comprising contacting the target cell with the composition of the present invention, wherein the multispecific binding molecule comprises a retargeting ligand that binds the cell surface protein.

Described herein are recombinant AAV capsid proteins comprising an epitope that is heterologous to the capsid protein, wherein the epitope or portion thereof specifically binds an antibody paratope, and wherein the recombinant AAV capsid protein or an AAV capsid comprising the recombinant AAV capsid protein has a reduced to abolished natural tropism, e.g., in the absence of a multispecific, optionally bispecific, binding moiety.

In some embodiments, the heterologous epitope is inserted (displayed) by the recombinant AAV capsid protein such that the insertion and/or display of the heterologous epitope reduces or abolishes the (natural) tropism of the AAV capsid compared to a reference AAV capsid lacking the heterologous epitope, e.g., the AAV capsid comprises a mutation comprising an insertion of the epitope at the amino acid position and/or a substitution of an amino acid with the epitope at the amino acid position, wherein the mutation reduces or abolishes the (natural) tropism of the AAV capsid protein, e.g., in the absence of a multispecific, optionally bispecific, binding moiety. In some embodiments, the heterologous epitope is inserted (displayed by) the AAV capsid protein such that the insertion and/or display partially reduces the (natural) tropism of the recombinant AAV capsid compared to a reference AAV capsid lacking the heterologous epitope, e.g., in the absence of a multispecific, optionally bispecific, binding moiety, and the AAV capsid further comprises a further mutation (e.g., a substitution, deletion, insertion other than the insertion of a heterologous epitope) that reduces further and/or abolishes the (natural) tropism of the recombinant AAV capsid or recombinant AAV vectors comprising same, e.g., in the absence of a multispecific, optionally bispecific, binding moiety, compared to a reference AAV capsid lacking the mutation.

Generally, the recombinant AAV capsid proteins described herein may be derived from an AAV capsid gene, e.g., is encoded by an AAV capsid gene modified to express the epitope and/or is a genetically modified AAV. In some embodiments, a recombinant AAV capsid protein described herein is derived from an AAV capsid gene, e.g., is encoded by a capsid gene modified to express the epitope and/or is a genetically modified adeno-associated virus (AAV) capsid protein of a AAV serotype that infects primates, optionally wherein the AAV is selected from the group consisting of AAV1, AAV2, AAV3, AAV4, AAV5, AAV6, AAV7, AAV8 and AAV9. In some embodiments, the recombinant AAV capsid protein is derived from an AAV2, AAV6, AAV8, or AAV9 capsid gene, e.g., is a genetically modified AAV2 capsid protein, a genetically modified AAV6 capsid protein, a genetically modified AAV8 capsid protein, or a genetically modified AAV9 capsid protein. In some embodiments, the recombinant AAV capsid protein is derived from an AAV2 capsid gene, e.g., is encoded by an AAV2 capsid gene modified to express the epitope and/or is a genetically modified AAV2 VP1, VP2, and/or VP3 capsid protein, for which the amino acid sequence of a wildtype AAV2 VP1 protein is set forth respectively as SEQ ID NO:1. In some embodiments, the recombinant AAV capsid protein is derived from an AAV6 capsid gene, e.g., is encoded by an AAV6 capsid gene modified to express the epitope and/or is a genetically modified AAV6 VP1, VP2 and/or VP3 capsid protein, for which the amino acid sequences of wildtype AAV6 VP1 is set forth as SEQ ID NO:3. In some embodiments, the recombinant AAV capsid protein is derived from an AAV8 capsid gene, e.g., is encoded by an AAV8 capsid gene modified to express the epitope and/or is a genetically modified AAV8 VP1, VP2, and/or VP3 capsid protein, for which the amino acid sequence of a wildtype AAV VP1 protein is set forth respectively as SEQ ID NO:21. In some embodiments the recombinant AAV capsid protein is derived from an AAV9 capsid gene, e.g., is encoded by an AAV9 capsid gene modified to express the epitope and/or is a genetically modified AAV9 VP1, VP2 or VP3 capsid protein, for which the wildtype amino acid sequence of AAV9 VP1 is set forth respectively as SEQ ID NO:5. In some embodiments, the recombinant AAV capsid protein is derived from an AAV2 capsid gene, e.g., is encoded by an AAV2 capsid gene modified to express the epitope and/or is a genetically modified VP1, VP2, and/or VP3 capsid protein of AAV2. In some embodiments, the recombinant AAV capsid protein is derived from an AAV6 capsid gene, e.g., is encoded by an AAV6 capsid gene modified to express the epitope and/or is a genetically modified VP1, VP2, and/or VP3 capsid protein of AAV6. In some embodiments, the recombinant AAV capsid protein is derived from an AAV8 capsid gene, e.g., is encoded by an AAV8 capsid gene modified to express the epitope and/or is a genetically modified VP1, VP2, and/or VP3 capsid protein of AAV8. In some embodiments, the recombinant AAV capsid protein is derived from an AAV9 capsid gene, e.g., is encoded by an AAV9 capsid gene modified to express the epitope and/or is a genetically modified VP1, VP2, and/or VP3 capsid protein of AAV9.

In some embodiments, the recombinant AAV capsid protein is derived from, e.g., is encoded by a chimeric AAV capsid gene modified to express the epitope, wherein the chimeric AAV capsid gene comprises a plurality of nucleic acid sequences, wherein each of the plurality of nucleic acid sequences encodes a portion of an AAV capsid protein of a different AAV serotype, and wherein the plurality of nucleic acid sequences together encodes a chimeric AAV capsid protein. In some embodiments, the recombinant AAV capsid protein is derived from a chimeric AAV2 capsid gene. In some embodiments, the recombinant AAV capsid protein is derived from a chimeric AAV6 capsid gene. In some embodiments, the AAV capsid protein is derived from a chimeric AAV8 capsid gene. In some embodiments, the recombinant AAV capsid protein is derived from a chimeric AAV9 capsid gene.

Generally, a recombinant AAV capsid protein as described herein comprises a heterologous epitope inserted into and/or displayed by the recombinant AAV capsid protein such that the heterologous epitope itself reduces and/or abolishes the natural tropism of the recombinant AAV capsid protein or capsid comprising same, as compared to a reference AAV capsid protein lacking the heterologous epitope or AAV capsid comprising the reference AAV capsid protein, respectively. In some embodiments, the heterologous epitope is inserted into (displayed in) a region of the AAV capsid protein involved with the natural tropism of the wildtype reference AAV capsid protein, e.g., a region of the AAV capsid protein involved with cell targeting. In some embodiments, the heterologous epitope is inserted into and/or displayed by a knob domain of an Ad fiber protein. In some embodiments, the heterologous epitope is inserted into and/or displayed by the HI loop of an Ad fiber protein. In some embodiments, the heterologous epitope is inserted into and/or displayed in the heparin binding site of an AAV capsid protein. In some embodiments, the heterologous epitope is inserted into and/or displayed in the heparin binding site of an AAV2 capsid protein. In some embodiments, the heterologous epitope is inserted into and/or displayed in the heparin binding site of an AAV6 capsid protein. In some embodiments, the heterologous epitope is inserted into and/or displayed in the heparin binding site of an AAV8 capsid protein. In some embodiments, the heterologous epitope is inserted into and/or displayed in the heparin binding site of an AAV9 capsid protein. In some embodiments, (i) the AAV capsid protein is derived from an AAV2 capsid gene and the epitope is inserted after and/or replaces an amino acid at position I-453 or I-587 of the AAV2 VP1 capsid protein and/or amino acids at corresponding positions of the AAV2 VP2 and/or VP3 capsid proteins; (ii) the AAV capsid protein is derived from an AAV6 capsid gene and the epitope is inserted after and/or replaces an amino acid at position 1-585 of the AAV6 VP1 capsid protein and/or amino acids at corresponding positions of the AAV6 VP2 and/or VP3 capsid proteins; (iii) the AAV capsid is derived from an AAV8 capsid gene and the epitope is inserted after and/or replaces an amino acid at position 1-590 of the AAV8 VP1 capsid protein and/or amino acids at corresponding positions of the AAV8 VP2 and/or VP3 capsid proteins, or (iv) the AAV capsid protein is derived from an AAV9 capsid gene and the epitope is inserted after and/or replaces an amino acid at position I-453 or I-589 of the AAV9 VP1 capsid protein and/or amino acids at corresponding positions of the AAV9 VP2 and/or VP3 capsid proteins. In some embodiments, the heterologous epitope is inserted immediately after (e.g., is fused to the C-terminus of) an amino acid selected from the group consisting of G-453 of AAV2 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV3, AAV4, AAV5, AAV6, AAV7, AAV8, and AAV9), N587 of AAV2 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV3, AAV4, AAV5, AAV6, AAV7, AAV8, and AAV9), Q585 of AAV6 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV7, AAV8, and AAV9), N-590 of AAV8 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV9), G453 of AAV9 capsid protein VP1(or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV8), or A-589 of AAV9 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV8). In some embodiments, the heterologous epitope is inserted immediately after (e.g., is fused to the C-terminus of) G-453 of AAV2 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, AAV8, and AAV9). In some embodiments, the heterologous epitope is inserted immediately after (e.g., is fused to the C-terminus of) N587 of AAV2 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV3, AAV4, AAV5, AAV6, AAV7, AAV8, and AAV9). In some embodiments, the heterologous epitope is inserted immediately after (e.g., is fused to the C-terminus of Q-585 of AAV6 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV7, AAV8, and AAV9). In some embodiments, the heterologous epitope is inserted immediately after (e.g., is fused to the C-terminus of) N-590 of AAV8 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV9). In some embodiments, the heterologous epitope is inserted immediately after (e.g., is fused to the C-terminus of) G453 of AAV9 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV8). In some embodiments, the heterologous epitope is inserted immediately after (e.g., is fused to the C-terminus of) A-589 of AAV9 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV8). In some embodiments, the heterologous epitope is inserted and/or displayed between amino acids N-587 and R-588 of an AAV2 VP1 capsid protein (or corresponding positions of the VP2 and/or VP3 capsids encoded from the same capsid gene). In some embodiments, a recombinant AAV capsid, AAV vector comprising a recombinant AAV capsid, and/or compositions comprising a recombinant AAV capsid comprises an amino acid sequence set forth as SEQ ID NO:2. In some embodiments, a recombinant AAV capsid, AAV vector comprising a recombinant AAV capsid, and/or compositions comprising a recombinant AAV capsid comprises an amino acid sequence set forth as SEQ ID NO:4. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:25. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:26. In some embodiments, a recombinant AAV capsid, AAV1 vector comprising a recombinant AAV capsid, and/or compositions comprising a recombinant AAV capsid comprises an amino acid sequence encoded by the nucleic acid sequence set forth as SEQ ID NO:27.

In some embodiments, a recombinant AAV capsid protein as described herein comprises a second and different mutation, in addition to the heterologous epitope. For example, in some embodiments, a recombinant AAV capsid protein as described herein may be a genetically modified AAV2 capsid protein, comprise a heterologous epitope, and may further comprise a mutation, e.g., a R-585-A and/or R-588-A mutation. In some embodiments, a recombinant AAV capsid protein is derived from an AAV2 capsid gene, e.g., is a genetically modified AAV2 VP1 capsid protein, comprises a heterologous epitope inserted immediately after (e.g., fused to the C-terminus of) G-453 of the AAV2 VP1 protein, and further comprises a mutation selected from the group consisting of R-585A and/or R-5889A. In some embodiments, a recombinant AAV capsid protein is derived from an AAV2 capsid gene, e.g., is a genetically modified AAV2 VP1 capsid protein, comprises a heterologous epitope inserted immediately after (e.g., fused to the C-terminus of) N587 of the AAV2 VP1 protein, and further comprises a mutation selected from the group consisting of R-585A and/or R-588A. In some embodiments, a recombinant AAV capsid protein is derived from an AAV9 capsid gene, e.g., is a genetically modified AAV9 VP1 capsid protein, comprises a heterologous epitope inserted immediately after (e.g., fused to the C-terminus of) G453 of the AAV9 VP1 protein, and further comprises a W503A mutation. In some embodiments, a recombinant AAV capsid protein is derived from an AAV9 capsid gene, e.g., is a genetically modified AAV9 VP1 capsid protein, comprises a heterologous epitope inserted immediately after (e.g., fused to the C-terminus of) A589 of AAV9 VP1 capsid protein, and further comprises a W503A mutation.

Generally, a recombinant AAV capsid protein and/or a AAV vector comprising the recombinant AAV capsid comprises a heterologous epitope that is at least one amino acid in length. In some embodiments, the heterologous epitope may be between about 5 amino acids and about 35 amino acids in length, and forms a binding pair with an antibody paratope, e.g., an immunoglobulin variable domain. In some embodiments, the heterologous epitope comprises at least 10 amino acids in length. In some embodiments, the heterologous epitope comprises an affinity tag. In some aspects, the heterologous epitope and/or affinity tag does not form a binding pair with an immunoglobulin constant domain. In some aspects, the heterologous epitope and/or affinity tag does not form a binding pair with a metal ion, e.g., Ni²⁺, Co²⁺, Cu²⁺, Zn²⁺, Fe³⁺, etc. In some embodiments, the heterologous epitope is not a polypeptide selected from the group consisting of Streptavidin, Strep II, HA, L14, 4C-RGD, LH, and Protein A. In some embodiments, the affinity tag is selected from the group consisting of FLAG (SEQ ID NO:7), HA (SEQ ID NO:8) and c-myc (EQKLISEEDL; SEQ ID NO:6). In some embodiments, the heterologous epitope comprises c-myc (EQKLISEEDL; SEQ ID NO:6).

In some embodiments, a recombinant AAV capsid protein is a genetically modified AAV2 VP1 capsid protein and comprises a heterologous epitope comprising the sequence EQKLISEEDL (SEQ ID NO:6) inserted immediately after (e.g., fused to the C-terminus of) G-453 of the AAV2 VP1 capsid protein. In some embodiments, a recombinant AAV capsid protein (i) is derived from an AAV2 capsid gene, e.g., is a genetically modified AAV2 VP1 capsid protein, (ii) comprises a heterologous epitope that comprises the sequence EQKLISEEDL (SEQ ID NO:6) and is inserted immediately after (e.g., fused to the C-terminus of) G-453 of the AAV2 VP1 capsid protein, and (iii) further comprises a mutation selected from the group consisting of R-585A and/or R-5889-A. In some embodiments, a recombinant AAV capsid protein is a genetically modified AAV2 VP1 capsid protein and comprises a heterologous epitope comprising the sequence EQKLISEEDL (SEQ ID NO:6) inserted immediately after (e.g., fused to the C-terminus of) N587 of the AAV2 VP1 capsid protein. In some embodiments, a recombinant AAV capsid protein (i) is derived from an AAV2 capsid gene, e.g., is a genetically modified AAV2 VP1 capsid protein, (ii) comprises a heterologous epitope that comprises the sequence EQKLISEEDL (SEQ ID NO:6) and is inserted immediately after (e.g., fused to the C-terminus of) N587 of the AAV2 VP1 capsid protein, and (iii) further comprises a mutation selected from the group consisting of R585A and/or R588A. In some embodiments, a recombinant AAV capsid protein (i) is derived from an AAV6 capsid gene, e.g., is a genetically modified AAV6 VP1 capsid protein, (ii) comprises a heterologous epitope that comprises the sequence EQKLISEEDL (SEQ ID NO:6) and is inserted immediately after (e.g., fused to the C-terminus of) Q-585 of the AAV6 VP1 capsid protein. In some embodiments, a recombinant AAV capsid protein is a genetically modified AAV8 VP1 capsid protein and comprises a heterologous epitope comprising the sequence EQKLISEEDL (SEQ ID NO:6) inserted immediately after (e.g., fused to the C-terminus of) N590 of the AAV8 VP1 capsid protein. In some embodiments, a recombinant AAV capsid protein is a genetically modified AAV9 VP1 capsid protein and comprises a heterologous epitope comprising the sequence EQKLISEEDL (SEQ ID NO:6) inserted immediately after (e.g., fused to the C-terminus of) G453 of the AAV9 VP1 capsid protein. In some embodiments, a recombinant AAV capsid protein (i) is derived from an AAV9 capsid gene, e.g., is a genetically modified AAV9 VP1 capsid protein, (ii) comprises a heterologous epitope that comprises the sequence EQKLISEEDL (SEQ ID NO:6) and is inserted immediately after (e.g., fused to the C-terminus of) G453 of the AAV9 VP1 capsid protein, and (iii) further comprises a W503A mutation. In some embodiments, a recombinant AAV capsid protein is a genetically modified AAV9 VP1 capsid protein and comprises a heterologous epitope comprising the sequence EQKLISEEDL (SEQ ID NO:6) inserted immediately after (e.g., fused to the C-terminus of) A589 of the AAV9 VP1 capsid protein. In some embodiments, a recombinant AAV capsid protein (i) is derived from an AAV9 capsid gene, e.g., is a genetically modified AAV9 VP1 capsid protein, (ii) comprises a heterologous epitope that comprises the sequence EQKLISEEDL (SEQ ID NO:6) and is inserted immediately after (e.g., fused to the C-terminus of) A589 of the AAV9 VP1 capsid protein, and (iii) further comprises a W503A mutation.

In some embodiments, a recombinant AAV capsid as described herein comprises the amino acid sequence EQKLISEEDL (set forth as SEQ ID NO: 6) flanked by and/or operably linked to at least 5 contiguous amino acids of an AAV VP1 capsid protein. In some embodiments, a recombinant AAV capsid as described herein comprises the amino acid sequence EQKLISEEDL (set forth as SEQ ID NO: 6) flanked by and/or operably linked to at least 5 contiguous amino acids of an AAV2 VP1 capsid protein. In some embodiments, a recombinant AAV capsid as described herein comprises EQKLISEEDL (set forth as SEQ ID NO:6) inserted at I587 of an AAV2 VP1 capsid protein. In some embodiments, a recombinant AAV capsid as described herein comprises EQKLISEEDL (set forth as SEQ ID NO:6) inserted between N587 and R588 of an AAV2 VP1 capsid protein, e.g., comprises an amino acid sequence set forth as SEQ ID NO: 2.

In some embodiments, a recombinant AAV capsid as described herein comprises the amino acid sequence EQKLISEEDL (set forth as SEQ ID NO: 6) flanked by and/or operably linked to at least 5 contiguous amino acids of an AAV6 VP1 capsid protein. In some embodiments, a recombinant AAV capsid as described herein comprises EQKLISEEDL (set forth as SEQ ID NO:6) inserted at 1585 of an AAV6 VP1 capsid protein. In some embodiments, a recombinant AAV capsid as described herein comprises EQKLISEEDL (set forth as SEQ ID NO:6) inserted between Q585 and S586 of an AAV6 VP1 capsid protein, e.g., comprises an amino acid sequence set forth as SEQ ID NO: 4.

In some embodiments, a recombinant AAV capsid as described herein comprises the amino acid sequence EQKLISEEDL (set forth as SEQ ID NO:6) flanked by and/or operably linked to at least 5 contiguous amino acids of an AAV8 VP1 capsid. In some embodiments, a recombinant AAV capsid as comprises EQKLISEEDL (set forth as SEQ ID NO:6) inserted at I590 of an AAV8 VP1 capsid protein. In some embodiments, a recombinant AAV capsid as comprises EQKLISEEDL (set forth as SEQ ID NO:6) inserted between N590 and T591 of an AAV8 VP1 capsid protein, e.g., comprises an amino acid sequence set forth as SEQ ID NO:25.

In some embodiments, a recombinant AAV capsid as described herein comprises the amino acid sequence EQKLISEEDL (set forth as SEQ ID NO: 6) flanked by and/or operably linked to at least 5 contiguous amino acids of an AAV9 VP1 capsid protein. In some embodiments, a recombinant AAV capsid as comprises EQKLISEEDL (set forth as SEQ ID NO:6) inserted at I453 of an AAV9 VP1 capsid protein. In some embodiments, a recombinant AAV capsid as comprises EQKLISEEDL (set forth as SEQ ID NO:6) inserted between G-453 and S-454 of an AAV9 VP1 capsid protein, e.g., comprises an amino acid sequence set forth as SEQ ID NO:26. In some embodiments, a recombinant AAV capsid as comprises EQKLISEEDL (set forth as SEQ ID NO:6) inserted at I589 of an AAV9 VP1 capsid protein. In some embodiments, a recombinant AAV capsid as comprises EQKLISEEDL (set forth as SEQ ID NO:6) inserted between A-589 and Q-590 of an AAV9 VP1 capsid protein, e.g., comprises an amino acid sequence set forth as SEQ ID NO:27.

In some embodiments, the heterologous epitope comprises an affinity tag and one or more linkers. In some embodiments, the heterologous epitope comprises an affinity tag flanked by a linker, e.g., the heterologous epitope comprises from N-terminus to C-terminus a first linker, an affinity tag, and a second linker. In some embodiments, the first and second linkers are each independently a polypeptide of at least 1 amino acid in length. In some embodiments, a heterologous epitope as described herein, e.g., an affinity tag by itself or in combination with one or more linkers, is between about 5 amino acids to about 35 amino acids in length. In some embodiments, the first and second linkers are identical lengths and/or comprise identical amino acid sequences.

Generally, in the absence of an appropriate multispecific, optionally bispecific, binding molecule, a recombinant AAV capsid comprising a recombinant AAV capsid protein as described herein has reduced to abolished natural tropism, e.g., has a reduced capacity or is unable to target and bind a reference cell naturally permissive to transduction compared to that of a reference AAV capsid, e.g., a capsid comprising a reference AAV capsid protein, e.g., a AAV capsid protein that would be identical to the recombinant AAV capsid protein but for the lack of the heterologous epitope. In some embodiments and in the absence of an appropriate multispecific, optionally bispecific, binding molecule, a recombinant AAV capsid comprising a recombinant AAV capsid protein as described herein exhibits at least 10% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 20% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 30% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 40% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 50% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 60% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 70% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 75% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 80% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 85% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 90% decrease in transduction efficiency compared to a reference AAV capsid. In some embodiments and in the absence of an appropriate multispecific, optionally bispecific, binding molecule, a recombinant AAV capsid comprising a recombinant AAV capsid protein as described herein exhibits at least 95% decrease in transduction efficiency compared to a reference AAV 1 capsid. In some embodiments and in the absence of an appropriate multispecific, optionally bispecific, binding molecule, a recombinant AAV capsid comprising a recombinant AAV capsid protein as described herein exhibits at least 99% decrease in transduction efficiency compared to a reference AAV capsid. In some embodiments and in the absence of an appropriate multispecific, optionally bispecific, binding molecule, transduction of a control cell by a recombinant AAV capsid comprising a recombinant AAV capsid protein as described herein is abolished, e.g., undetectable.

In some embodiments an AAV capsid comprising a recombinant AAV capsid protein as described herein is a mosaic capsid, e.g., comprises a recombinant AAV capsid protein comprising a heterologous epitope and a reference AAV capsid protein that does not comprise the heterologous epitope at a certain ratio. In some embodiments, a reference AAV capsid protein is a wildtype reference AAV capsid protein in that it comprises an amino acid sequence of a wildtype AAV capsid protein having the same serotype as the recombinant AAV capsid protein. In some embodiments, a reference capsid protein is a control reference AAV capsid protein in that it comprises an amino acid sequence of the recombinant AAV capsid protein except that the control reference AAV capsid protein lacks the heterologous epitope. In some embodiments, a reference AAV capsid protein is a mutated wildtype reference protein in that it comprises an amino acid sequence substantially identical to that of a wildtype AAV capsid protein having the same serotype as the recombinant AAV capsid protein but for a mutation, (e.g., an insertion of an amino acid sequence, chimerization, etc.) that reduces the tropism of the wildtype AAV capsid protein. In some embodiments, a composition described herein comprises, or a method described herein combines, a recombinant AAV capsid protein and a reference capsid protein at a ratio that ranges from 1:1 to 1:15. In some embodiments, the ratio is 1:2. In some embodiments, the ratio is 1:3. In some embodiments, the ratio is 1:4. In some embodiments, the ratio is 1:5. In some embodiments, the ratio is 1:6. In some embodiments, the ratio is 1:7. In some embodiments, the ratio is 1:8. In some embodiments, the ratio is 1:9. In some embodiments, the ratio is 1:10. In some embodiments, the ratio is 1:1 1. In some embodiments, the ratio is 1:12. In some embodiments, the ratio is 1:13. In some embodiments, the ratio is 1:14. In some embodiments, the ratio is 1:15.

Also disclosed herein are nucleic acids that encode a recombinant AAV capsid protein described herein, compositions comprising such nucleic acids (e.g., which may be used in methods of making a recombinant AAV capsid) and/or recombinant AAV capsid proteins (e.g., compositions consisting essentially of a recombinant AAV capsid protein, compositions comprising only AAV vectors encapsulated by a capsid comprising a AAV capsid protein described herein, compositions comprising such AAV vectors and a multispecific, optionally bispecific, binding molecule (e.g., at certain AAV vector to multispecific, optionally bispecific, binding molecule (molecule:molecule) ratios), compositions comprising such AAV vectors, retargeting moieties, and a pharmaceutically acceptable carrier, etc.). In some embodiments, a nucleic acid as described herein comprises a nucleotide sequence that encodes the amino acid sequence of EQKLISEEDL (SEQ ID NO:6) and a nucleotide sequence that encodes at least 5 contiguous amino acids of an adeno-associated virus capsid protein.

In some embodiments, a nucleic acid as described herein comprises a nucleotide sequence that encodes the amino acid sequence EQKLISEEDL (set forth as SEQ ID NO: 6) flanked by and/or operably linked to at least 5 contiguous amino acids of an AAV2 VP1 capsid protein. In some embodiments, a nucleic acid as described herein comprises a nucleotide sequence that encodes EQKLISEEDL (set forth as SEQ ID NO:6) inserted at I587 of an AAV2 VP1 capsid protein. In some embodiments, a nucleic acid as described herein comprises a nucleotide sequence that encodes EQKLISEEDL (set forth as SEQ ID NO:6) inserted between N587 and R588 of an AAV2 VP1 capsid protein, e.g., in some embodiments, a nucleic acid as described encodes an amino acid sequence comprising an amino acid sequence set forth as SEQ ID NO:2.

In some embodiments, a nucleic acid as described herein comprises a nucleotide sequence that encodes the amino acid sequence EQKLISEEDL (set forth as SEQ ID NO: 6) flanked by and/or operably linked to at least 5 contiguous amino acids of an AAV6 VP1 capsid protein. In some embodiments, a nucleic acid as described herein comprises a nucleotide sequence that encodes EQKLISEEDL (set forth as SEQ ID NO:6) inserted at I-585 of an AAV6 VP1 capsid protein. In some embodiments, a nucleic acid as described herein comprises a nucleotide sequence that encodes EQKLISEEDL (set forth as SEQ ID NO:6) inserted between Q-585 and S-586 of an AAV6 VP1 capsid protein, e.g., in some embodiments, a nucleic acid as described encodes an amino acid sequence comprising an amino acid sequence set forth as SEQ ID NO:4.

In some embodiments, a nucleic acid as described herein comprises a nucleotide sequence that encodes the amino acid sequence EQKLISEEDL (set forth as SEQ ID NO: 6) flanked by and/or operably linked to at least 5 contiguous amino acids of an AAV8 VP1 capsid protein. In some embodiments, a nucleic acid as described herein comprises a nucleotide sequence that encodes EQKLISEEDL (set forth as SEQ ID NO:6) inserted at I-590 of an AAV8 VP1 capsid protein. In some embodiments, a nucleic acid as described herein comprises a nucleotide sequence that encodes EQKLISEEDL (set forth as SEQ ID NO:6) inserted between N-590 and T-591 of an AAV8 VP1 capsid protein, e.g., in some embodiments, a nucleic acid as described encodes an amino acid sequence comprising an amino acid sequence set forth as SEQ ID NO:25.

In some embodiments, a nucleic acid as described herein comprises a nucleotide sequence that encodes the amino acid sequence EQKLISEEDL (set forth as SEQ ID NO: 6) flanked by and/or operably linked to at least 5 contiguous amino acids of an AAV9 VP1 capsid protein. In some embodiments, a nucleic acid as described herein comprises a nucleotide sequence that encodes EQKLISEEDL (set forth as SEQ ID NO:6) inserted at I453 of an AAV9 VP1 capsid protein. In some embodiments, a nucleic acid as described herein comprises a nucleotide sequence that encodes EQKLISEEDL (set forth as SEQ ID NO:6) inserted between G453 and S454 of an AAV9 VP1 capsid protein, e.g., in some embodiments, a nucleic acid as described encodes an amino acid sequence comprising an amino acid sequence set forth as SEQ ID NO:26. In some embodiments, a nucleic acid as described herein comprises a nucleotide sequence that encodes EQKLISEEDL (set forth as SEQ ID NO:6) inserted at I589 of an AAV9 VP1 capsid protein. In some embodiments, a nucleic acid as described herein comprises a nucleotide sequence that encodes EQKLISEEDL (set forth as SEQ ID NO:6) inserted between A589 and Q590 of an AAV9 VP1 capsid protein, e.g., in some embodiments, a nucleic acid as described encodes an amino acid sequence comprising an amino acid sequence set forth as SEQ ID NO:27.

Generally, recombinant AAV vectors as described herein comprise an AAV capsid comprising a recombinant AAV capsid protein as described herein, wherein the AAV capsid encapsulates a nucleotide of interest. In some embodiments, the nucleotide of interest is under the control of a promoter selected from the group consisting of a viral promoter, a bacterial promoter, a mammalian promoter, an avian promoter, a fish promoter, an insect promoter, and any combination thereof. In some embodiments, the nucleotide of interest is under the control of a non-human promoter. In some embodiments, the promoter is a cytomegalovirus (CMV) promoter. In some embodiments, the promoter is an EF1α promoter.

Generally, a nucleotide of interest may be one or more genes, which may encode a detectable marker, e.g., reporter, or a therapeutic polypeptide. In some embodiments, the nucleotide of interest is a reporter gene. In some embodiments, the nucleotide of interest is a reporter gene that encodes a detectable marker selected from the group consisting of green fluorescence protein, luciferase, β-galactosidase, etc. In some embodiments, the detectable marker is green fluorescence protein. In other embodiments, the nucleotide of interest is selected from the group consisting of a suicide gene, a nucleotide encoding an antibody or fragment thereof, a nucleotide encoding a CRISPR/Cas system or portion(s) thereof, a nucleotide encoding antisense RNA, a nucleotide encoding siRNA, a secreted enzyme, etc. In one embodiment, the nucleotide of interest encodes a multidomain therapeutic, e.g., a protein that comprises at least two domains providing two distinct functions.

Compositions described herein generally comprise an AAV vector that comprises a recombinant AAV capsid protein as described herein, e.g., comprises a capsid comprising the recombinant AAV capsid protein, wherein the capsid encapsulates a nucleotide of interest. In some embodiments, a composition described herein comprises (1) an AAV vector having a capsid comprising a recombinant AAV capsid protein genetically modified to comprise a heterologous epitope, (2) a multispecific, optionally bispecific, binding molecule comprising (i) an antibody paratope that specifically binds the epitope and (ii) a retargeting ligand that specifically binds a receptor, and optionally (3) a pharmaceutically acceptable carrier.

An antibody paratope as described herein generally comprises at a minimum a complementarity determining region (CDR) that specifically recognizes the heterologous epitope, e.g., a CDR3 region of a heavy and/or light chain variable domain. In some embodiments, a multispecific, optionally bispecific, binding molecule comprises an antibody (or portion thereof) that comprises the antibody paratope that specifically binds the heterologous epitope. For example, a multispecific, optionally bispecific, binding molecule may comprise a single domain heavy chain variable region or a single domain light chain variable region, wherein the single domain heavy chain variable region or single domain light chain variable region comprises an antibody paratope that specifically binds the heterologous epitope. In some embodiments, a multispecific, optionally bispecific, binding molecule may comprise an Fv region, e.g., a multispecific, optionally bispecific, binding molecule may comprise an scFv, that comprises an antibody paratope that specifically binds the heterologous epitope. In some embodiments, the multispecific, optionally bispecific, binding molecule comprises an antibody (or portion thereof) that comprises an antibody paratope that specifically binds the heterologous epitope, wherein the antibody (or portion thereof) further comprises one or more antibody constant domains (e.g., a heavy chain constant domain (e.g., CH1, hinge, CH2, CH3, CH4, etc.) and/or a light chain constant domain (e.g., CL), wherein the one or more antibody constant domains do not bind to the heterologous epitope.

A multispecific, optionally bispecific, binding molecule as described herein further comprises a retargeting ligand, in addition to a paratope (e.g., an antibody or portion thereof comprising the paratope) that specifically binds the heterologous epitope inserted into/displayed by a recombinant AAV capsid protein. In some embodiments, the retargeting ligand specifically binds a receptor on the surface of a bead (e.g., for isolation of and/or purification of a recombinant AAV capsid protein as described herein). In some embodiments, the retargeting ligand specifically binds a cell surface protein, e.g., a receptor, cell surface marker, etc., expressed on the surface of a mammalian (e.g., human) eukaryotic cell, e.g., a target cell. In some embodiments, a retargeting ligand binds a (human) liver cell, a (human) brain cell, a (human) T cell, a (human) kidney cell, a (human) intestinal cell, a (human) pancreas cell, a (human) cancerous cell, and/or a (human) cell infected with heterologous pathogen. In some embodiments, a retargeting ligand binds a (human) liver cell specific marker, a (human) brain cell specific marker, a (human) T cell specific marker, a (human) kidney cell specific marker, a (human) intestinal cell specific marker, a (human) pancreas cell specific marker, a (human) tumor cell specific marker, and/or pathogenic epitope.

In some embodiments, the retargeting ligand binds a receptor expressed by a (human) liver cell, e.g., an asialoglycoprotein receptor, e.g., hASGR1. In some embodiments, the retargeting ligand binds a receptor expressed by a (human) neuronal cell, e.g., GABA, transferrin, etc. In some embodiments, the retargeting ligand binds a receptor expressed by a (human) T cell, e.g., CD3, e.g., CD3ε. In some embodiments, the retargeting ligand binds a receptor expressed by a (human) hematopoietic stem cell, e.g., CD34. In some embodiments, the retargeting ligand binds a receptor expressed by a (human) kidney cell. In some embodiments, the retargeting ligand binds a receptor expressed by a (human) muscle cell, e.g., an integrin. In some embodiments, the retargeting ligand binds a receptor expressed by a (human) cancerous cell, e.g., a tumor associated antigen, e.g., adipophilin, AIM-2, ALDH1A1, alpha-actinin-4, alpha-fetoprotein ("AFP"), ARTC1, B-RAF, BAGE-1, BCLX (L), BCR-ABL fusion protein b3a2, beta-catenin, BING-4, CA-125, CALCA, carcinoembryonic antigen ("CEA"), CASP-5, CASP-8, CD274, CD45, Cdc27, CDK12, CDK4, CDKN2A, CEA, CLPP, COA-1, CPSF, CSNK1A1, CTAG1, CTAG2, cyclin D1, Cyclin-A1, dek-can fusion protein, DKK1, EFTUD2, Elongation factor 2, ENAH (hMena), Ep-CAM, EpCAM, EphA3, epithelial tumor antigen ("ETA"), ETV6-AML1 fusion protein, EZH2, E6, E7, FGF5, FLT3-ITD, FN1, G250/MN/CAIX, GAGE-1,2,8, GAGE-3,4,5,6,7, GAS7, glypican-3, GnTV, gp100/Pme117, GPNMB, HAUS3, Hepsin, HER-2/neu, HERV-K-MEL, HLA-A11, HLA-A2, HLA-DOB, hsp70-2, IDOl, IGF2B3, IL13Ralpha2, Intestinal carboxyl esterase, K-ras, Kallikrein 4, KIF20A, KK-LC-1, KKLC1, KM-HN-1, KMHN1 (also known as CCDC110), LAGE-1, LDLR-fucosyltransferaseAS fusion protein, Lengsin, M-CSF, MAGE-A1, MAGE-A10, MAGE-A12, MAGE-A2, MAGE-A3, MAGE-A4, MAGE-A6, MAGE-A9, MAGE-C1, MAGE-C2, malic enzyme, mammaglobin-A, MART2, MATN, MC1R, MCSP, mdm-2, ME1, Melan-A/MART-1, Meloe, Midkine, MMP-2, MMP-7, MUC1, MUC5AC, mucin, MUM-1, MUM-2, MUM-3, Myosin, Myosin class I, N-raw, NA88-A, neo-PAP, NFYC, NY-BR-1, NY-ESO-1/LAGE-2, OA1, OGT, OS-9, P polypeptide, p53, PAP, PAX5, PBF, pml-RARalpha fusion protein, polymorphic epithelial mucin ("PEM"), PPP1R3B, PRAME, PRDX5, PSA, PSMA, PTPRK, RAB38/NY-MEL-1, RAGE-1, RBAF600, RGS5, RhoC, RNF43, RU2AS, SAGE, secernin 1, SIRT2, SNRPD1, SOX10, Sp17, SPA17, SSX-2, SSX-4, STEAP1, survivin, SYT-SSX1 or -SSX2 fusion protein, TAG-1, TAG-2, Telomerase, TGF-betaRII, TPBG, TRAG-3, Triosephosphate isomerase, TRP-1/gp75, TRP-2, TRP2-INT2, tyrosinase, tyrosinase ("TYR"), VEGF, WT1, XAGE-lb/GAGED2a, Kras, NY-ESO1, MAGE-A3, HPV E2, HPV E6, HPV E7, WT-1 antigen (in lymphoma and other solid tumors), ErbB receptors, Melan A [MART1], gp 100, tyrosinase, TRP-1/gp 75, and TRP-2 (in melanoma); MAGE-1 and MAGE-3 (in bladder, head and neck, and non-small cell carcinoma); HPV EG and E7 proteins (in cervical cancer); Mucin [MUC-1] (in breast, pancreas, colon, and prostate cancers); prostate-specific antigen [PSA] (in prostate cancer); carcinoembryonic antigen [CEA] (in colon, breast, and gastrointestinal cancers), and such shared tumor-specific antigens as MAGE-2, MAGE-4, MAGE-6, MAGE-10, MAGE-12, BAGE-1, CAGE-1,2,8, CAGE-3 TO 7, LAGE-1, NY-ESO-1/LAGE-2, NA-88, GnTV, TRP2-INT2, etc. In some embodiments, the retargeting ligand binds E6 and/or E7. In some embodiments, the retargeting ligand binds Her2. In some embodiments, the retargeting ligand binds human glucagon receptor (hGCGR). In some embodiments, the retargeting ligand binds human ectonucleoside triphosphate diphosphohydrolase 3 (hENTPD3).

In some embodiments, the paratope (e.g., an antibody or portion thereof) and the retargeting ligand are directly fused to each other. In some embodiments, the paratope (e.g., an antibody or portion thereof) that specifically binds the heterologous epitope and the retargeting ligand are covalently linked to each other.

In some embodiments, the multispecific binding molecule is a bispecific binding molecule, e.g., an antibody comprising first and second antigen-binding domains, wherein the first antigen-binding domain comprises a paratope that specifically binds the heterologous epitope inserted into/displayed by a recombinant AAV capsid protein and the second antigen-binding domain specifically binds a cell surface protein or marker expressed by a target cell. In some embodiments, the bispecific binding molecule is a bispecific antibody comprising first and second antigen-binding domains, wherein the first antigen-binding domain comprises a paratope that specifically binds the heterologous epitope inserted into/displayed by a recombinant AAV capsid protein and the second antigen-binding domain specifically binds a receptor expressed by a target cell, wherein the first antigen-binding domain is operably linked to a first heavy chain region comprising a first CH3 domain, wherein the second antigen-binding domain is operably linked to a second heavy chain region comprising a second CH3 domain, wherein the first and second Ig C_{H}3 domains differ from one another by at least one amino acid, and wherein at least one amino acid difference reduces binding of the bispecific antibody to Protein A as compared to a bispecific antibody lacking the amino acid difference. In one embodiment, the first Ig C_{H}3 domain binds Protein A and the second Ig C_{H}3 domain contains a mutation that reduces or abolishes Protein A binding such as an H-95-R modification (by IMGT exon numbering; H435R by EU numbering). The second C_{H}3 domain may further comprise a Y-96-F modification (by IMGT; Y-436-F by EU). Further modifications that may be found within the second C_{H}3 domain include: D-16-E, L-18-M, N-44-S, K-52-N, V-57-M, and V-82-I (by IMGT; D-356-E, L-358-M, N-384-S, K-392-N, V-397-M, and V-422-I by EU) in the case of IgG1 antibodies; N-44-S, K-52-N, and V-82-I (IMGT; N-384-S, K-392-N, and V-422-I by EU) in the case of IgG2 antibodies; and Q-15-R, N-44-S, K-52-N, V-57-M, R-6-9K, E-79-Q, and V-82-I (by IMGT; Q-355-R, N-384-S, K-392-N, V-397-M, R-409-K, E-419-Q, and V-422-I by EU) in the case of IgG4 antibodies.

In some embodiments, the multispecific binding molecule is a bispecific binding molecule, e.g., a bispecific antibody comprising first and second antigen-binding domains, wherein the first antigen-binding domain binds an affinity tag displayed by a recombinant AAV capsid protein as described herein, and wherein the second antigen binding domain binds a receptor expressed on the surface of a target cell. In some embodiments, the multispecific binding molecule is a bispecific binding molecule, e.g., a bispecific antibody comprising first and second antigen-binding domains, wherein the first antigen-binding domain binds the amino acid sequence EQKLISEEDL (SEQ ID NO:6) displayed by a recombinant AAV capsid protein as described herein, and wherein the second antigen binding domain binds a receptor expressed on the surface of a target cell. In some embodiments, the multispecific binding molecule is a bispecific binding molecule, e.g., a bispecific antibody comprising first and second antigen-binding domains, wherein the first antigen-binding domain binds the amino acid sequence EQKLISEEDL (SEQ ID NO:6) displayed by a recombinant AAV capsid protein as described herein, and wherein the second antigen binding domain binds hASGR1. In some embodiments, the multispecific binding molecule is a bispecific binding molecule, e.g., a bispecific antibody comprising first and second antigen-binding domains, wherein the first antigen-binding domain binds the amino acid sequence EQKLISEEDL (SEQ ID NO:6) displayed by a recombinant AAV capsid protein as described herein, and wherein the second antigen binding domain binds a CD3 protein, e.g., CD3ε. In some embodiments, the multispecific binding molecule is a bispecific binding molecule, e.g., a bispecific antibody comprising first and second antigen-binding domains, wherein the first antigen-binding domain binds the amino acid sequence EQKLISEEDL (SEQ ID NO:6) displayed by a recombinant AAV capsid protein as described herein, and wherein the second antigen binding domain binds an integrin. In some embodiments, the multispecific binding molecule is a bispecific binding molecule, e.g., a bispecific antibody comprising first and second antigen-binding domains, wherein the first antigen-binding domain binds the amino acid sequence EQKLISEEDL (SEQ ID NO:6) displayed by a recombinant AAV capsid protein as described herein, and wherein the second antigen binding domain binds a human glucagon receptor, e.g., hGCGR. In some embodiments, the multispecific binding molecule is a bispecific binding molecule, e.g., a bispecific antibody comprising first and second antigen-binding domains, wherein the first antigen-binding domain binds the amino acid sequence EQKLISEEDL (SEQ ID NO:6) displayed by a recombinant AAV capsid protein as described herein, and wherein the second antigen binding domain binds ENTPD3.
Also described herein are methods of making and using the recombinant AAV capsid proteins, AAV vectors comprising same, compositions, etc. In some embodiments, a method of redirecting an adeno-associated virus, etc.; delivering diagnostic/therapeutic cargo to a target cell, etc. comprises combining a recombinant AAV vector comprising a recombinant AAV capsid protein as described herein, e.g., an AAV vector comprising a capsid comprising a recombinant AAV capsid displaying a heterologous epitope, with a bispecific binding molecule, wherein the bispecific binding molecule comprises (i) an antibody paratope that specifically binds the epitope and (ii) a retargeting ligand that specifically binds a receptor. Such methods may include as a first step producing a recombinant AAV vector, e.g., culturing a packaging cell in conditions sufficient for the production of AAV vectors, wherein the packaging cell comprises a plasmid encoding the AAV capsid protein comprising the epitope. When delivering diagnostic/therapeutic cargo to a target cell, methods described herein may comprise contacting the target cell with the combination of a AAV vector comprising an AAV capsid comprising a recombinant AAV capsid displaying a heterologous epitope and a multispecific binding molecule, wherein the multispecific binding molecule comprises (i) an antibody paratope that specifically binds the epitope and (ii) a retargeting ligand that specifically binds a receptor expressed by the target cell. In some embodiments, the target cell is *in vitro.* In other embodiments, the present invention also provides a composition of the present invention for use in an *in vivo* method of treatment or diagnosis, the method comprising delivering the nucleotide of interest to a target cell expressing a cell surface protein comprising contacting the target cell with the composition, wherein the multispecific binding molecule comprises a retargeting ligand that binds the cell surface protein. Hence, in such embodiments, the target cell is *in vivo* in a subject, e.g., a human.

In some embodiments, a composition described herein comprises, or a method described herein combines, a recombinant AAV vector comprising a nucleotide of interest encapsulated with a capsid comprising a recombinant capsid protein as described herein and multispecific binding molecule at a molecule:molecule ratio that restores the transduction efficiency of the AAV vector similar to that of a reference AAV vector. In some embodiments, the recombinant AAV vector to multispecific binding molecule (molecule:molecule) ratio ranges from 1:0.5 to 1:100. In some embodiments, the recombinant AAV vector to multispecific binding molecule (molecule:molecule) ratio ranges from 1:4 to 1:20. In some embodiments, the recombinant AAV vector to bispecific binding molecule (molecule:molecule) ratio ranges from 1:8 to 1:15. In some embodiments, the recombinant AAV vector to multispecific binding molecule (molecule:molecule) ratio is 1:4. In some embodiments, the recombinant AAV vector to multispecific binding molecule (molecule:molecule) ratio is 1:8. In some embodiments, the recombinant AAV vector to multispecific binding molecule (molecule:molecule) ratio is 1:15. In some embodiments, the recombinant AAV vector to multispecific binding molecule (molecule:molecule) ratio is 1:20.

Also described herein are methods of inactivating an AAV capsid and/or producing AAV vectors, which methods generally comprise (a) inserting a nucleic acid encoding a heterologous protein into a nucleic acid sequence encoding an AAV capsid protein to form a nucleotide sequence encoding a genetically modified AAV capsid protein comprising the heterologous protein and/or (b) culturing a packaging cell in conditions sufficient for the production of AAV vectors, wherein the packaging cell comprises the nucleotide sequence. In some embodiments, the packaging cell further comprises a helper plasmid and/or a transfer plasmid comprising a nucleotide of interest. In some embodiments, the methods further comprise isolating self-complementary adeno-associated viral vectors from culture supernatant. In some embodiments, the methods further comprise lysing the packaging cell and isolating single-stranded adeno-associated viral vectors from the cell lysate. In some embodiments, the methods further comprise (a) clearing cell debris, (b) treating the supernatant containing AAV vectors with DNase I and MgCl2, (c) concentrating AAV vectors, (d) purifying the AAV vectors, and (e) any combination of (a)-(d). Also provided herein are AAV vector made according to the method described herein, and packaging cell useful for producing a AAV vector as described herein, e.g., packaging cells comprising a plasmid encoding a recombinant AAV capsid protein described.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

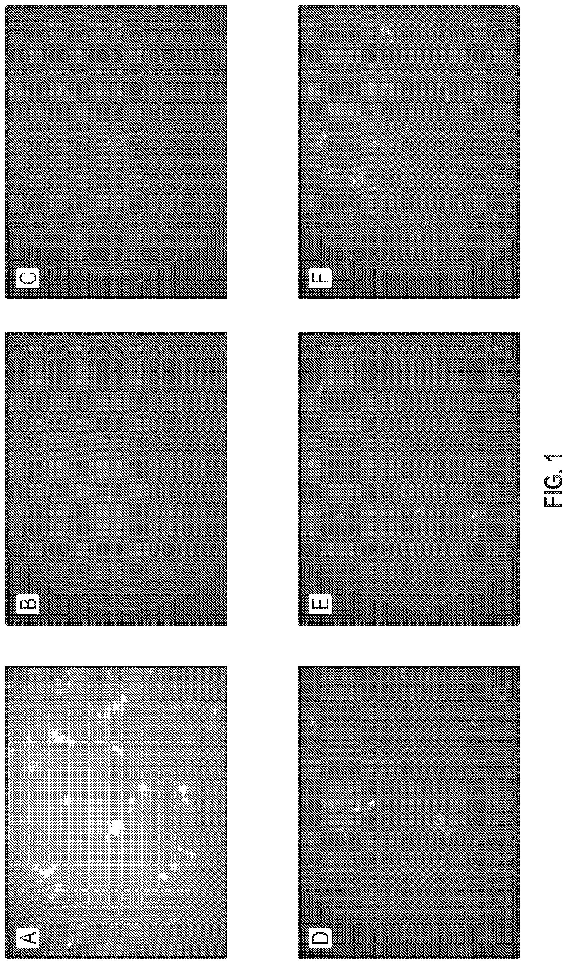

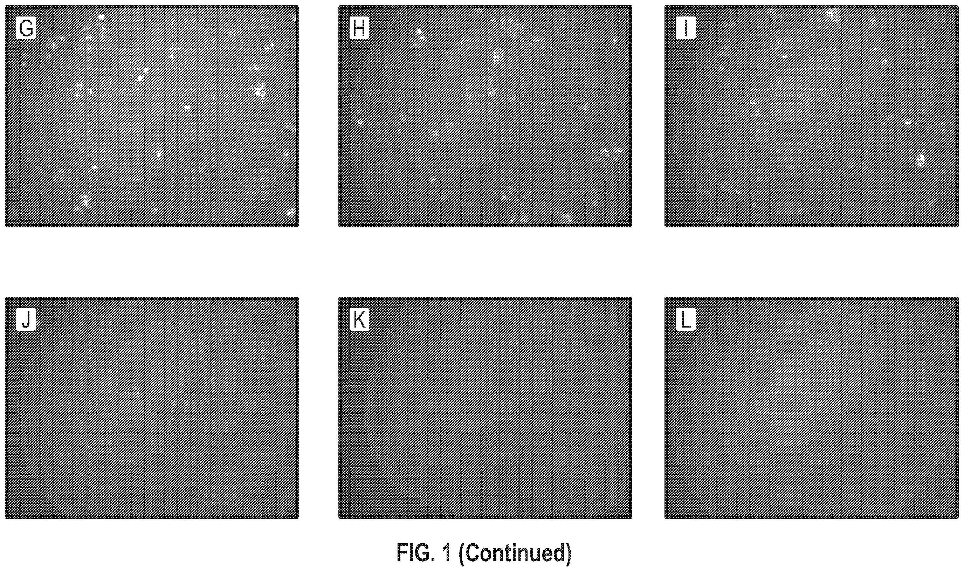

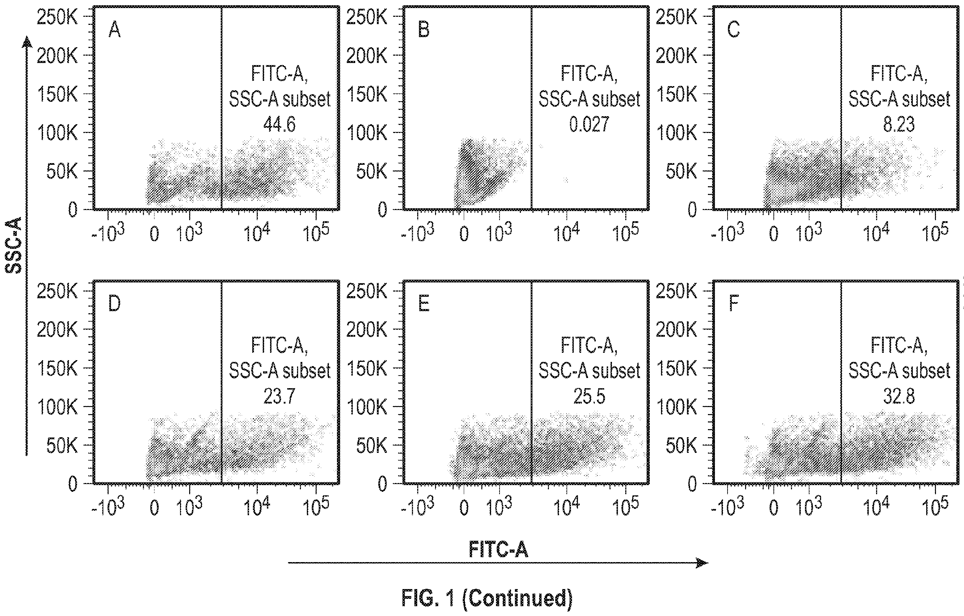

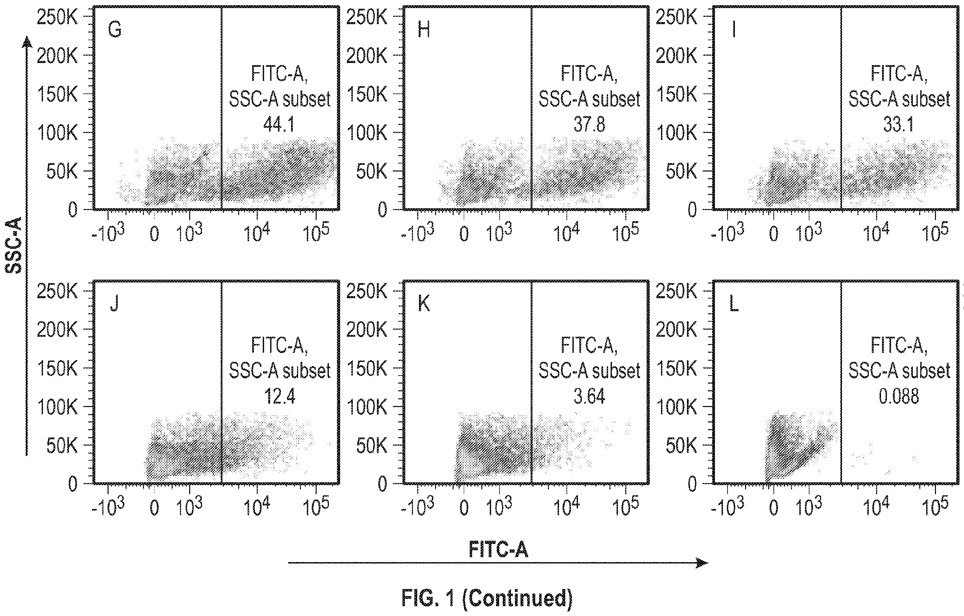

**Figure 1** provides immunofluorescence microscopy images **(top panel)** or histograms obtained from fluorescence-activated cell sorting (FACS) **(bottom panel)** evaluating green fluorescence (GFP) expression by HepG2 cells incubated with (**A**) wildtype scAAV2-CMV-eGFP viral vectors only; (**B**) scAAV2-N587Myc-CMV-hrGFP viral vectors only; scAAV2-N587Myc viral vectors mixed with bispecific anti-Myc-ASGR1 antibodies at the following ratios: (**C**) 1:0.5, (**D**) 1:1, (**E**) 1:2, (**F**) 1:4, (**G**) 1:8, (**H**) 1:15, (**I**) 1:20, (**J**) 1:50, or (**K**) 1:100; (**L**) or scAAV-N587Myc viral vectors mixed with monospecific anti-Myc antibody at a ratio of 1:8.

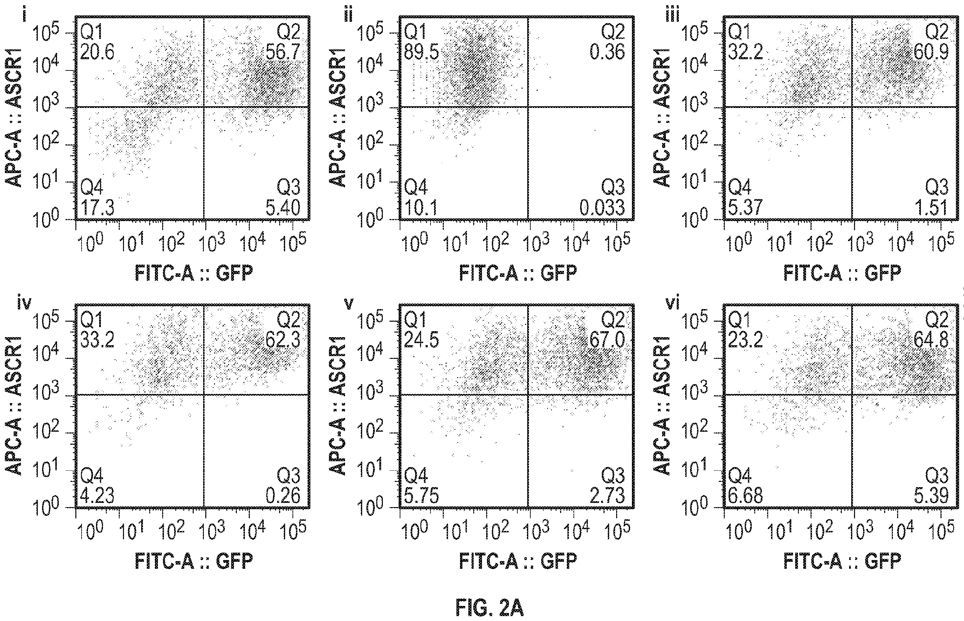

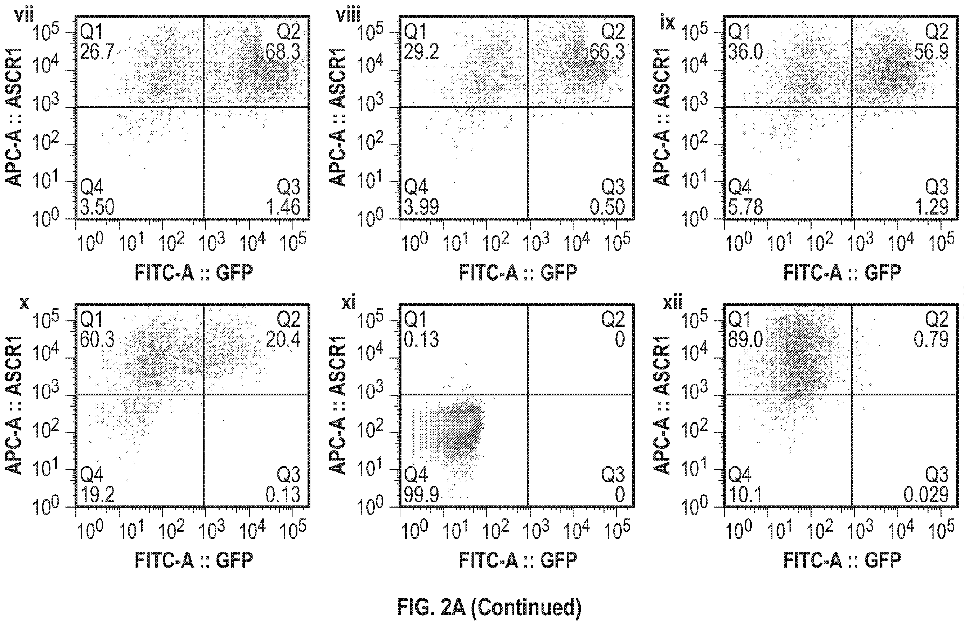

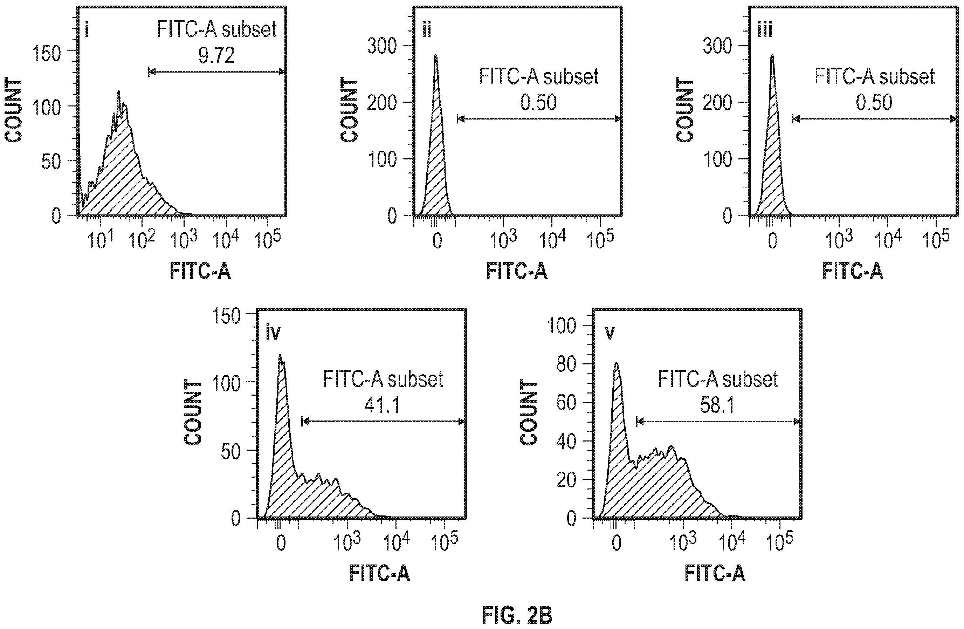

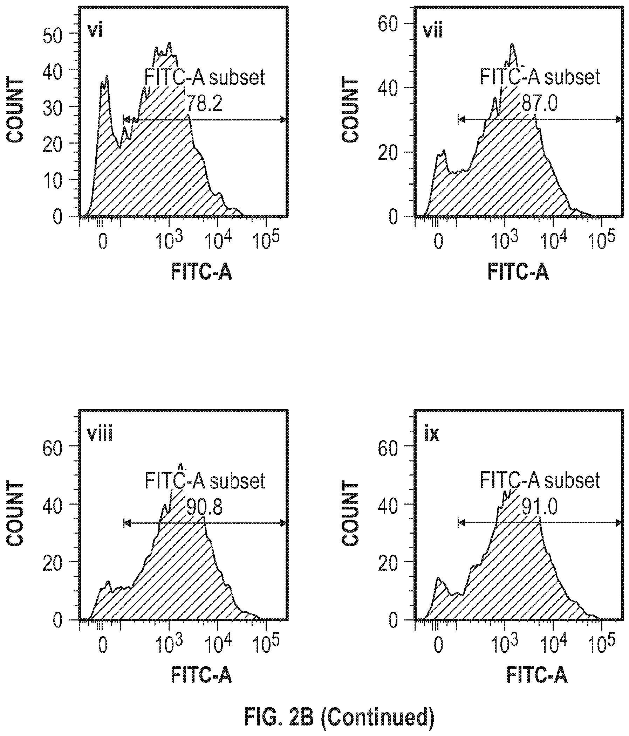

Figure 2A provides dot plots obtained from fluorescence-activated cell sorting (FACS) evaluating green fluorescence (GFP) expression by 29T3-hASGR1 cells incubated with wildtype scAAV2 viral vectors only (**i**), scAAV2-N587Myc-CMV-eGFP viral vectors only (**ii**), scAAV2-N587Myc-CMV-eGFP viral vectors mixed with bispecific anti-Myc-ASGR1 antibodies at the following ratios: 1:0.5 (**iii**), 1:1 (**iv**), 1:2 (**v**), 1:4 (**vi**), 1:8 (**vii**), 1:15 (**viii**), 1:20 (**ix**), or 1:100 (**x**), or scAAV2-N587Myc-CMV-eGFP viral vectors mixed with irrelevant bispecific anti-Myc-GCGR antibody at a ratio of 1:8 (**xii**). GFP expression by 29T3 cells incubated with scAAV-N587Myc viral vectors mixed with bispecific anti-Myc-ASGR1 antibodies at a 1:8 ratio is also shown (**xi**). For each experiment, 2×10⁵ cells and 5×10⁹ viral vectors were used.
Figure 2B provides histograms obtained from fluorescence-activated cell sorting (FACS) evaluating green fluorescence (GFP) expression by 29T3-hASGR1 cells incubated with unmodified AAV9-CAGG-GFP viral vectors only (**i**) AAV9- A589Myc -CAGG-eGFP viral vectors only (**ii**), AAV9- A589Myc -CAGG-eGFP viral vectors mixed with bispecific anti-Myc-ASGR1 antibodies at the following ratios: 1:1 (**iii**), 1:2 (**iv**), 1:4 (**v**), 1:8 (**vi**), 1:20 (**vii**), 1:50 (**viii**), or 1:100 (**ix**). For each experiment, 2×10⁵ cells and 1×10¹⁰ viral vectors (titrated by qPCR) were used.

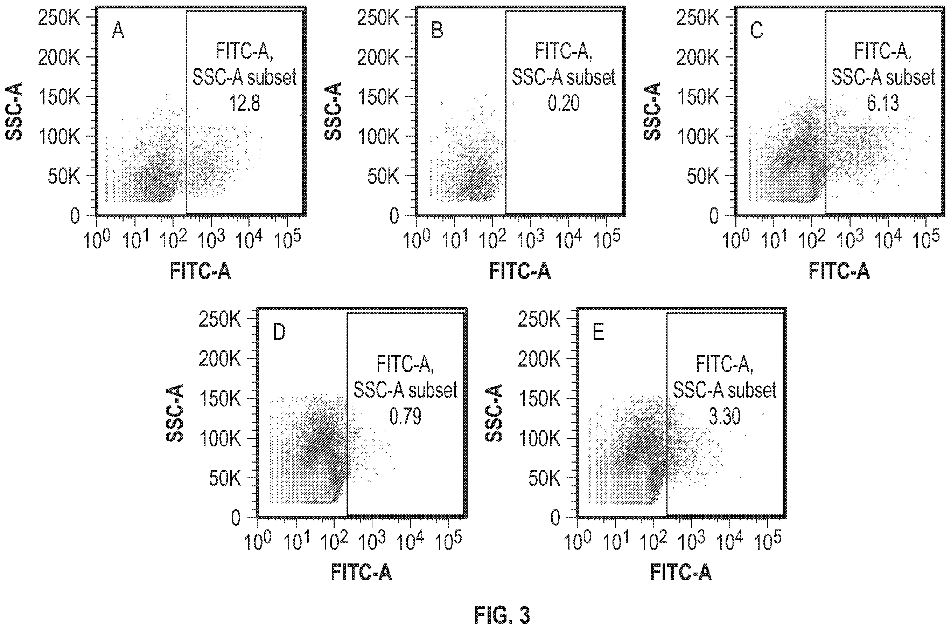

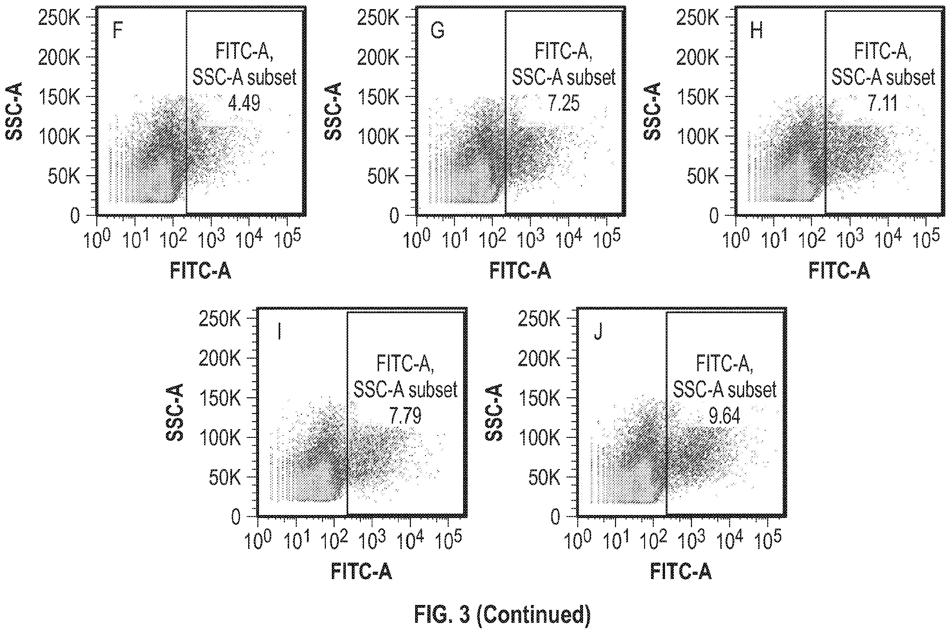

**Figure 3** provides dot plots obtained from fluorescence-activated cell sorting (FACS) evaluating green fluorescence (GFP) expression by 29T3-hASGR1 cells pre-incubated with bivalent anti-ASGR1 antibody at a concentration of 0 nM (**C**), 50 nM (**D**), 10 nM (**E**), 2 nM (**F**), 0.4 nM (**G**), 0.08 nM (**H**), 0.016 nM (**I**) or 0.0032 nM (**J**) and subsequently infected with scAAV2-N587Myc-CMV-eGFP viral vectors mixed with bispecific anti-Myc-ASGR1 antibodies at a ratio of 1:8 (**L**). 293T3-hASGR1 cells incubated with wildtype scAAV viral vectors only (**A**) or scAAV2-N587Myc-CMV-eGFP viral vectors only (**B**) serve as controls. For each experiment, 2×10⁵ cells and 5×10⁹ viral vectors (as titrated by qPCR) were used.

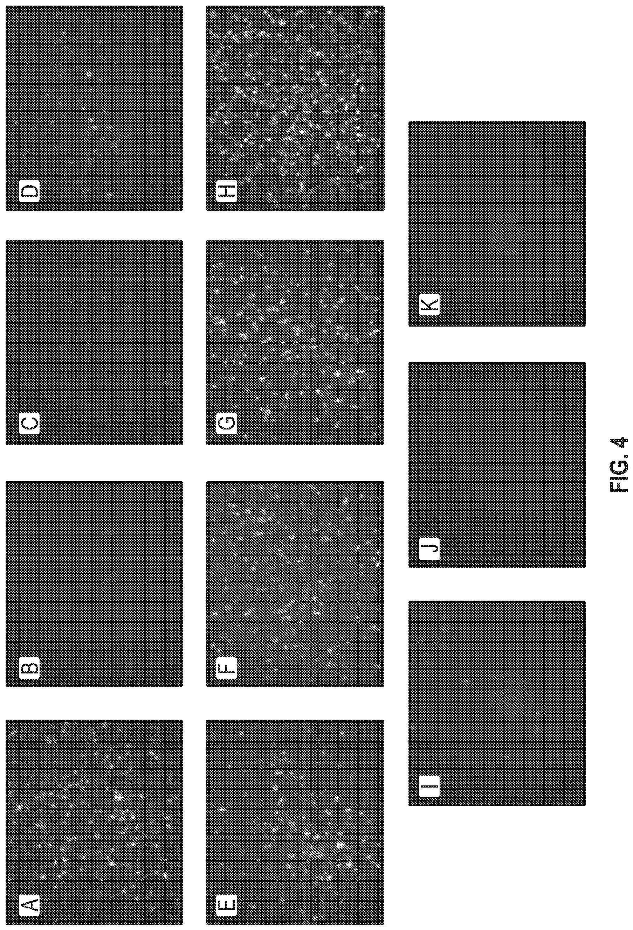

**Figure 4** provides immunofluorescence microscopy images evaluating green fluorescence (GFP) expression by 293T-hASGR1 cells incubated with wildtype scAAV viral vectors only (**A**), scAAV2-N587Myc-CMV-eGFP viral vectors only (**B**), or sequentially incubated with 1×10⁹ (**C**), 2×10⁹ (**D**), 4×10⁹(**E**), 8×10⁹ (**F**), 2×10¹⁰ (**G**), 1×10¹¹ (**H**), 1×10¹² (**I**) bispecific anti-Myc-ASGR1 antibodies followed by 1×10⁹ scAAV2-N587Myc-CMV-eGFP viral vectors. Also shown are immunofluorescence microscopy images of 293T cells sequentially incubated with 1×10¹¹ anti-myc-ASGR1 antibody molecules followed by 1×10⁹ scAAV2-N587Myc-CMV-eGFP viral vectors (**J**), and 293T-hASGR1 cells sequentially incubated with 1×10¹¹ irrelevant bispecific anti-Myc-GCGR antibody molecules followed by 1×10⁹ scAAV2-N587Myc-CMV-eGFP viral vectors (**K**).

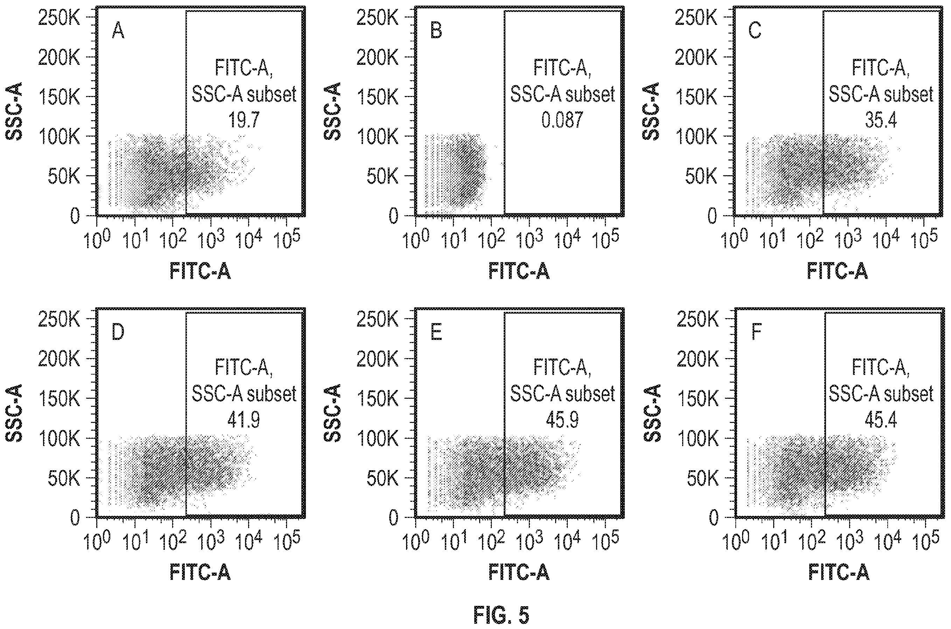

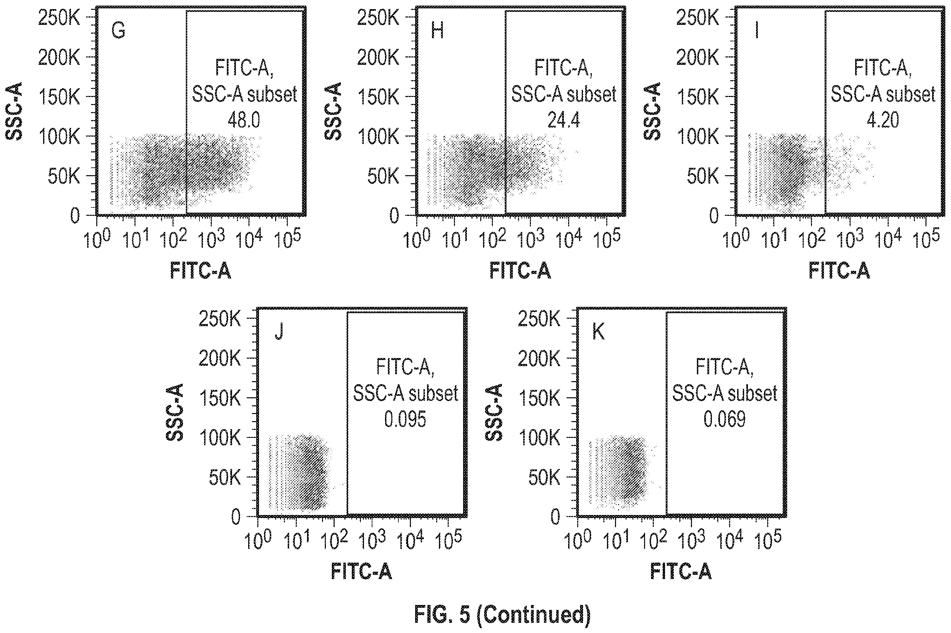

**Figure 5** provides dot plots obtained from fluorescence-activated cell sorting (FACS) evaluating green fluorescence (GFP) expression by 29T3-hASGR1 cells incubated with wildtype ssAAV viral vectors only (**A**), ssAAV2-N587Myc-CMV-hrGFP viral vectors only (**B**), ssAAV2-N587Myc-CMV-hrGFP viral vectors mixed with bispecific anti-Myc-ASGR1 antibodies at the following ratios: 1:1 (**C**), 1:2 (**D**), 1:4 (**E**), 1:8 (**F**), 1:20 (**G**), 1:100 (**H**), 1:1000 (**I**), or ssAAV-N587Myc viral vectors mixed with irrelevant bispecific anti-Myc-GCGR antibody at a ratio of 1:8 (**K**). GFP expression by 29T3 cells incubated with ssAAV-N587Myc viral vectors mixed with bispecific anti-Myc-ASGR1 antibodies at a 1:8 ratio is also shown (**J**). For each experiment, 2×10⁵ cells and 5×10⁹ viral vectors were used.

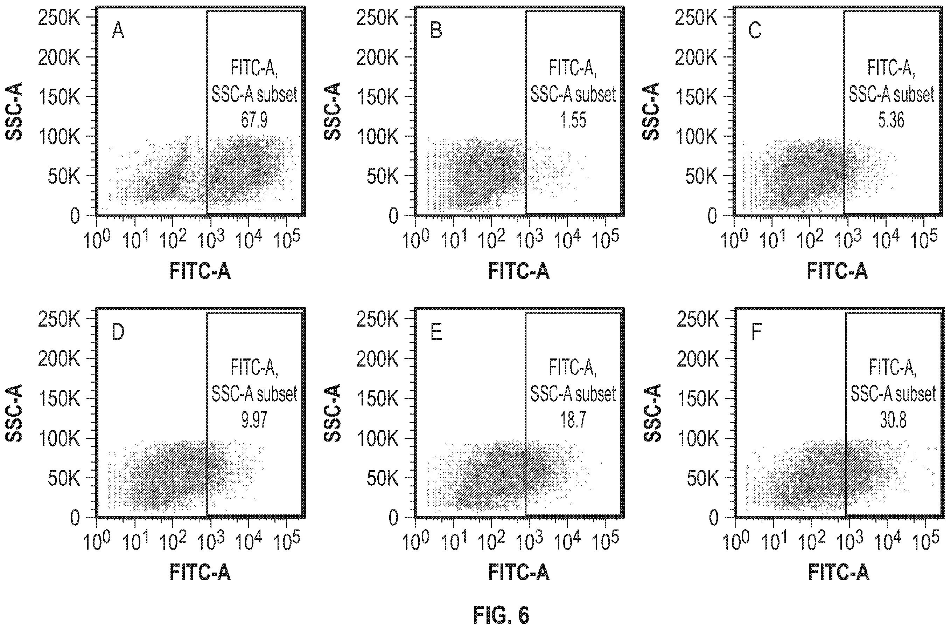

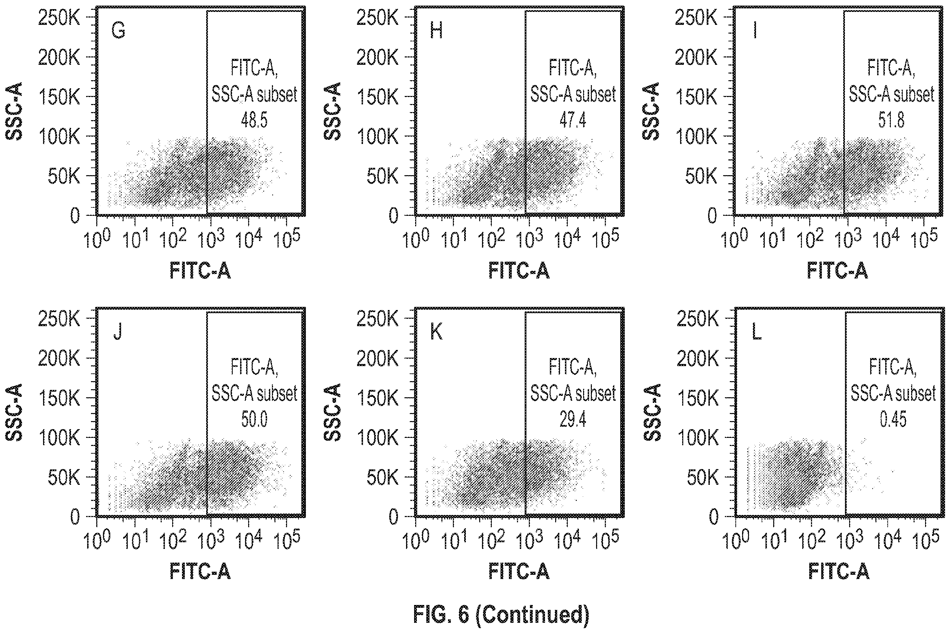

**Figure 6** provides dot plots obtained from fluorescence-activated cell sorting (FACS) evaluating green fluorescence (GFP) expression by 29T3-hGCGR cells incubated with wildtype scAAV viral vectors only (**A**), scAAV2-N587Myc-CMV-eGFP viral vectors only (**B**), scAAV2-N587Myc-CMV-eGFP viral vectors mixed with bispecific anti-Myc-GCGR antibodies at the following ratios: 1:0.5 (**C**), 1:1 (**D**), 1:2 (**E**), 1:4 (**F**), 1:8 (**G**), 1:15 (**H**), 1:20 (**I**), 1:50 (**J**) or 1:100 (**K**), or scAAV2-N587Myc-CMV-eGFP viral vectors mixed with an irrelevant monospecific anti-Myc antibody (Regeneron Pharmaceuticals, Tarrytown, NY) at a ratio of 1:8 (**L**). For each experiment, 2×10⁵ cells and 5×10⁹ viral vectors were used.

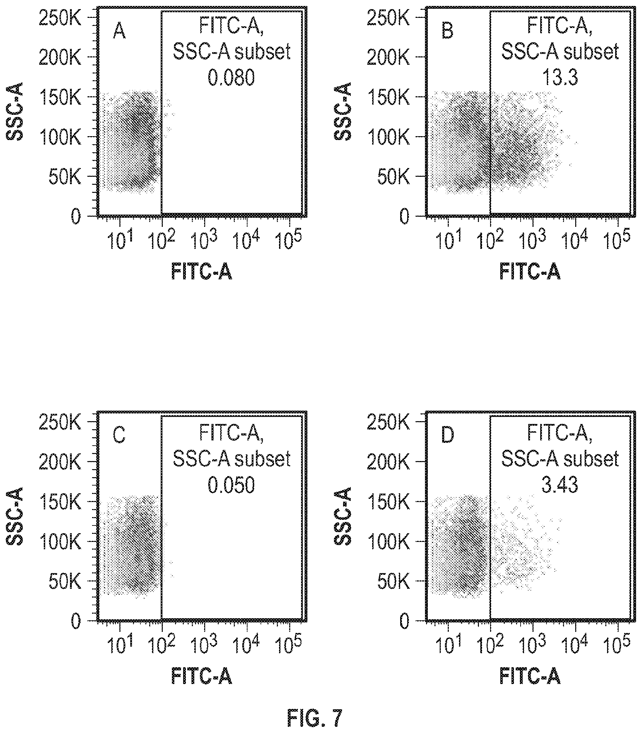

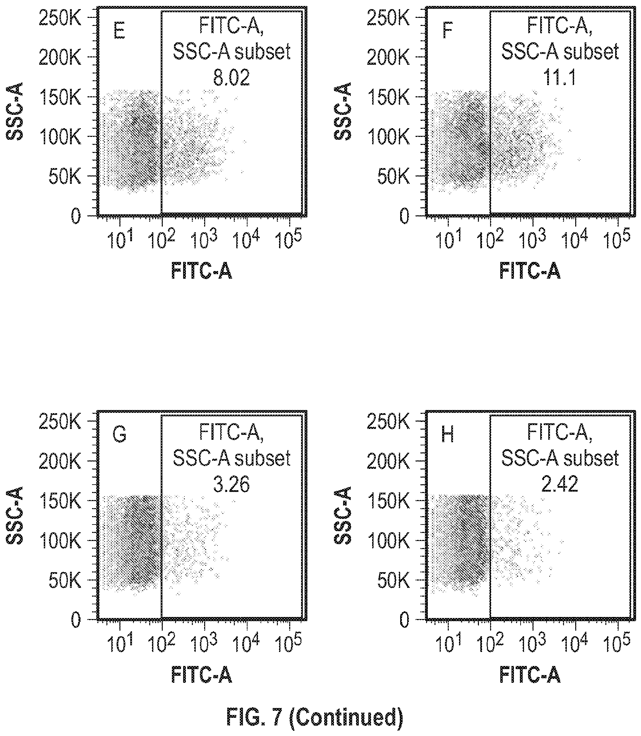

**Figure 7** provides dot plots obtained from fluorescence-activated cell sorting (FACS) evaluating green fluorescence (GFP) expression by Jurkat cells alone (**A**) or Jurkat cells incubated with wildtype scAAV6-EF1-eGFP viral vectors only (**B**), AAV6-Q585Myc-EFla-eGFP viral vectors only (**C**), AAV6-Q585Myc-EF1a-eGFP viral vectors mixed with bispecific anti-Myc-CD3 antibodies at the following ratios: 1:1 (**D**), 1:5 (**E**), 1:10 (**F**), 1:100 or (**G**), 1:1000 (**H**). For each experiment, 2×10⁵ cells and 1×10⁹ viral vectors were used.

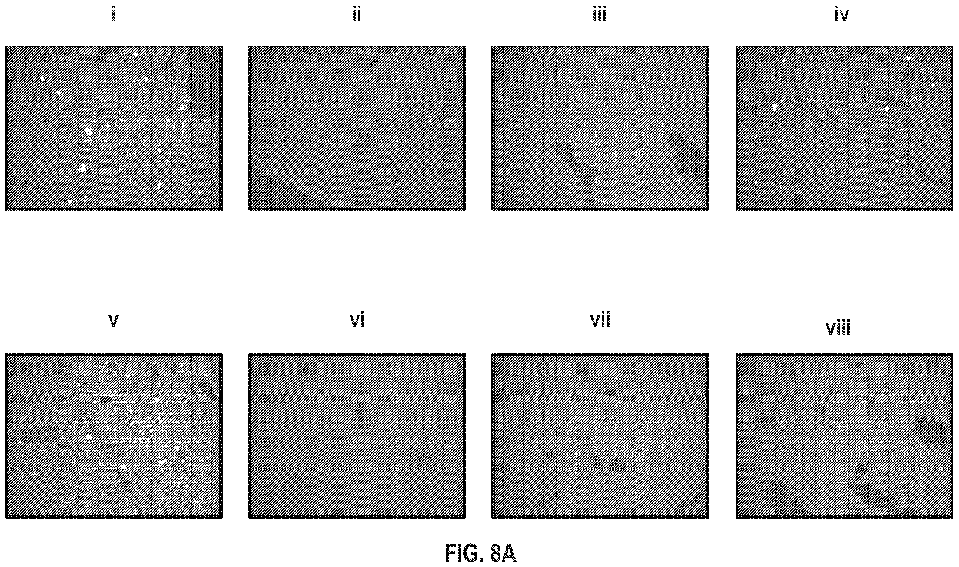

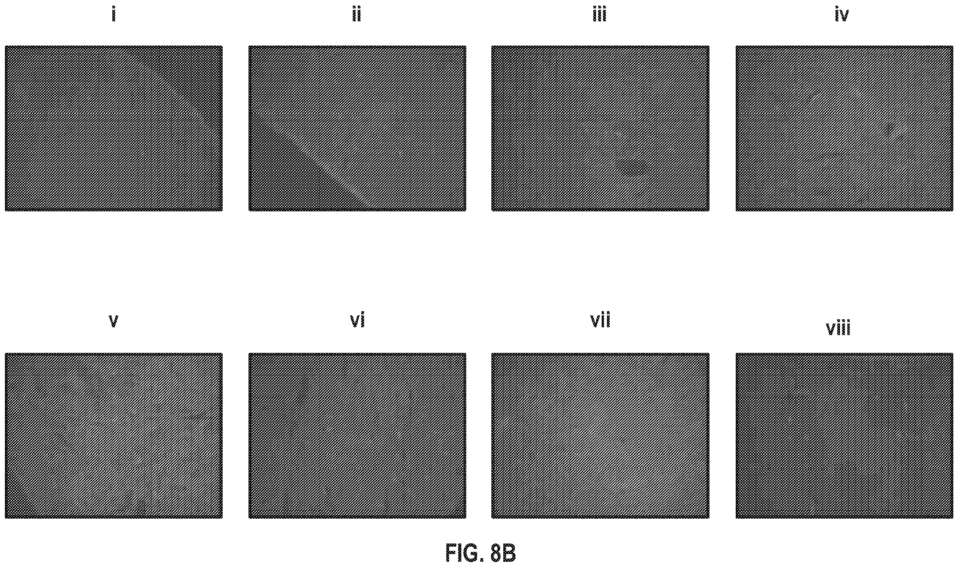

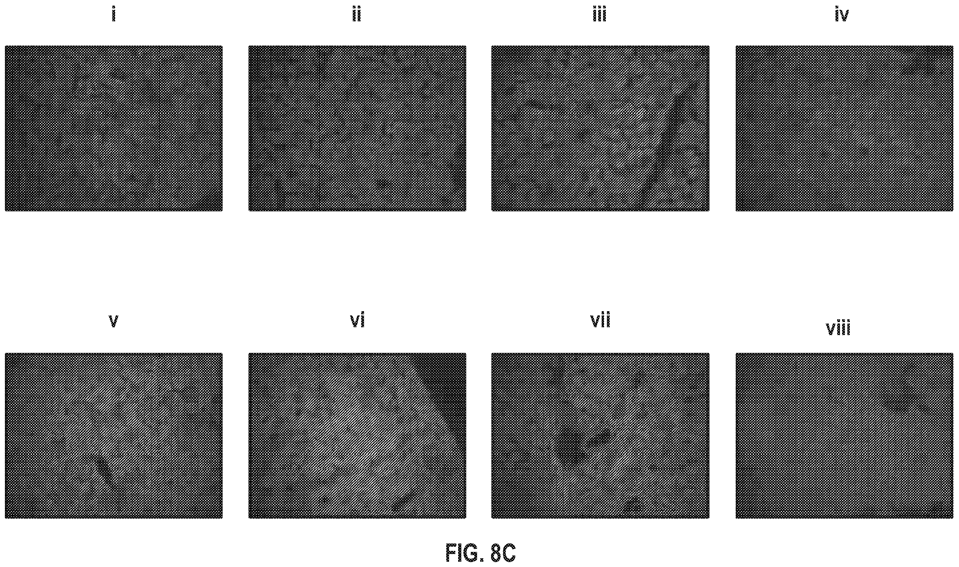

Figure 8A provides immunofluorescence microscopy images of liver. Figure 8B provides immunofluorescence microscopy images of spleen. Figure 8C provides immunofluorescence microscopy images of kidney. All samples are taken from C57BL/6 mice transgenically modified to express human ASGR1 by liver cells (**i-iv**) or wildtype C57BL/6 mice (v-viii) ten days post intravenous injection with 1×10¹¹ wildtype scAAV2-CMV-eGFP (**i, v**), saline (ii, vi), 1×10¹¹ scAAV2-N587myc-CMV-eGFP viral vectors alone (iii, vii), or scAAV2-N587myc-CMV-eGFP viral vectors with bispecific anti-myc-ASGR1 antibody (iv, viii).

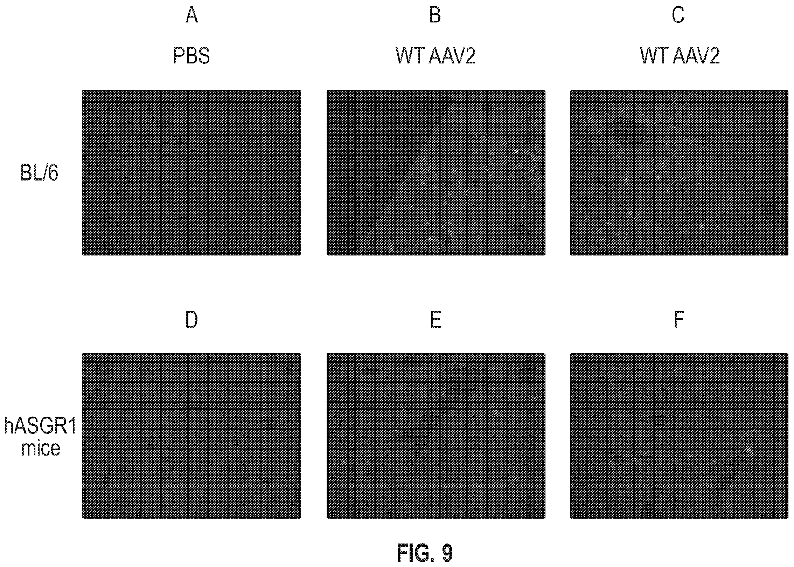

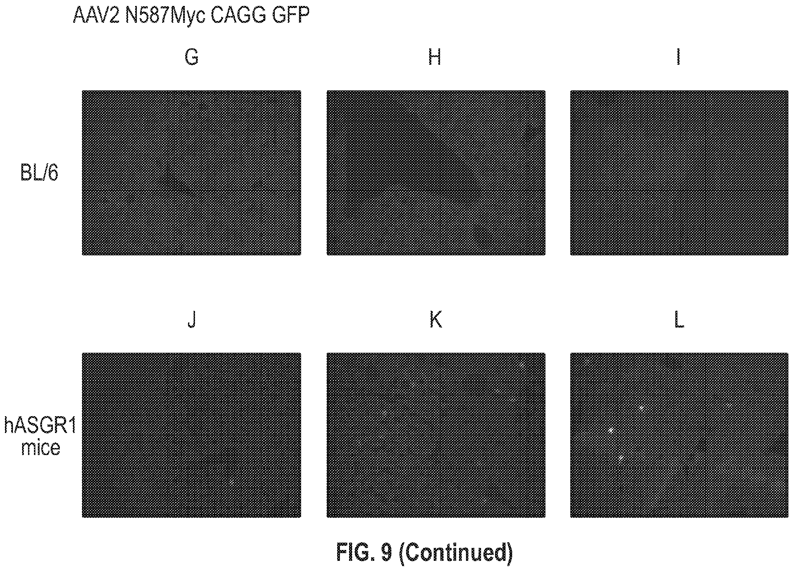

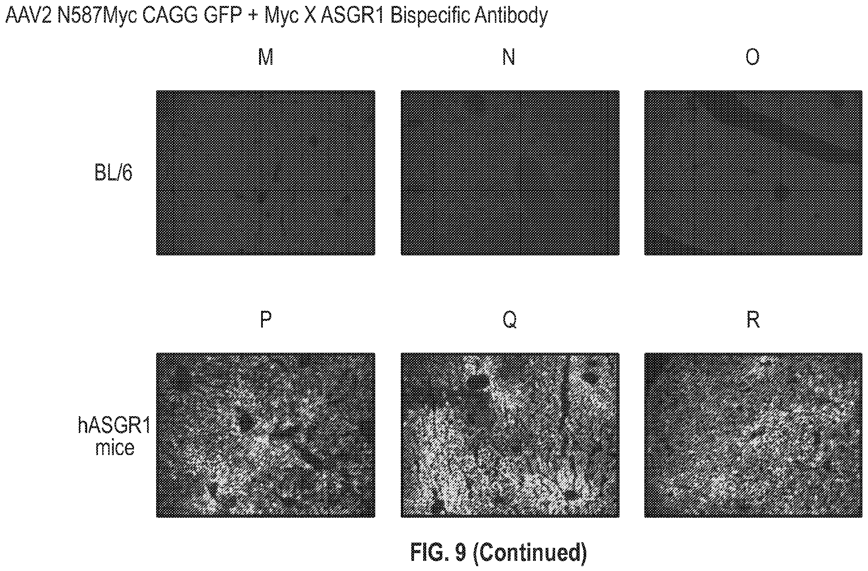

**Figure 9** provides immunofluorescence microscopy images of liver samples taken from C57BL/6 mice transgenically modified to express human ASGR1 on liver cells (D-F, J-L, P-R) or wildtype C57BL/6 mice (A-C, G-I, M-O) four weeks post intravenous injection with 2.18×10¹¹ wildtype ssAAV2-CAGG-eGFP (B, C, E, F), saline (A, D), 2.18×10¹¹ ssAAV2-N587myc-CAGG-eGFP viral vectors alone (G-I, J-L), or ssAAV2-N587myc-CAGG-eGFP viral vectors with bispecific anti-myc-ASGR1 antibody (M-O, P-R). Each image represents one mouse.

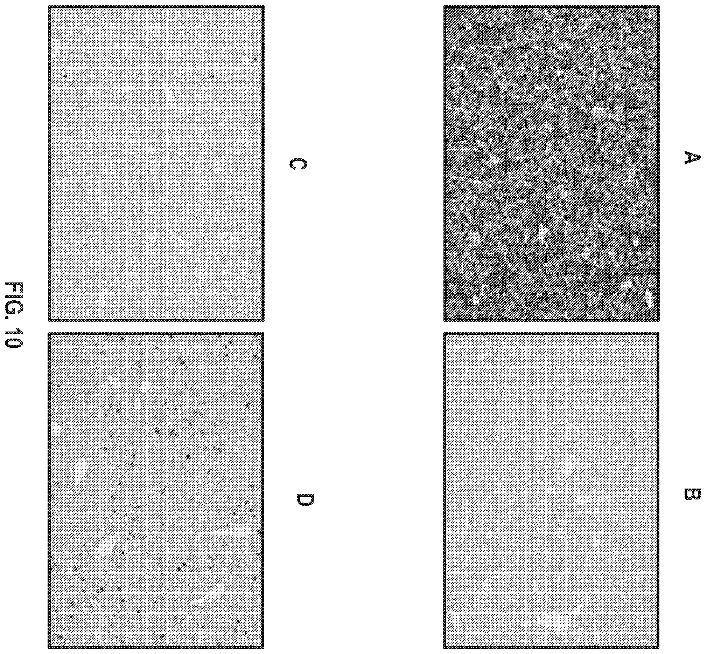

**Figure 10** provides immunofluorescence microscopy images of liver samples taken from C57BL/6 mice transgenically modified to express human ASGR1 by liver cells ten days post intravenous injection with (**A**) wildtype AAV9, (**B**) 250 nM NaCl (**C**) AAV9-A589myc-CAGG-eGFP viral particles in combination with bispecific anti-myc-hCD3 antibody, or (**D**) AAV9-A589myc-CAGG-eGFP viral particles in combination with bispecific anti-myc-ASGR1 antibody.

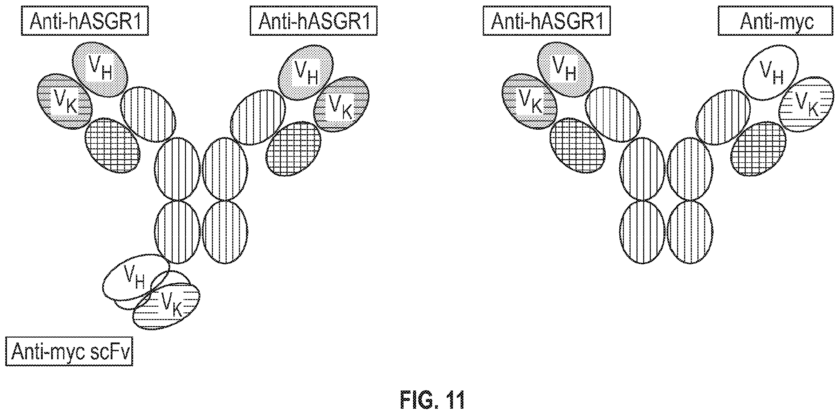

**Figure 11** provides illustrative, not to scale, and non-limiting exemplary multispecific binding molecule formats useful in some embodiments of the invention.

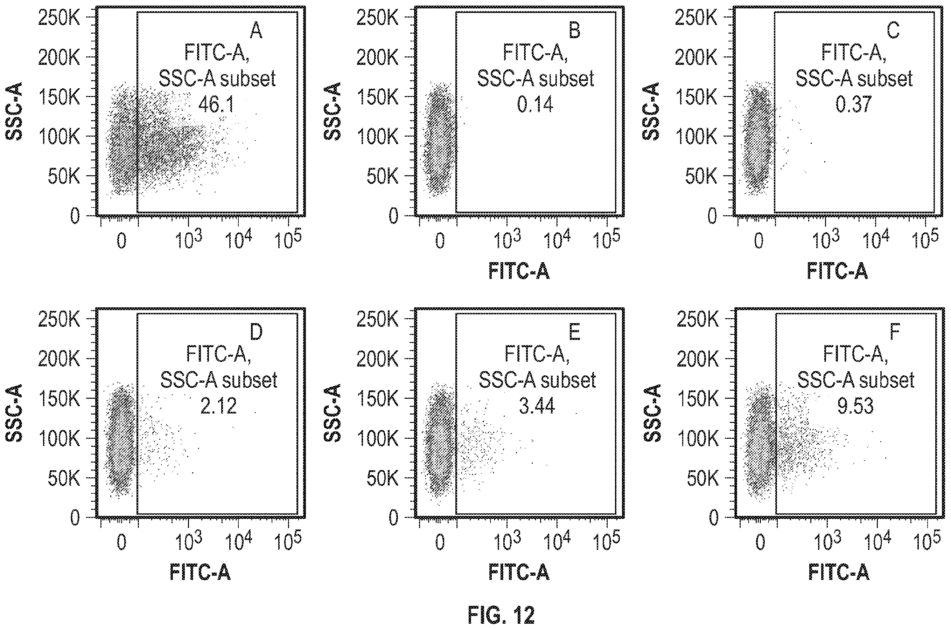

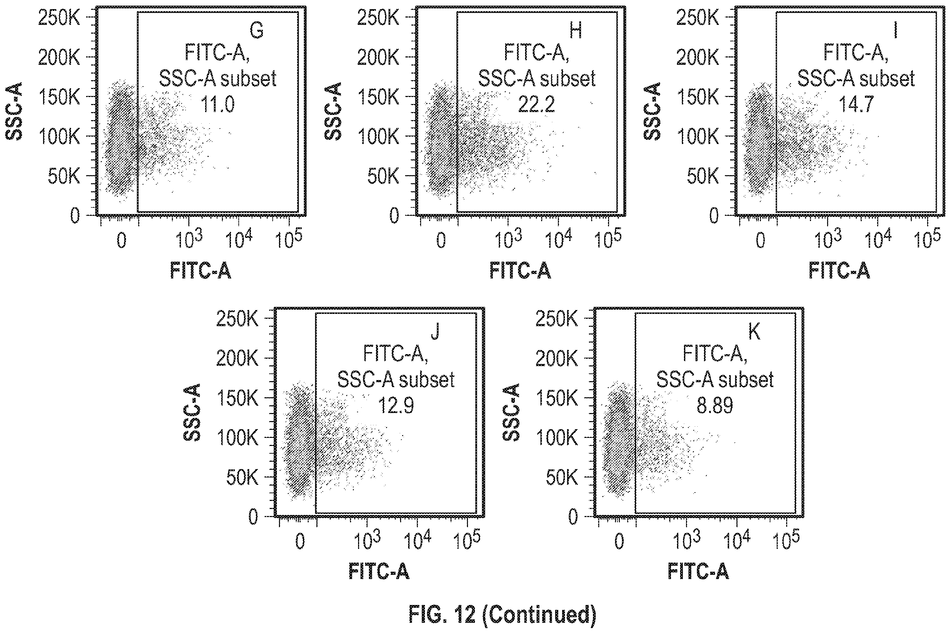

**Figure 12** provides dot plots obtained from fluorescence-activated cell sorting (FACS) evaluating green fluorescence (GFP) expression by 29T3-hASGR1 cells incubated with (A) wildtype AAV8 viral vectors only, (**C**) AA8-N590 -myc viral vectors only, or pAAV RC8 N590myc viral vectors mixed with bispecific anti-hASGR1-IgG4-Fc/anti-myc bispecific molecules at the following ratios (**D**) 1:1, (**E**) 1:2, (**F**) 1:4, (**G**) 1:8, (**H**) 1:12, (**I**) 1:15, (**J**) 1:50, or (**K**) 1:100. GFP expression by mock-transfected 29T3-hASGR1 cells is also shown (**B**). For each experiment, 2×10⁵ cells and 1×10⁹ viral vectors were used.

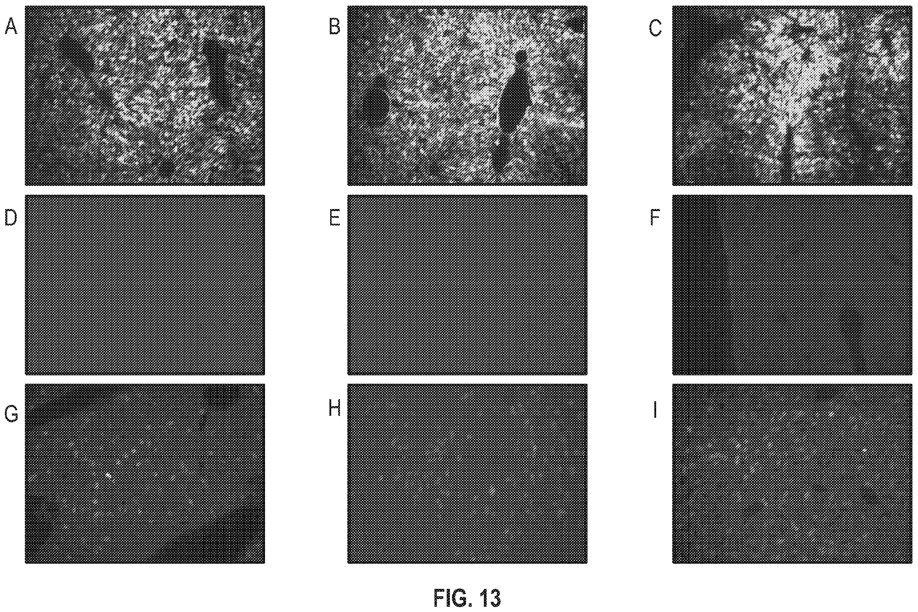

**Figure 13** provides immunofluorescence microscopy images of liver samples taken from C57BL/6 mice transgenically modified to express human ASGR1 by liver cells ten days post intravenous injection with **(A)-(C)** wildtype AAV8, **(D)-(F)** AA8-N590 -myc viral vectors and control bispecific binding molecule, or **(G)-(I)** AAV8 N590myc-CAGG-eGFP viral particles in combination with the anti-hASGR1-IgG4-Fc/anti-myc bispecific binding molecules.

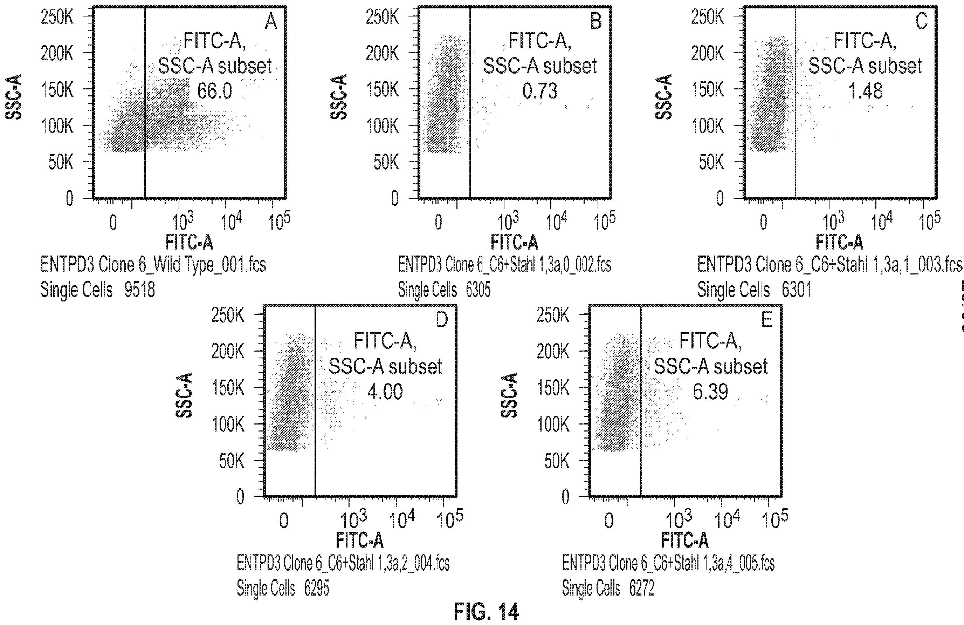

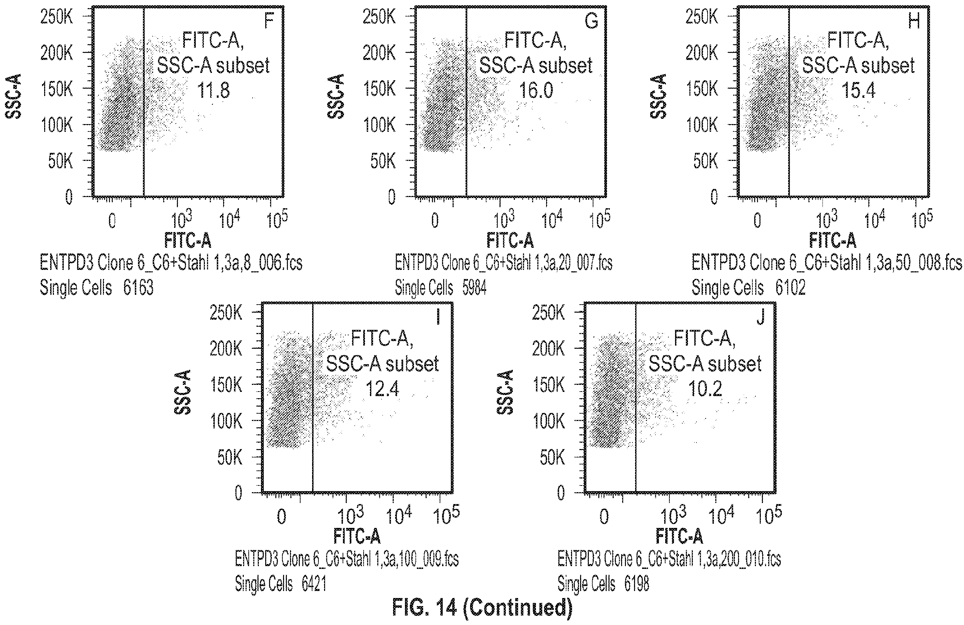

**Figure 14** provides dot plots obtained from fluorescence-activated cell sorting (FACS) evaluating green fluorescence (GFP) expression by 29T3-h ENTPD3 cells incubated with (**A**) wildtype AAV2 viral vectors only, (**B**) AAV2-N587Myc-CAGG-eGFP viral vectors only, or AAV2-N587Myc-CAGG-eGFP viral vectors mixed with bispecific anti-hENTPD3-IgG4-Fc/anti-myc bispecific molecules at the following ratios (**C**) 1:1, (**D**) 1:2, (**E**) 1:4, (**F**) 1:8, (**G**) 1:20, (**H**) 1:50, (**I**) 1:100, or (**K**) 1:200. For each experiment, 2×10⁵ cells and 1×10⁹ viral vectors were used

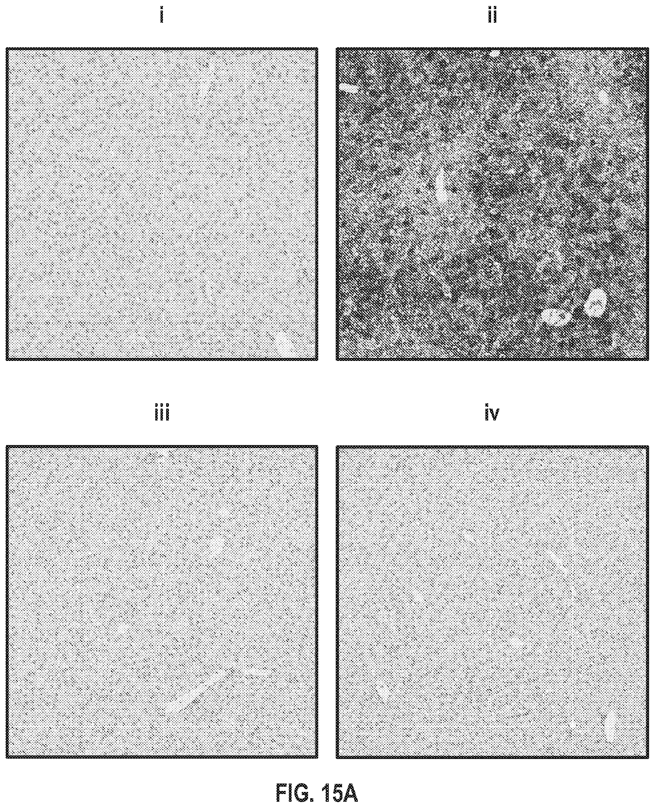

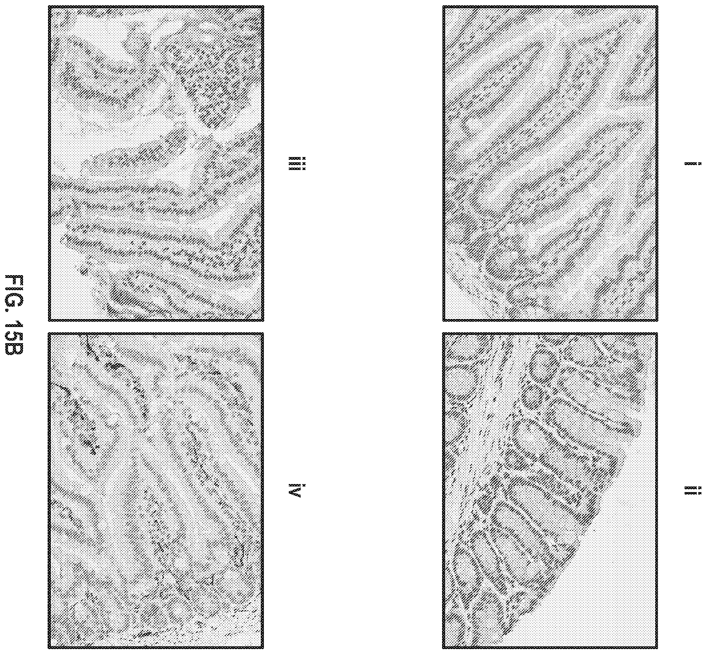

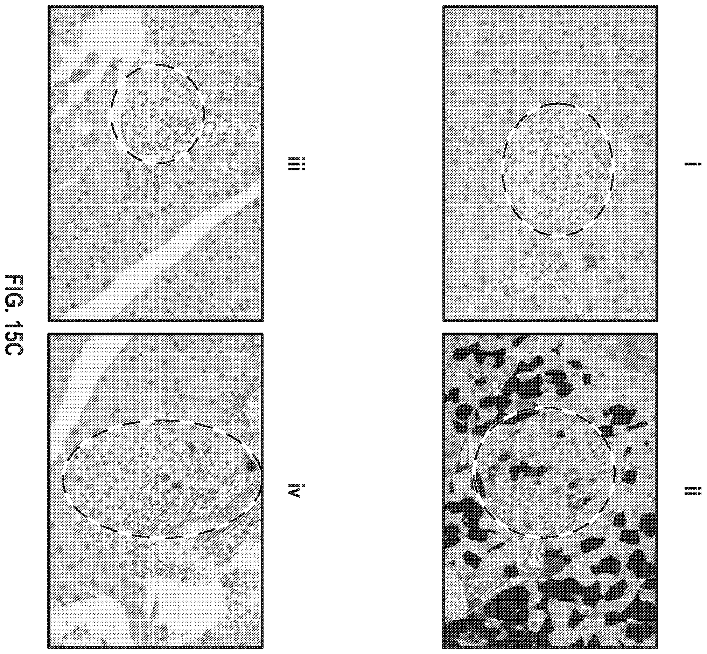

Figure 15A provides immunofluorescence microscopy images of liver samples, Figure 15B provides immunofluorescence microscopy images of intestine samples, and Figure 15C provides immunofluorescence microscopy images of pancreas samples. All samples are taken from wildtype C57BL/6 mice ten days post intravenous injection with PBS **(15A(i), 15B(i) and 15C(i))**, 5×10¹¹ wildtype AAV9 **(15A(ii), 15B(ii)**, and **15C(ii)),** 5×10¹¹ AAV2-N587myc-CAGG-eGFP viral vectors with 1×10³ irrelevant bispecific IgG4-Fc/anti-myc binding proteins **(15A(iii), 15B(iii)**, and **15C(iii)),** or 5×10¹¹ AAV2-N587myc-CAGG-eGFP viral vectors with 1×10¹³ bispecific hENTPD3-IgG4-Fc/anti-myc binding proteins **(15A(iv), 15B(iv)** and **15C(iv)).**

### DETAILED DESCRIPTION

A common problem with the adaptor approaches utilizing non-modified viral capsids or scaffold modified viral capsids has been the suboptimal transduction efficiencies of the modified capsids. (Grifman et al. (2001) Mol. Ther. 3:964-75). For example, Curiel et al. describe the generation and characterization of recombinant adenoviral vector containing fibers with an RGD-4C sequence genetically incorporated within the HI loop of the carboxy terminal knob domain and demonstrate the utility of the HI loop of the fiber knob as an optimal site for incorporation of short peptide ligands. *See, e.g.,* U.S. Patent No. 7,297,542; *see also* Beatty and Curiel (2012) Adv. Cancer. Res. 115:39-67. Similarly, insertion of ligand peptides into AAV capsid proteins resulted in capsids that were able to display the ligand on the surface of the capsid and mediate transduction through the interaction of the ligand with its receptor thereby redirecting viral tropism by genetic capsid modifications (Girod et al. (1999) Nat. Med. 5(9):1052-6, 1438 (errata included) (1999); Grifman et al. (2001) Mol. Ther. 3(6):964-75; Nicklin et al. (2001) Mol. Ther. 4(3):174-81; Shi et al. (2001) Hum Gene Ther. 17(3):353-61 (2006); Wu et al. (2000) J. Virol. 74(18):8635-47). In particular, it has been demonstrated that the insertion of an integrin binding Arg-Gly-Asp (RGD) motif at the insertion site I-587 of the AAV capsid protein VP1 enabled AAV viral vectors to transduce cells via αᵥβ₁ integrins (Girod et al. (1999) *supra*). In contrast, although the insertion of the 14-amino-acid peptide L14 after amino acid R447 (I-447) led to capsids still recognized by the conformation-sensitive antibody A20, such recombinant viral vectors were unable to transduce cells expressing the L-14 receptor, (Girod et al., 1999; cf Wu et al. (2000) (reporting the insertion of a hemagglutinin (HA) peptide at the position I-447and successful transduction of cells expressing HA peptide). Insertion of a Myc epitope between T448 and N449 was recognized by an anti-Myc antibody and was therefore present on the surface of the capsid, but led to inactivated viral vectors. (Grifman et al., 2001). In contrast, successful retargeting was again reported for the insertion of an NGR motif after N587, but not insertion of c-myc after N587. (Grifman et al., 2001). U.S. Patent No. 9,624,274 describes I-453 of an AAV capsid protein as a suitable insertion site for a heterologous epitope. Although these studies demonstrate the successful insertion and display of a heterologous peptide, e.g., epitope, by AAV capsid proteins, none of these studies provide any expectation that a multispecific binding molecule, e.g., a bispecific binding molecule such as a bispecific antibody, that specifically binds the heterologous peptide and a cell surface protein, can retarget the modified viral vectors toward cells expressing the cell surface protein and restore their transduction efficiencies.

Disclosed herein are recombinant AAV capsid proteins that are modified with a heterologous epitope, which can be used in connection with a multispecific binding molecule that comprises a paratope, e.g., Fv domain, that specifically binds the epitope and a ligand that binds a receptor expressed on the surface of a target cell. As shown herein, contacting AAV vectors having capsids formed with the AAV capsid proteins described herein with a multispecific binding molecule at certain ratios restores the transduction efficiency of the AAV capsid to levels comparable to wildtype virus, *see, e.g.,* Example 2. Capsid proteins modified with heterologous epitopes as described herein are derived from adenovirus (Ad) and adeno-associated virus (AAV).

While the invention has been particularly shown and described with reference to a number of embodiments, it would be understood by those skilled in the art that the various embodiments disclosed herein are not intended to act as limitations on the scope of the claims.

Although any methods and materials similar or equivalent to those described herein can be used in the practice or testing of the present invention, some preferred methods and materials are now described. Unless defined otherwise, all technical and scientific terms used herein have the same meaning as commonly understood by one of ordinary skill in the art to which this invention belongs.

### Definitions

Unless defined otherwise, all technical and scientific terms used herein have the same meaning as commonly understood by one of ordinary skill in the art to which this invention belongs.

Singular forms "a", "an", and "the" include plural references unless the context clearly dictates otherwise. Thus, for example, a reference to "a method" includes one or more methods, and/or steps of the type described herein and/or which will become apparent to those persons skilled in the art upon reading this disclosure.

The term "antibody" includes immunoglobulin molecules comprising four polypeptide chains, two heavy (H) chains and two light (L) chains inter-connected by disulfide bonds. Each heavy chain comprises a heavy chain variable domain (V_{H}) and a heavy chain constant region (C_{H}). The heavy chain constant region comprises at least three domains, C_{L}1, C_{H}2, C_{H}3 and optionally CH₄. Each light chain comprises a light chain variable domain (C_{H}) and a light chain constant region (C_{L}). The heavy chain and light chain variable domains can be further subdivided into regions of hypervariability, termed complementarity determining regions (CDR), interspersed with regions that are more conserved, termed framework regions (FR). Each heavy and light chain variable domain comprises three CDRs and four FRs, arranged from amino-terminus to carboxy-terminus in the following order: FR1, CDR1, FR2, CDR2, FR3, CDR3, FR4 (heavy chain CDRs may be abbreviated as HCDR1, HCDR2 and HCDR3; light chain CDRs may be abbreviated as LCDR1, LCDR2 and LCDR3. Typical tetrameric antibody structures comprise two identical antigen-binding domains, each of which formed by association of the V_{H} and V_{L} domains, and each of which together with respective C_{H} and C_{L} domains form the antibody Fv region. Single domain antibodies comprise a single antigen-binding domain, e.g., a V_{H} or a V_{L}. The term "antibody" encompasses monoclonal antibodies, multispecific (e.g., bispecific) antibodies, human antibodies, humanized antibodies, chimeric antibodies, singlechain Fvs (scFv), single chain antibodies, Fab fragments, F(ab') fragments, disulfide-linked Fvs (sdFv), intrabodies, minibodies, diabodies and anti-idiotypic (anti-Id) antibodies (including, e.g., anti-Id antibodies to antigen-specific TCR), and epitope-binding fragments of any of the above. The terms "antibody" and "antibodies" also refer to covalent diabodies such as those disclosed in U.S. Pat. Appl. Pub. 2007/0004909 and Ig-DARTS such as those disclosed in U.S. Pat. Appl. Pub. 2009/0060910. Antibodies include immunoglobulin molecules and immunologically active fragments of immunoglobulin molecules, i.e., molecules that contain an antigen binding site. Immunoglobulin molecules can be of any type (e.g., IgG, IgE, IgM, IgD, IgA and IgY), class (e.g., IgG1, IgG2, IgG3, IgG4, IgA1 and IgA2) or subclass.

The antigen-binding domain of an antibody, e.g., the part of an antibody that recognizes and binds to the epitope of an antigen, is also referred to as a "paratope." It is a small region (of 5 to 10 amino acids) of an antibody's Fv region, part of the fragment antigen-binding (Fab region), and may contains parts of the antibody's heavy and/or light chains. A paratope specifically binds an epitope when the paratope binds the epitope with a high affinity. The term "high affinity" antibody refers to an antibody that has a K_{D} with respect to its target epitope about of 10⁻⁹ M or lower (*e.g*., about 1 × 10⁻⁹ M, 1 × 10⁻¹⁰ M, 1 × 10⁻¹¹ M, or about 1 × 10⁻¹² M). In one embodiment, K_{D} is measured by surface plasmon resonance, *e.g*., BIACORE^{™}; in another embodiment, K_{D} is measured by ELISA.

The phrase "complementarity determining region," or the term "CDR," includes an amino acid sequence encoded by a nucleic acid sequence of an organism's immunoglobulin genes that normally (i.e., in a wild-type animal) appears between two framework regions in a variable region of a light or a heavy chain of an immunoglobulin molecule (e.g., an antibody or a T cell receptor). A CDR can be encoded by, for example, a germ line sequence or a rearranged or unrearranged sequence, and, for example, by a naive or a mature B cell or a T cell. A CDR can be somatically mutated (e.g., vary from a sequence encoded in an animal's germ line), humanized, and/or modified with amino acid substitutions, additions, or deletions. In some circumstances (e.g., for a CDR3), CDRs can be encoded by two or more sequences (e.g., germ line sequences) that are not contiguous (e.g., in an unrearranged nucleic acid sequence) but are contiguous in a B cell nucleic acid sequence, e.g., as the result of splicing or connecting the sequences (e.g., V-D-J recombination to form a heavy chain CDR3).

An "epitope" is the part of a macromolecule that is recognized by the immune system, specifically by antibodies, B cells, or cytotoxic T cells. Although epitopes are usually thought to be derived from nonself proteins, sequences derived from the host that can be recognized are also classified as epitopes. Epitopes have a length of at least 4 amino acids, preferably 4 to 30 amino acids, more preferably 5 to 20 amino acids, especially 5 to 15 amino acids. Epitopes can be linear or three-dimensional formed typically by amino acids that are distant from each other in the primary protein structure but become closely related in a secondary and/or tertiary structure. Epitopes that are specifically recognized by B cells are referred to as B-cell epitopes.

The phrase "Inverted terminal repeat" or "ITR" includes symmetrical nucleic acid sequences in the genome of adeno-associated viruses required for efficient replication. ITR sequences are located at each end of the AAV DNA genome. The ITRs serve as the origins of replication for viral DNA synthesis and are essential cis components for generating AAV integrating vectors.

The phrase "light chain" includes an immunoglobulin light chain sequence from any organism, and unless otherwise specified includes human κ and λ light chains and a VpreB, as well as surrogate light chains. Light chain variable domains typically include three light chain CDRs and four framework (FR) regions, unless otherwise specified. Generally, a full-length light chain includes, from amino terminus to carboxyl terminus, a variable domain that includes FR1-CDR1-FR2-CDR2-FR3-CDR3-FR4, and a light chain constant region. A light chain variable domain is encoded by a light chain variable region gene sequence, which generally comprises V_{L} and J_{L} segments, derived from a repertoire of V and J segments present in the germ line. Sequences, locations and nomenclature for V and J light chain segments for various organisms can be found in IMGT database, www.imgt.org. Light chains include those, e.g., that do not selectively bind either a first or a second epitope selectively bound by the epitope-binding protein in which they appear. Light chains also include those that bind and recognize, or assist the heavy chain or another light chain with binding and recognizing, one or more epitopes selectively bound by the epitope-binding protein in which they appear. Common or universal light chains include those derived from a human Vκ1-39Jκ gene or a human Vκ3-20Jκ gene, and include somatically mutated (e.g., affinity matured) versions of the same. Exemplary human V_{L} segments include a human Vκ1-39 gene segment, a human Vκ3-20 gene segment, a human Vλ1-40 gene segment, a human Vλ1-44 gene segment, a human Vλ2-8 gene segment, a human Vλ2-14 gene segment, and human Vλ3-21 gene segment, and include somatically mutated (e.g., affinity matured) versions of the same. Light chains can be made that comprise a variable domain from one organism (e.g., human or rodent, e.g., rat or mouse; or bird, e.g., chicken) and a constant region from the same or a different organism (e.g., human or rodent, e.g., rat or mouse; or bird, e.g., chicken).

The term "about" or "approximately" includes being within a statistically meaningful range of a value. Such a range can be within an order of magnitude, preferably within 50%, more preferably within 20%, still more preferably within 10%, and even more preferably within 5% of a given value or range. The allowable variation encompassed by the term "about" or "approximately" depends on the particular system under study, and can be readily appreciated by one of ordinary skill in the art.

The term "affinity tag" includes a polypeptide sequence that is a member of a specific binding pair, e.g., that specifically binds with another polypeptide sequence, e.g., an antibody paratope, with high affinity. Exemplary and non-limiting affinity tags include hexahistidine tag, FLAG tag, Strep II tag, streptavidin-binding peptide (SBP) tag, calmodulinbinding peptide (CBP), glutathione S-transferase (GST), maltose-binding protein (MBP), S-tag, HA tag, and c-Myc tag. (Reviewed in Zhao et al. (2013) J. Analytical Meth. Chem. 1-8).

The term "capsid protein" includes a protein that is part of the capsid of the virus. For adeno-associated viruses, the capsid proteins are generally referred to as VP1, VP2 and/or VP3, and each are encoded by a single *cap* gene. For AAV, the three AAV capsid proteins are produced in an overlapping fashion from the *cap* open reading frame (ORF) via alternative mRNA splicing and/or alternative translational start codon usage, although all three proteins use a common stop codon. Warrington et al. (2004) J. Virol. 78:6595. VP1 of AAV2 is generally translated from an ATG start codon (amino acid M1) on a 2.4-kb mRNA, while VP2 and VP3 of AAV2 arise from a smaller 2.3-kb mRNA, using a weaker ACG start codon for VP2 production (amino acid T138) and readthrough translation to the next available ATG codon (amino acid M203) for the production of the most abundant capsid protein, VP3. *Warrington, supra;* Rutledge et al. (1998) J. Virol. 72:309-19. The amino acid sequences of capsid proteins of adeno-associated viruses are well-known in the art and generally conserved, particularly upon the dependoparvoviruses. *See, Rutledge et al., supra.* For example, *Rutledge et al.* (1998), *supra,* provides at Figure 4B amino acid sequence alignments for VP1, VP2, and VP3 capsid proteins of AAV2, AAV3, AAV4 and AAV6, wherein the start sites for each of the VP1, VP2, and VP3 capsid proteins are indicated by arrows and the variable domains are boxed. Accordingly, although amino acid positions provided herein may be provided in relation to the VP1 capsid protein of the AAV, and amino acid positions provided herein that are not further specified refer to the AAV2 sequence of the major coat protein VP1 set forth as SEQ ID NO:1, a skilled artisan would be able to respectively and readily determine the position of that same amino acid within the VP2 and/or VP3 capsid protein of the AAV, and the corresponding position of amino acids among different serotypes. Additionally, a skilled artisan would be able to swap domains between capsid proteins of a different AAV serotypes for the formation of a "chimeric capsid protein."

Domain swapping between two AAV capsid protein constructs for the generation of a "chimeric AAV capsid protein" has been described, *see, e.g.,* Shen et al. (2007) Mol. Therapy 15(11):1955-1962. A "chimeric AAV capsid protein" includes an AAV capsid protein that comprises amino acid sequences, e.g., domains, from two or more different AAV serotypes and that is capable of forming and/or forms an AAV-like viral capsid/viral particle. A chimeric AAV capsid protein is encoded by a chimeric AAV capsid gene, e.g., a nucleotide comprising a plurality, e.g., at least two, nucleic acid sequences, each of which plurality is identical to a portion of a capsid gene encoding a capsid protein of distinct AAV serotypes, and which plurality together encodes a functional chimeric AAV capsid protein. Reference to a chimeric capsid protein in relation to a specific AAV serotype indicates that the capsid protein comprises one or more domains from a capsid protein of that serotype and one or more domains from a capsid protein of a different serotype. For example, an AAV2 chimeric capsid protein includes a capsid protein comprising one or more domains of an AAV2 VP1, VP2, and/or VP3 capsid protein and one or more domains of a VP1, VP2, and/or VP3 capsid protein of a different AAV.

A "mosaic capsid" comprises at least two sets of VP1, VP2, and/or VP3 proteins, each set of which is encoded by a different *cap* gene.

In some embodiments, a mosaic capsid described herein comprises recombinant VP1, VP2, and/or VP3 proteins encoded by a *cap* gene genetically modified with an insertion of a nucleic acid sequence encoding a heterologous epitope, and further comprises VP1, VP2, and/or VP3 proteins encoded by a reference *cap* gene, e.g., a wildtype reference *cap* gene encoding the wildtype VP1, VP2, and/or VP3 proteins of the same AAV serotype as the recombinant VP1, VP2, and/or VP3 proteins, a control reference *cap* gene encoding VP1, VP2, and/or VP3 proteins identical to the recombinant VP1, VP2, and VP3 proteins but for the absence of the heterologous epitope, a mutated wildtype reference *cap* gene encoding substantially wildtype VP1, VP2, and/or VP3 proteins of the same AAV serotype as the recombinant VP1, VP2, and/or VP3 proteins but for a mutation (e.g., insertion, substitution, deletion), which mutation preferably reduces the tropism of the wildtype VP1, VP2, and VP3 proteins. In some embodiments, the reference capsid protein is a chimeric reference protein comprising at least one domain of VP1, VP2, and/or VP3 proteins of the same AAV serotype as the recombinant VP1, VP2, and/or VP3 proteins. In some embodiments, the reference *cap* gene encodes a chimeric VP1, VP2, and/or VP3 protein.

The phrase "heavy chain," or "immunoglobulin heavy chain" includes an immunoglobulin heavy chain sequence, including immunoglobulin heavy chain constant region sequence, from any organism. Heavy chain variable domains include three heavy chain CDRs and four FR regions, unless otherwise specified. Fragments of heavy chains include CDRs, CDRs and FRs, and combinations thereof. A typical heavy chain has, following the variable domain (from N-terminal to C-terminal), a C_{H}1 domain, a hinge, a C_{H}2 domain, and a C_{H}3 domain. A functional fragment of a heavy chain includes a fragment that is capable of specifically recognizing an epitope (*e.g*., recognizing the epitope with a K_{D} in the micromolar, nanomolar, or picomolar range), that is capable of expressing and secreting from a cell, and that comprises at least one CDR. Heavy chain variable domains are encoded by variable region nucleotide sequence, which generally comprises V_{H}, D_{H}, and J_{H} segments derived from a repertoire of V_{H}, D_{H}, and J_{H} segments present in the germline. Sequences, locations and nomenclature for V, D, and J heavy chain segments for various organisms can be found in IMGT database, which is accessible via the internet on the world wide web (www) at the URL "imgt.org."

The term "heavy chain only antibody," "heavy chain only antigen binding protein," "single domain antigen binding protein," "single domain binding protein" or the like refers to a monomeric or homodimeric immunoglobulin molecule comprising an immunoglobulin-like chain comprising a variable domain operably linked to a heavy chain constant region, that is unable to associate with a light chain because the heavy chain constant region typically lacks a functional C_{H}1 domain. Accordingly, the term "heavy chain only antibody," "heavy chain only antigen binding protein," "single domain antigen binding protein," "single domain binding protein" or the like encompasses a both (i) a monomeric single domain antigen binding protein comprising one of the immunoglobulin-like chain comprising a variable domain operably linked to a heavy chain constant region lacking a functional C_{H}1 domain, or (ii) a homodimeric single domain antigen binding protein comprising two immunoglobulin-like chains, each of which comprising a variable domain operably linked to a heavy chain constant region lacking a functional C_{H}1 domain. In various aspects, a homodimeric single domain antigen binding protein comprises two identical immunoglobulin-like chains, each of which comprising an identical variable domain operably linked to an identical heavy chain constant region lacking a functional C_{H}1 domain. Additionally, each immunoglobulin-like chain of a single domain antigen binding protein comprises a variable domain, which may be derived from heavy chain variable region gene segments (e.g., V_{H}, D_{H}, J_{H}), light chain gene segments (e.g., V_{L}, J_{L}), or a combination thereof, linked to a heavy chain constant region (C_{H}) gene sequence comprising a deletion or inactivating mutation in a C_{H}1 encoding sequence (and, optionally, a hinge region) of a heavy chain constant region gene, e.g., IgG, IgA, IgE, IgD, or a combination thereof. A single domain antigen binding protein comprising a variable domain derived from heavy chain gene segments may be referred to as a "V_{H}- single domain antibody" or "V_{H}-single domain antigen binding protein", *see, e.g.,* U.S. Patent No. 8,754,287; U.S. Patent Publication Nos. 20140289876; 20150197553; 20150197554; 20150197555; 20150196015; 20150197556 and 20150197557. A single domain antigen binding protein comprising a variable domain derived from light chain gene segments may be referred to as a or "V_{L}-single domain antigen binding protein," *see, e.g.,* U.S. Publication No. 20150289489.

The phrase "light chain" includes an immunoglobulin light chain sequence from any organism, and unless otherwise specified includes human kappa (κ) and lambda (λ) light chains and a VpreB, as well as surrogate light chains. Light chain variable domains typically include three light chain CDRs and four framework (FR) regions, unless otherwise specified. Generally, a full-length light chain includes, from amino terminus to carboxyl terminus, a variable domain that includes FR1-CDR1-FR2-CDR2-FR3-CDR3-FR4, and a light chain constant region amino acid sequence. Light chain variable domains are encoded by the light chain variable region nucleotide sequence, which generally comprises light chain V_{L} and light chain J_{L} gene segments, derived from a repertoire of light chain V and J gene segments present in the germline. Sequences, locations and nomenclature for light chain V and J gene segments for various organisms can be found in IMGT database, which is accessible via the internet on the world wide web (www) at the URL "imgt.org." Light chains include those, *e.g.,* that do not selectively bind either a first or a second epitope selectively bound by the epitope-binding protein in which they appear. Light chains also include those that bind and recognize, or assist the heavy chain with binding and recognizing, one or more epitopes selectively bound by the epitope-binding protein in which they appear. Light chains also include those that bind and recognize, or assist the heavy chain with binding and recognizing, one or more epitopes selectively bound by the epitope-binding protein in which they appear. Common or universal light chains include those derived from a human Vκ1-39Jκ5 gene or a human Vκ3-20Jκ1 gene, and include somatically mutated (*e.g*., affinity matured) versions of the same.

The phrase "operably linked", as used herein, includes a physical juxtaposition (e.g., in three-dimensional space) of components or elements that interact, directly or indirectly with one another, or otherwise coordinate with each other to participate in a biological event, which juxtaposition achieves or permits such interaction and/or coordination. To give but one example, a control sequence (e.g., an expression control sequence) in a nucleic acid is said to be "operably linked" to a coding sequence when it is located relative to the coding sequence such that its presence or absence impacts expression and/or activity of the coding sequence. In many embodiments, "operable linkage" involves covalent linkage of relevant components or elements with one another. Those skilled in the art will readily appreciate, however, that in some embodiments, covalent linkage is not required to achieve effective operable linkage. For example, in some embodiments, nucleic acid control sequences that are operably linked with coding sequences that they control are contiguous with the nucleotide of interest. Alternatively, or additionally, in some embodiments, one or more such control sequences acts in trans or at a distance to control a coding sequence of interest. In some embodiments, the term "expression control sequence" as used herein refers to polynucleotide sequences which are necessary and/or sufficient to affect the expression and processing of coding sequences to which they are ligated. In some embodiments, expression control sequences may be or comprise appropriate transcription initiation, termination, promoter and/or enhancer sequences; efficient RNA processing signals such as splicing and polyadenylation signals; sequences that stabilize cytoplasmic mRNA; sequences that enhance translation efficiency (e.g., Kozak consensus sequence); sequences that enhance protein stability; and/or, in some embodiments, sequences that enhance protein secretion. In some embodiments, one or more control sequences are preferentially or exclusively active in a particular host cell or organism, or type thereof. To give but one example, in prokaryotes, control sequences typically include promoter, ribosomal binding site, and transcription termination sequence; in eukaryotes, in many embodiments, control sequences typically include promoters, enhancers, and/or transcription termination sequences. Those of ordinary skill in the art will appreciate from context that, in many embodiments, the term "control sequences" refers to components whose presence is essential for expression and processing, and in some embodiments, includes components whose presence is advantageous for expression (including, for example, leader sequences, targeting sequences, and/or fusion partner sequences).

The term "recombinant capsid protein" includes a capsid protein that has at least one mutation in comparison to the corresponding capsid protein of the wild-type virus, which may be a reference and/or control virus for comparative study. A recombinant capsid protein includes a capsid protein that comprises a heterologous epitope, which may be inserted into and/or displayed by the capsid protein. "Heterologous" in this context means heterologous as compared to the virus, from which the capsid protein is derived. The inserted amino acids can simply be inserted between two given amino acids of the capsid protein. An insertion of amino acids can also go along with a deletion of given amino acids of the capsid protein at the site of insertion, e.g. 1 or more capsid protein amino acids are substituted by 5 or more heterologous amino acids).

The terms "multispecific binding molecule" and "bispecific binding molecule," and the like generally and respectively refer to a binding molecule comprising at least two and only two nonidentical binding components, with each binding component specifically binding a different epitope-either on two different molecules (e.g., different epitopes on two different immunogens) or on the same molecule (e.g., different epitopes on the same immunogen). Generally, one of the binding components of a bispecific binding molecule herein specifically binds a heterologous epitope displayed by a AAV capsid protein and the second binding component is specific for a protein, e.g., a cell surface marker, expressed primarily and/or preferentially by a target cell, e.g., a T cell marker (e.g., CD3, CD28, etc.). Bispecific binding molecules may be made, for example, by combining binding components that recognize different epitopes of the same immunogen. For example, nucleic acid sequences encoding binding components (e.g., light or heavy chain variable sequences) that recognize different epitopes can be fused to nucleic acid sequences encoding the same or different heavy chain constant regions, the same or different light chain constant regions, or respectively a heavy chain constant region and a light chain constant region, and such sequences can be expressed in a cell as a multispecific antigen-binding protein in a format that is similar to a Fab structure, scFab structure, a diabody structure, an scFv structure, an scFv-Fc structure, an scFv-zipper structure, a tetrameric structure similar to a typical antibody that includes the cognate universal light chain, a tetrameric structure comprising a typical bivalent antibody that includes the cognate universal light chain and/or an additional binding component (e.g., scFv, scFv-zipper, scFab, etc.) appended to one or both of the heavy chains (e.g., at the N- and/or C-terminus) and/or to one or both of the light chains (e.g., at the N- and/or C-terminus). The various formats of multispecific, particularly bispecific, binding molecules are well-known, *see e.g.,* Brinkmann and Konterman (2017) mAbs 9:182-212.

An exemplary multispecific binding molecule has two heavy chains each having heavy chain CDRs, followed by (N-terminal to C-terminal) a C_{H}1 domain, a hinge, a C_{H}2 domain, and a C_{H}3 domain, and an immunoglobulin light chain that either does not confer epitope-binding specificity but that can associate with each heavy chain (e.g., common light chain), or that can associate with each heavy chain and that can bind one or more of the epitopes bound by the heavy chain epitope-binding regions, or that can associate with each heavy chain and enable binding of one or both of the heavy chains to one or both epitopes. In some embodiments, a multispecific binding molecule comprises: (1) an immunoglobulin heavy chain variable domain operably linked to a first heavy chain constant region comprising a first CH3 amino acid sequence of a human IgG selected from IgG1, IgG2, IgG4, and a combination thereof; and (2) a immunoglobulin heavy chain variable domain, wherein the second immunoglobulin heavy chain variable domain is operably linked to a second heavy chain constant region comprising a second CH3 amino acid sequence of the human IgG selected from IgG1, IgG2, IgG4, and a combination thereof, wherein the first or the second heavy chain variable domains (with or without a cognate light chain) binds a heterologous epitope as described herein and the other heavy chain variable domain (with or without a cognate light chain) binds a receptor on a target cell, and wherein the first heavy chain constant region associates with the second constant chain region in a manner that provides for easier isolation of the multispecific binding protein, e.g., wherein the first and second heavy chain constant regions form a knobs-into-hole (KIH) format or wherein the second CH3 amino acid sequence comprises a modification that reduces or eliminates binding for the second CH3 amino acid sequence to Protein A (see, for example, US Pat. No. 8,586,713). In some embodiments, a multispecific binding molecule comprises: (1) an immunoglobulin heavy chain variable domain operably linked to a first heavy chain constant region comprising a first CH3 amino acid sequence of a human IgG selected from IgG1, IgG2, IgG4, and a combination thereof; and (2) a immunoglobulin heavy chain variable domain, wherein the second immunoglobulin heavy chain variable domain is operably linked to a second heavy chain constant region comprising a second CH3 amino acid sequence of the human IgG selected from IgG1, IgG2, IgG4, and a combination thereof, wherein the first and the second heavy chain variable domains (with or without a cognate light chain) binds the same or different antigens, wherein the first or the second heavy chain constant regions are modified to further comprise an additional binding domain (e.g., an scFv or Fv that binds to a heterologous epitope as described herein, e.g., wherein the additional binding domain is appended to the C-terminus or N-terminus of one or both heavy chains), and wherein the first heavy chain constant region associates with the second constant chain region in a manner that provides for easier isolation of the multispecific binding protein, e.g., wherein the first and second heavy chain constant regions form a knobs-into-hole (KIH) format or wherein the second CH3 amino acid sequence comprises a modification that reduces or eliminates binding for the second CH3 amino acid sequence to Protein A. In some embodiments in which the second CH3 amino acid sequence comprises reduced or eliminated binding to Protein A, the second CH3 amino acid sequence comprises an H95R modification (by IMGT exon numbering; H435R by EU numbering). In one embodiment the second CH3 amino acid sequence further comprises an Y96F modification (by IMGT exon numbering; H436F by EU). In another embodiment, the second CH3 amino acid sequence comprises both an H95R modification (by IMGT exon numbering; H435R by EU numbering) and an Y96F modification (by IMGT exon numbering; H436F by EU). In some embodiments, the second CH3 amino acid sequence is from a modified human IgG1 and further comprises a mutation selected from the group consisting of D16E, L18M, N44S, K52N, V57M, and V82I (IMGT; D356E, L38M, N384S, K392N, V397M, and V422I by EU). In some embodiments, the second CH3 amino acid sequence is from a modified human IgG2 and further comprises a mutation selected from the group consisting of N44S, K52N, and V82I (IMGT: N384S, K392N, and V422I by EU). In some embodiments, the second CH3 amino acid sequence is from a modified human IgG4 and further comprises a mutation selected from the group consisting of Q15R, N44S, K52N, V57M, R69K, E79Q, and V82I (IMGT: Q355R, N384S, K392N, V397M, R409K, E419Q, and V422I by EU). In some embodiments, the heavy chain constant region amino acid sequence is a non-human constant region amino acid sequence, and the heavy chain constant region amino acid sequence comprises one or more of any of the types of modifications described above.

In various embodiments, Fc domains are modified to have altered Fc receptor binding, which in turn affects effector function. In some embodiments, an engineered heavy chain constant region (CH), which includes the Fc domain, is chimeric. As such, a chimeric CH region combines CH domains derived from more than one immunoglobulin isotype. For example, a chimeric CH region comprises part or all of a CH2 domain derived from a human IgG1, human IgG2 or human IgG4 molecule, combined with part or all of a CH3 domain derived from a human IgG1, human IgG2 or human IgG4 molecule. In some embodiments, a chimeric CH region contain a chimeric hinge region. For example, a chimeric hinge may comprise an "upper hinge" amino acid sequence (amino acid residues from positions 216 to 227 according to EU numbering; amino acid residues from positions 226 to 240 according to Kabat numbering) derived from a human IgG1, a human IgG2 or a human IgG4 hinge region, combined with a "lower hinge" sequence (amino acid residues from positions 228 to 236 according to EU numbering; amino acid positions from positions 241 to 249 according to Kabat numbering) derived from a human IgG1, a human IgG2 or a human IgG4 hinge region. In some embodiments, the chimeric hinge region comprises amino acid residues derived from a human IgG1 or a human IgG4 upper hinge and amino acid residues derived from a human IgG2 lower hinge.

In some embodiments, the Fc domain may be engineered to activate all, some, or none of the normal Fc effector functions, without affecting the Fc-containing protein's (e.g. antibody's) desired pharmacokinetic properties. For examples of proteins comprising chimeric CH regions and having altered effector functions, see WO2014022540.

The term "target cells" includes any cells in which expression of a nucleotide of interest is desired. Preferably, target cells exhibit a protein, e.g., a receptor, on their surface that allows the cell to be targeted with a retargeting ligand, as described below. Preferably, the targeted protein, e.g., receptor is specific for the target cell, e.g., is a "cell specific marker," "cell specific antigen," or the like. The term "cell specific marker," "cell specific antigen," "organ specific marker," "tissue specific marker," or the like refers to and includes those proteins for which expression is enriched by the cell, tissue and/or organ for which it is a specific marker. "Enriched" in the context of protein expression refers to and includes expression or overexpression of the cell/tissue/organ specific protein primarily, preferentially, or solely by the cell/tissue/organ for which the protein is a specific marker, although such marker may also be expressed by other cells/tissues/or organs at minimal levels. The Human Protein Atlas may be used to determine whether a protein is a cell/tissue/organ specific marker, and also provides a repository of cell/tissue/organ specific proteins. *See,* www.proteinatlas.org; *see also* Uhlen et al. (2010) Nat. Biotech. 28:1248-50.

The term "transduction" or "infection" or the like refers to the introduction of a nucleic acid into a target cell by a viral vector. The term efficiency in relation to transduction or the like, e.g., "transduction efficiency" refers to the fraction (e.g., percentage) of cells expressing a nucleotide of interest after incubation with a set number of viral vectors comprising the nucleotide of interest. Well-known methods of determining transduction efficiency include fluorescence activated cell sorting of cells transduced with a fluorescent reporter gene, PCR for expression of the nucleotide of interest, etc.

The term "wild-type", as used herein, includes an entity having a structure and/or activity as found in nature in a "normal" (as contrasted with mutant, diseased, altered, etc.) state or context. Those of ordinary skill in the art will appreciate that wildtype viral vectors, e.g., wildtype capsid proteins, may be used as reference viral vector in comparative studies. Generally, a reference viral capsid protein/capsid/vector are identical to the test viral capsid protein/capsid/vector but for the change for which the effect is to be tested. For example, to determine the effect, e.g., on transduction efficiency, of inserting a heterologous epitope into a test viral vector, the transduction efficiencies of the test viral vector (in the absence or presence of an appropriate multispecific binding molecule) can be compared to the transduction efficiencies of a reference viral vector (in the absence or presence of an appropriate multispecific binding molecule if necessary) which is identical to the test viral vector in every instance (e.g., additional mutations, nucleotide of interest, numbers of viral vectors and target cells, etc.) except for the presence of a heterologous epitope.

### Recombinant virus capsid proteins and AAV vectors and nucleic acids

In some embodiments, a recombinant AAV capsid protein as described herein is an Ad capsid protein, e.g., a capsid protein of an Ad serotype selected from the group consisting of Ad1, Ad2, Ad3, Ad4, Ad5, Ad6, and Ad7. In some embodiments, a recombinant AAV capsid protein is derived from an Ad2 capsid gene. In some embodiments, a recombinant AAV capsid protein is derived from an Ad5 capsid gene. In some embodiments, a recombinant Ad AAV capsid protein as described herein comprises a heterologous epitope in a fiber protein domain, e.g., at the carboxy terminus of the fiber protein, fiber knob, and/or HI loop of the fiber knob.

In some embodiments, a recombinant AAV capsid protein derived from an adeno-associated virus (AAV) capsid gene is a genetically modified capsid protein of an AAV serotype selected from the group consisting of AAV1, AAV2, AAV3, AAV4, AAV5, AAV6, AAV7, AAV8 and AAV9. In some embodiments, the recombinant AAV capsid protein is derived from an AAV2 capsid gene, an AAV6 capsid gene, an AAV8 capsid gene, or an AAV9 capsid gene. In some embodiments, the recombinant AAV capsid protein is derived from an AAV2 capsid gene, e.g., is a genetically modified AAV2 VP1 capsid protein, the amino acid sequence for the wildtype of which is set forth respectively as SEQ ID NO:1. In some embodiments, the recombinant AAV capsid protein is derived from an AAV8 capsid gene, e.g., is a genetically modified AAV8 VP1 capsid protein, the amino acid sequence for the wildtype of which is set forth as SEQ ID NO:21. In some embodiments the recombinant AAV capsid protein is derived from an AAV9 capsid gene, e.g., is a genetically modified AAV9 VP1 capsid protein, the amino acid sequence for the wildtype of which is set forth respectively as SEQ ID NO:5. In some embodiments, the recombinant AAV capsid protein is derived from an AAV6 capsid gene, e.g., is a genetically modified VP1 capsid protein of AAV6. In some embodiments, a heterologous epitope is inserted into I-453 of an AAV9 capsid protein.

Generally, a recombinant AAV capsid protein as described herein comprises a heterologous epitope inserted into and/or displayed by the AAV capsid protein such that the heterologous epitope reduces and/or abolishes the natural tropism of the AAV capsid protein or capsid comprising same. In some embodiments, the heterologous epitope is inserted into a region of the AAV capsid protein involved with the natural tropism of the wildtype reference capsid protein, e.g., a region of the capsid protein involved with a cell receptor. In some embodiments, the heterologous epitope is inserted into and/or displayed by a knob domain of an Ad fiber protein. In some embodiments, the heterologous epitope is inserted into and/or displayed by the HI loop of an Ad fiber protein. In some embodiments, the heterologous epitope is inserted after an amino acid position selected from the group consisting of G-453 of AAV2 capsid protein VP1, N-587 of AAV2 capsid protein VP1, Q-585 of AAV6 capsid protein VP1, G-453 of AAV9 capsid protein VP1, and A-589 of AAV9 capsid protein VP1. In some embodiments, the heterologous epitope is inserted and/or displayed between amino acids N-587 and R-588 of an AAV2 VP1 capsid. In some embodiments, a recombinant AAV capsid, AAV vector comprising a recombinant AAV capsid, and/or compositions comprising a recombinant AAV capsid comprises an amino acid sequence set forth as SEQ ID NO:2. In some embodiments, a recombinant AAV capsid, AAV vector comprising a recombinant AAV capsid, and/or compositions comprising a recombinant AAV capsid comprises an amino acid sequence set forth as SEQ ID NO:4. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:25. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:26. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:27. Additional suitable insertion sites identified by using AAV2 are well known in the art (Wu et al. (2000) J. Virol. 74:8635-8647) and include I-1, I-34, I-138, I-139, I-161, I-261, I-266, I-381, I-447, I-448, I-459, I-471, I-520, I-534, I-570, I-573, I-584, I-587, I-588, I-591, I-657, I-664, I-713 and I-716. A recombinant virus capsid protein as described herein may be an AAV2 capsid protein comprising a heterologous epitope inserted into a position selected from the group consisting of I-1, I-34, I-138, I-139, I-161, I-261, I-266, I-381, I-447, I-448, I-459, I-471, I-520, I-534, I-570, I-573, I-584, I-587, I-588, I-591, I-657, I-664, I-713, I-716, and a combination thereof. Additional suitable insertion sites identified by using additional AAV serotypes are well-known and include I-587 (AAV1), I-589 (AAV1), I-585 (AAV3), I-585 (AAV4), and I-585 (AAV5). In some embodiments, a recombinant AAV capsid protein as described herein may be an AAV2 capsid protein comprising a heterologous epitope inserted into a position selected from the group consisting of I-587 (AAV1), I-589 (AAV1), I-585 (AAV3), I-585 (AAV4), I-585 (AAV5), and a combination thereof.

The used nomenclature I-### herein refers to the insertion site with ### naming the amino acid number relative to the VP1 protein of an AAV capsid protein, however such the insertion may be located directly N- or C-terminal, preferably C-terminal of one amino acid in the sequence of 5 amino acids N- or C-terminal of the given amino acid, preferably 3, more preferably 2, especially 1 amino acid(s) N- or C-terminal of the given amino acid. Additionally, the positions referred to herein are relative to the VP1 protein encoded by an AAV capsid gene, and corresponding positions (and mutations thereof) may be easily identified for the VP2 and VP3 capsid proteins encoding by the capsid gene by performing a sequence alignment of the VP1, VP2 and VP3 proteins encoding by the reference AAV capsid gene.

Accordingly, an insertion into the corresponding position of the coding nucleic acid of one of these sites of the cap gene leads to an insertion into VP1, VP2 and/or VP3, as the capsid proteins are encoded by overlapping reading frames of the same gene with staggered start codons. Therefore, for AAV2, for example, according to this nomenclature insertions between amino acids 1 and 138 are only inserted into VP1, insertions between 138 and 203 are inserted into VP1 and VP2, and insertions between 203 and the C-terminus are inserted into VP1, VP2 and VP3, which is of course also the case for the insertion site I-587. Therefore, the present invention encompasses structural genes of AAV with corresponding insertions in the VP1, VP2 and/or VP3 proteins.

Additionally, due to the high conservation of at least large stretches and the large member of closely related family member, the corresponding insertion sites for AAV other than the enumerated AAV can be identified by performing an amino acid alignment or by comparison of the capsid structures. *See, e.g.,* Rutledge et al. (1998) J. Virol. 72:309-19 and U.S. Patent No. 9,624,274 for exemplary alignments of different AAV capsid proteins.

In some compositions disclosed herein consisting of the recombinant AAV capsid (e.g., in the absence of a multispecific binding molecule), the recombinant AAV capsid protein is an AAV2 capsid protein VP1 with a heterologous epitope is inserted at an 1587 site, wherein the heterologous epitope does not comprise an Arg-Gly-Asp (RGD) motif, an NGR motif, or c-myc. In some compositions disclosed herein consisting of the recombinant AAV capsid (e.g., in the absence of a multispecific binding molecule), the recombinant AAV capsid protein is a VP1 capsid protein with a heterologous epitope is inserted between T-448 and N-449, wherein the heterologous epitope does not comprise c-myc. In some compositions disclosed herein consisting of the recombinant AAV capsid (e.g., in the absence of a multispecific binding molecule), the recombinant AAV capsid protein is a VP1 capsid protein with a heterologous epitope is inserted at an I-447 site, wherein the heterologous epitope does not comprise L14 or HA.

In some compositions comprising the recombinant AAV capsid (e.g., further comprising a multispecific binding molecule), the recombinant AAV capsid protein is a VP1 capsid protein with a heterologous epitope is inserted at an I-587 site, wherein the heterologous epitope comprises an Arg-Gly-Asp (RGD) motif, an NGR motif, or c-myc. In some compositions disclosed herein comprising the recombinant AAV capsid (e.g., further comprising a multispecific binding molecule), the AAV capsid is a VP1 capsid, the heterologous epitope comprises c-myc, and the heterologous epitope is inserted between T-448 and N-449, or between N-587 and R-588. In some compositions disclosed herein comprising the recombinant AAV capsid (e.g., further comprising a multispecific binding molecule), the recombinant AAV capsid protein is a VP1 capsid protein with a heterologous epitope is inserted at an I-447 site, wherein the heterologous epitope comprises L14 or HA. In some compositions disclosed herein comprising the recombinant AAV capsid (e.g., in the presence of a multispecific binding molecule), the recombinant AAV capsid protein is a VP1 capsid protein with a heterologous epitope is inserted between T-448 and N-449, wherein the heterologous epitope comprises c-myc. U.S. Patent No. 9,624,274 describes I-453 of an AAV capsid protein as a suitable insertion site for a heterologous epitope.

In some embodiments, insertion (display) of the heterologous epitope abolishes the natural tropism of the AAV vector, e.g., transduction of a cell naturally permissive to infection by wildtype reference AAV vectors and/or a target cell is undetectable in the absence of an appropriate multispecific binding molecule. In some embodiments, insertion (display) of the heterologous epitope reduces the natural tropism of the AAV vector, e.g., compared to transduction of a cell naturally permissive to infection by wildtype reference AAV vectors. In some embodiments, the insertion (display) of the heterologous epitope reduces the natural tropism of the AAV vector by at least 5%. In some embodiments, the insertion (display) of the heterologous epitope reduces the natural tropism of the AAV vector by at least 5%. In some embodiments, the insertion (display) of the heterologous epitope reduces the natural tropism of the AAV vector by at least 10%. In some embodiments, the insertion (display) of the heterologous epitope reduces the natural tropism of the AAV vector by at least 20%. In some embodiments, the insertion (display) of the heterologous epitope reduces the natural tropism of the AAV vector by at least 30%. In some embodiments, the insertion (display) of the heterologous epitope reduces the natural tropism of the AAV vector by at least 40%. In some embodiments, the insertion (display) of the heterologous epitope reduces the natural tropism of the AAV vector by at least 50%. In some embodiments, the insertion (display) of the heterologous epitope reduces the natural tropism of the AAV vector by at least 60%. In some embodiments, the insertion (display) of the heterologous epitope reduces the natural tropism of the AAV vector by at least 70%. In some embodiments, the insertion (display) of the heterologous epitope reduces the natural tropism of the AAV vector by at least 80%. In some embodiments, the insertion (display) of the heterologous epitope reduces the natural tropism of the AAV vector by at least 90%. In some embodiments, the insertion (display) of the heterologous epitope reduces the natural tropism of the AAV vector by at least 95%. In some embodiments, the insertion (display) of the heterologous epitope reduces the natural tropism of the AAV vector by at least 90%. In these embodiments, wherein the insertion (display) of the heterologous epitope does not abolish the natural tropism of the recombinant AAV capsids, the natural tropism of such recombinant AAV capsids may be abolished by a second and different mutation. For example, in one embodiment, a recombinant AAV capsid protein as described herein may be derived from an AAV9 capsid gene, comprise a heterologous epitope, and may further comprise a mutation, e.g., a W503A mutation.

This detargeting of the virus from its natural host cell is important especially if systemic versus local or loco-regional administration of the AAV vectors is intended, as uptake of the AAV vectors by the natural host cells limits the effective dose of the AAV vectors. In the case of AAV2 and AAV6, HSPG is reported to be the primary receptor for viral uptake in a large number of cells, especially liver cells. For AAV2, HSPG-binding activity is dependent on a group of 5 basic amino acids, R484, R487, R585, R588 and K532 (Kern et al., (2003) J Virol. 77(20):11072-81). Recently it was reported that the lysine-to-glutamate amino acid substitution K531E leads to the suppression of AAV6's ability to bind heparin or HSPG ((Wu et al., 2006) J. of Virology 80(22):11393-11397). Accordingly, preferred point mutations are those that reduce the transducing activity of the AAV vector for a given target cell mediated by the natural receptor by at least 50%, preferably at least 80%, especially at least 95%, in the case of HSPG as the primary receptor the binding of the AAV vectors to HSPG.

Consequently, further mutations preferred for HSPG-binding AAV vectors are those mutations that deplete or replace a basic amino acid such as R, K or H, preferably R or K which is involved in HSPG binding of the respective virus, by a non-basic amino acid such as A, D, G, Q, S and T, preferably A or an amino acid that is present at the corresponding position of a different but highly conserved AAV serotype lacking such basic amino acid at this position. Consequently, preferred amino acid substitutions are R-484-A, R-487-A, R-487-G, K-532-A, K-532-D, R-585-A, R-585-S, R-585-Q, R-585-A or R-588-T, especially R-585-A and/or R-588-A for AAV2, and K-531-A or K-531-E for AAV6. One especially preferred embodiment of the invention are such capsid protein mutants of AAV2 that additionally contain the two-point mutations R-585-A and R-588-A as these two point mutations are sufficient to ablate HSPG binding activity to a large extent. These point mutations enable an efficient detargeting from HSPG-expressing cells which--for targeting purposes--increases specificity of the respective mutant virus for its new target cell.

One embodiment of the present invention is a multimeric structure comprising a recombinant AAV capsid protein of the present invention. A multimeric structure comprises at least 5, preferably at least 10, more preferably at least 30, most preferably at least 60 recombinant AAV capsid proteins comprising a heterologous epitope as described herein. They can form regular AAV capsids (empty AAV particles) or AAV vectors (capsids encapsulating a nucleotide of interest). The formation of AAV vectors capable of packaging a viral genome is a highly preferred feature for use of the recombinant AAV capsids described herein as AAV vectors.

One embodiment of the present invention is a nucleic acid encoding a capsid protein as described above. The nucleic acid is preferably a vector comprising the claimed nucleic acid sequence. Nucleic acids, especially vectors are necessary to recombinantly express the capsid proteins of this invention.

A further embodiment of the present invention is the use of at least one recombinant AAV capsid protein and/or a nucleic acid encoding same, preferably at least one multimeric structure (e.g., AAV vector) for the manufacture of and use as a gene transfer vector.

### Heterologous Epitopes

Generally, a recombinant AAV protein and/or an AAV vector comprising the recombinant AAV capsid comprises a heterologous epitope, which enables the retargeting of the AAV vector, e.g., via a multispecific binding molecule. In some embodiments, a heterologous epitope is a B cell epitope, e.g., is between about 1 amino acid and about 35 amino acids in length, and forms a binding pair with an antibody paratope, e.g., an immunoglobulin variable domain. In some embodiments, the heterologous epitope comprises an affinity tag.

A large number of tags are known in the art. (See, e.g.: Nilsson et al. (1997) "Affinity fusion strategies for detection, purification, and immobilization of recombinant proteins" Protein Expression and Purification 11: 1-16, Terpe et al. (2003) "Overview of tag protein fusions: From molecular and biochemical fundamentals to commercial systems" Applied Microbiology and Biotechnology 60:523-533, and references therein). Affinity tags include, but are not limited to, a polyhistidine tag (e.g., a His-6, His-8, or His-10 tag) that binds immobilized divalent cations (e.g., Ni²⁺), a biotin moiety (e.g., on an *in vivo* biotinylated polypeptide sequence) that binds immobilized avidin, a GST (glutathione S-transferase) sequence that binds immobilized glutathione, an S tag that binds immobilized S protein, an antigen that binds an immobilized antibody or domain or fragment thereof (including, e.g., T7, myc, FLAG, and B tags that bind corresponding antibodies), a FLASH Tag (a high affinity tag that couples to specific arsenic based moieties), a receptor or receptor domain that binds an immobilized ligand (or vice versa), protein A or a derivative thereof (e.g., Z) that binds immobilized IgG, maltose-binding protein (MBP) that binds immobilized amylose, an albumin-binding protein that binds immobilized albumin, a chitin binding domain that binds immobilized chitin, a calmodulin binding peptide that binds immobilized calmodulin, and a cellulose binding domain that binds immobilized cellulose. Another exemplary tag is a SNAP-tag, commercially available from Covalys (www.covalvs.com). In some embodiments, a heterologous epitope disclosed herein comprises an affinity tag recognized only by an antibody paratope. In some embodiments, a heterologous epitope disclosed herein comprises an affinity tag recognized by an antibody paratope and other specific binding pairs.

In some aspects, the heterologous epitope and/or affinity tag does not form a binding pair with an immunoglobulin constant domain. In some aspects, the heterologous epitope and/or affinity tag does not form a binding pair with a metal ion, e.g., Ni²⁺, Co²⁺, Cu²⁺, Zn²⁺, Fe³⁺, etc. In some embodiments, the heterologous epitope is not a polypeptide selected from the group consisting of Streptavidin, Strep II, HA, L14, 4C-RGD, LH, and Protein A.

In some embodiments, the affinity tag is selected from the group consisting of FLAG (SEQ ID NO:7), HA (SEQ ID NO:8) and c-myc (EQKLISEEDL; SEQ ID NO:6). In some embodiments, the heterologous epitope is c-myc.

In some embodiments, a recombinant AAV capsid as described herein comprises the amino acid sequence EQKLISEEDL (set forth as SEQ ID NO: 6) flanked by and/or operably linked to at least 5 contiguous amino acids of an AAV VP1 capsid protein. In some embodiments, a recombinant AAV capsid as described herein comprises the amino acid sequence EQKLISEEDL (set forth as SEQ ID NO: 6) flanked by and/or operably linked to at least 5 contiguous amino acids of an AAV2 VP1 capsid protein. In some embodiments, a recombinant AAV capsid as described herein comprises EQKLISEEDL (set forth as SEQ ID NO:6) inserted between N587 and R588 of an AAV2 VP1 capsid protein. In some embodiments, a recombinant AAV capsid protein as described herein comprises an amino acid sequence set forth as SEQ ID NO:2. In some embodiments, a recombinant AAV capsid protein as described herein comprises an amino acid sequence set forth as SEQ ID NO:4. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:25. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:26. In some embodiments, a recombinant AAV capsid, AAV vector comprising a recombinant AAV capsid, and/or compositions comprising a recombinant AAV capsid comprises an amino acid sequence encoded by the nucleic acid sequence set forth as SEQ ID NO:27.

In some embodiments, the heterologous epitope comprises an affinity tag and one or more linkers. In some embodiments, the heterologous epitope comprises an affinity tag flanked by a linker, e.g., the heterologous epitope comprises from N-terminus to C-terminus a first linker, an affinity tag, and a second linker. In some embodiments, the first and second linkers are each independently at least one amino acid in length. In some embodiments, the first and second linkers are identical.

Generally, a heterologous epitope as described herein, e.g., an affinity tag by itself or in combination with one or more linkers, is between about 5 amino acids to about 35 amino acids in length. In some embodiments, the heterologous epitope (by itself or in combination with one or more linkers) is at least 5 amino acids in length. In some embodiments, the heterologous epitope (by itself or in combination with one or more linkers) is 6 amino acids in length. In some embodiments, the heterologous epitope (by itself or in combination with one or more linkers) is 7 amino acids in length. In some embodiments, the heterologous epitope (by itself or in combination with one or more linkers) is 8 amino acids in length. In some embodiments, the heterologous epitope (by itself or in combination with one or more linkers) is 9 amino acids in length. In some embodiments, the heterologous epitope (by itself or in combination with one or more linkers) is 10 amino acids in length. In some embodiments, the heterologous epitope (by itself or in combination with one or more linkers) is 11 amino acids in length. In some embodiments, the heterologous epitope (by itself or in combination with one or more linkers) is 12 amino acids in length. In some embodiments, the heterologous epitope (by itself or in combination with one or more linkers) is 13 amino acids in length. In some embodiments, the heterologous epitope (by itself or in combination with one or more linkers) is 14 amino acids in length. In some embodiments, the heterologous epitope (by itself or in combination with one or more linkers) is 15 amino acids in length. In some embodiments, the heterologous epitope (by itself or in combination with one or more linkers) is 16 amino acids in length. In some embodiments, the heterologous epitope (by itself or in combination with one or more linkers) is 17 amino acids in length. In some embodiments, the heterologous epitope (by itself or in combination with one or more linkers) is 18 amino acids in length. In some embodiments, the heterologous epitope (by itself or in combination with one or more linkers) is 19 amino acids in length. In some embodiments, the heterologous epitope (by itself or in combination with one or more linkers) is 20 amino acids in length. In some embodiments, the heterologous epitope (by itself or in combination with one or more linkers) is 21 amino acids in length. In some embodiments, the heterologous epitope (by itself or in combination with one or more linkers) is 22 amino acids in length. In some embodiments, the heterologous epitope (by itself or in combination with one or more linkers) is 23 amino acids in length. In some embodiments, the heterologous epitope (by itself or in combination with one or more linkers) is 24 amino acids in length. In some embodiments, the heterologous epitope (by itself or in combination with one or more linkers) is 25 amino acids in length. In some embodiments, the heterologous epitope (by itself or in combination with one or more linkers) is 26 amino acids in length. In some embodiments, the heterologous epitope (by itself or in combination with one or more linkers) is 27 amino acids in length. In some embodiments, the heterologous epitope (by itself or in combination with one or more linkers) is 28 amino acids in length. In some embodiments, the heterologous epitope (by itself or in combination with one or more linkers) is 29 amino acids in length. In some embodiments, the heterologous epitope (by itself or in combination with one or more linkers) is 30 amino acids in length. In some embodiments, the heterologous epitope (by itself or in combination with one or more linkers) is 31 amino acids in length. In some embodiments, the heterologous epitope (by itself or in combination with one or more linkers) is 32 amino acids in length. In some embodiments, the heterologous epitope (by itself or in combination with one or more linkers) is 33 amino acids in length. In some embodiments, the heterologous epitope (by itself or in combination with one or more linkers) is 34 amino acids in length. In some embodiments, the heterologous epitope (by itself or in combination with one or more linkers) is 35 amino acids in length.

### Retargeting Moieties

An AAV vector as described herein has reduced to abolished transduction capabilities in the absence of a multispecific binding molecule, specifically a multispecific binding molecule comprising (i) an antibody paratope that specifically binds the epitope and (ii) a retargeting ligand that specifically binds a receptor, which may be conjugated to the surface of a bead (e.g., for purification) or expressed by a target cell. Accordingly, a multispecific binding molecule comprising (i) an antibody paratope that specifically binds the epitope and (ii) a retargeting ligand that specifically binds a receptor retargets the AAV vector. Such "retargeting" or "redirecting" may include a scenario in which the wildtype AAV vector targets several cells within a tissue and/or several organs within an organism, which broad targeting of the tissue or organs is reduced to abolished by insertion of the heterologous epitope, and which retargeting to more specific cells in the tissue or more specific organ in the organism is achieved with the multispecific binding molecule. Such retargeting or redirecting may also include a scenario in which the wildtype AAV vector targets a tissue, which targeting of the tissue is reduced to abolished by insertion of the heterologous epitope, and which retargeting to a completely different tissue is achieved with the multispecific binding molecule. An antibody paratope as described herein generally comprises at a minimum a complementarity determining region (CDR) that specifically recognizes the heterologous epitope, e.g., a CDR3 region of a heavy and/or light chain variable domain. In some embodiments, a multispecific binding molecule comprises an antibody (or portion thereof) that comprises the antibody paratope that specifically binds the heterologous epitope. For example, a multispecific binding molecule may comprise a single domain heavy chain variable region or a single domain light chain variable region, wherein the single domain heavy chain variable region or single domain light chain variable region comprises an antibody paratope that specifically binds the heterologous epitope. In some embodiments, a multispecific binding molecule may comprise an Fv region, e.g., a multispecific binding molecule may comprise an scFv, that comprises an antibody paratope that specifically binds the heterologous epitope. In some embodiments, a multispecific binding molecule as described herein comprises an antibody paratope that specifically binds c-myc.

In some embodiments, a multispecific binding molecule as described herein comprises an antibody paratope that specifically binds c-myc, which paratope comprises the scFv, the heavy and light chain variable domains of the scFv, and/or the set of HCDR1-HCDR2-HCDR3-LCDR1-LCDR2-LCDR3 amino acid sequence, encoded by the nucleic acid sequence set forth as SEQ ID NO:28, e.g., the scFv comprising the amino acid sequence set forth as SEQ ID NO:37. In some embodiments, a multispecific binding molecule as described herein comprises an Fv or scFv encoded by the nucleic acid sequence set forth as SEQ ID NO:28.

Accordingly, the present invention includes as antibodies, antigen-binding fragments of antibodies, and multispecific binding proteins that specifically bind c-myc, wherein the antibodies, fragments of antibodies, and multispecific binding proteins comprise a paratope that comprises a heavy chain variable region (HCVR) comprising SEQ ID NO:29 and a light chain variable region (LCVR) comprising SEQ ID NO:30. The present invention also includes antibodies, antigen-binding fragments of antibodies, and/or multispecific binding proteins that comprise a paratope that specifically bind c-Myc, wherein the paratope comprises a heavy chain complementarity determining region 1 (HCDR1) comprising SEQ ID NO:31, a HCDR2 comprising SEQ ID NO:32, a HCDR3 comprising SEQ ID NO:33, a light chain complementarity determining region 1 (LCDR1) comprising SEQ ID NO:34, a LCDR2 comprising SEQ ID NO:35, and a LCDR3 comprising SEQ ID NO:36.

The present invention may employ antibodies, antigen-binding fragments thereof, and/or multispecific binding proteins comprising a paratope that comprises an HCVR comprising an amino acid sequence set forth as SEQ ID NO:29, or a substantially similar sequence thereof having at least 90%, at least 95%, at least 98% or at least 99% sequence identity thereto.

The present invention may employ antibodies, antigen-binding fragments thereof, and/or multispecific binding proteins comprising a paratope comprising an LCVR comprising an amino acid sequence set forth as SEQ ID NO:30, or a substantially similar sequence thereof having at least 90%, at least 95%, at least 98% or at least 99% sequence identity thereto.

The present invention may employ antibodies, antigen-binding fragments thereof, and/or multispecific binding proteins comprising a paratope that comprises an HCVR and an LCVR amino acid sequence pair (HCVR/LCVR) comprising the HCVR amino acid sequence set forth as SEQ ID NO:29 paired with the LCVR amino acid sequence set forth as SEQ ID NO:30. In some embodiments, the HCVR/LCVR amino acid sequence pair is selected from the group consisting of SEQ ID NOs: 29/30.

The present invention may employ antibodies, antigen-binding fragments thereof, and/or multispecific binding proteins comprising a paratope that comprises a heavy chain CDR1 (HCDR1) comprising an amino acid sequence set forth as SEQ ID NO:31 or a substantially similar sequence thereof having at least 90%, at least 95%, at least 98% or at least 99% sequence identity.

The present invention may employ antibodies, antigen-binding fragments thereof, and/or multispecific binding proteins comprising a paratope that comprises a heavy chain CDR2 (HCDR2) comprising an amino acid sequence set forth as SEQ ID NO:32, or a substantially similar sequence thereof having at least 90%, at least 95%, at least 98% or at least 99% sequence identity.

The present invention may employ antibodies, antigen-binding fragments thereof, and/or multispecific binding proteins comprising a paratope that comprises a heavy chain CDR3 (HCDR3) comprising an amino acid sequence set forth as SEQ ID NO:33, or a substantially similar sequence thereof having at least 90%, at least 95%, at least 98% or at least 99% sequence identity.

The present invention may employ antibodies, antigen-binding fragments thereof, and/or multispecific binding proteins comprising a paratope that comprises a light chain CDR1 (LCDR1) comprising an amino acid sequence set forth as SEQ ID NO:34, or a substantially similar sequence thereof having at least 90%, at least 95%, at least 98% or at least 99% sequence identity.

The present invention may employ antibodies, antigen-binding fragments thereof, and/or multispecific binding proteins comprising a paratope that comprises a light chain CDR2 (LCDR2) comprising an amino acid sequence set forth as SEQ ID NO:35, or a substantially similar sequence thereof having at least 90%, at least 95%, at least 98% or at least 99% sequence identity.

The present invention may employ antibodies, antigen-binding fragments thereof, and/or multispecific binding proteins comprising a paratope that comprises a light chain CDR3 (LCDR3) comprising an amino acid sequence set forth as SEQ ID NO:36, or a substantially similar sequence thereof having at least 90%, at least 95%, at least 98% or at least 99% sequence identity.

The present invention may employ antibodies, antigen-binding fragments thereof, and/or multispecific binding proteins comprising a paratope that comprises an HCDR3 and an LCDR3 amino acid sequence pair (HCDR3/LCDR3) comprising the HCDR3 amino acid set forth as SEQ ID NO:33 paired with the LCDR3 amino acid sequence set forth as SEQ ID NO:36. In some embodiments, the HCDR3/LCDR3 amino acid sequence pair is set forth as SEQ ID NOs: 33/36.

The present invention may employ antibodies, antigen-binding fragments thereof, and/or multispecific binding proteins comprising a paratope that comprises a set of six CDRs (*i.e.*, HCDR1-HCDR2-HCDR3-LCDR1-LCDR2-LCDR3) encoded by the nucleotide sequence set forth as SEQ ID NO:28. In certain embodiments, the HCDR1-HCDR2-HCDR3-LCDR1-LCDR2-LCDR3 amino acid sequences set is set forth as SEQ ID NOs: 31-32-33-34-35-36.

In a related embodiment, the present invention may employ antibodies, antigen-binding fragments thereof, and/or multispecific binding proteins comprising a paratope that comprises a set of six CDRs (*i.e.*, HCDR1-HCDR2-HCDR3-LCDR1-LCDR2-LCDR3) contained within an HCVR/LCVR amino acid sequence pair set forth as SEQ ID NOs:29/30. Methods and techniques for identifying CDRs within HCVR and LCVR amino acid sequences are well known in the art and can be used to identify CDRs within the specified HCVR and/or LCVR amino acid sequences disclosed herein. Exemplary conventions that can be used to identify the boundaries of CDRs include, *e.g.*, the Kabat definition, the Chothia definition, and the AbM definition. In general terms, the Kabat definition is based on sequence variability, the Chothia definition is based on the location of the structural loop regions, and the AbM definition is a compromise between the Kabat and Chothia approaches. *See*, *e.g.*, Kabat, "Sequences of Proteins of Immunological Interest," National Institutes of Health, Bethesda, Md. (1991); Al-Lazikani et al., J. Mol. Biol. 273:927-948 (1997); and Martin et al., Proc. Natl. Acad. Sci. USA 86:9268-9272 (1989). Public databases are also available for identifying CDR sequences within an antibody.

The present invention may employ nucleic acid molecules encoding the anti-myc antibodies or portions thereof.

A multispecific binding molecule as described herein further comprises a retargeting ligand, in addition to a paratope (e.g., an antibody or portion thereof) that specifically binds the heterologous epitope inserted into/displayed by a recombinant AAV capsid protein. In some embodiments, the retargeting ligand binds a protein expressed on the surface of a cell, e.g., a cell surface protein on a (human) eurkaryotic cell, e.g., a target cell. There are a large number of cell surface proteins, e.g., cell surface receptors, suitable which may be targeted by a retargeting ligand, and for which a retargeting ligand, e.g., antibodies or portions thereof, are already available. Such structures include, but are not limited to: the class I and class II Major Histocompatibility Antigens; receptors for a variety of cytokines (e.g., receptors for IL-1, IL-4, IL-6, IL-13, IL-22, IL-25, IL-33, etc.), cell-type specific growth hormones, brain derived neurotrophic factor (BDNF), ciliary neurotrophic factor (CTNF), colony stimulating growth factors, endothelial growth factors, epidermal growth factors, fibroblast growth factors, glially derived neurotrophic factor, glial growth factors, gro-beta/mip 2, hepatocyte growth factors, insulin-like growth factor, interferons (α-IFN, β-IFN, γIFN, consensus IFN), interleukins (IL-1, IL-2, IL-3, IL-4, IL-5, IL-6, IL-7, IL-8, IL-9, IL-10, IL-11, IL-12, IL-13, IL-14), keratinocyte growth factor, leukemia inhibitory factors, macrophage/monocyte chemotactic activating factor, nerve growth factor, neutrophil activating protein 2, platelet derived growth factor, stem cell factor, transforming growth factor, tumor necrosis factors. vascular endothelial growth factor, lipoproteins, including additional or other type 1 transmembrane receptors such as PRLR, G-protein coupled receptors such as GCGR, ion channels such as Nav1.7, ASIC1 or ASIC2; cell adhesion molecules; transport molecules for metabolites such as amino acids; the antigen receptors of B- and T-lymphocytes (e.g., B cell receptors and associated proteins (e.g., CD19, CD20, etc.) and T cell receptors and associated proteins (e.g., CD3, CD4, CD8, etc.); a tetraspanin protein (e.g., CD63). A recombinant AAV capsid described herein allows for the specific infection of a cell type by employing a multispecific binding molecule comprising a retargeting ligand that binds differentiation cell surface antigens as targets for the AAV vector complex.

In some embodiments the retargeting ligand binds a protein expressed primarily (e.g., solely) by (human) liver cells, i.e., a liver specific marker. In some embodiments the retargeting ligand binds a protein expressed primarily (e.g., solely) by (human) brain cells, a brain cell specific marker. In some embodiments the retargeting ligand binds a protein expressed primarily (e.g., solely) by (human) hematopoietic cells, i.e., a hematopoietic cell specific marker. In some embodiments the retargeting ligand binds a protein expressed primarily (e.g., solely) by (human) T cells, i.e., a T-cell specific marker. In some embodiments the retargeting ligand binds a protein expressed primarily (e.g., solely) by (human) B cells, i.e., a B-cell specific marker. In some embodiments the retargeting ligand binds a protein expressed primarily (e.g., solely) by (human) dendritic cells, i.e., a dendritic cell specific marker. In some embodiments the retargeting ligand binds a protein expressed primarily (e.g., solely) by (human) macrophages, i.e., a macrophage specific marker. In some embodiments the retargeting ligand binds a protein expressed primarily (e.g., solely) by (human) NK cells, i.e., an NK cell specific marker. In some embodiments the retargeting ligand binds a protein expressed primarily (e.g., solely) by (human) kidney cells, i.e., a kidney specific marker. In some embodiments, the retargeting ligand binds a receptor expressed primarily (e.g., solely) by (human) pancreas cells, i.e., a pancreas specific marker. In some embodiments, the retargeting ligand binds a receptor expressed primarily (e.g., solely) by (human) intestinal cells, i.e., an intestine specific marker. In some embodiments the retargeting ligand binds a protein expressed primarily (e.g., solely) by a (human) cancerous cell, i.e., a tumor associated antigen. In some embodiments the retargeting ligand binds a protein expressed primarily (e.g., solely) by (human) cell infected with heterologous pathogen. Proteins that (1) are specifically expressed by, or for which expression is enriched in, a cell/tissue/organ, and (2) recognized by an antigen-binding protein useful as a retargeting ligand as described herein are well-known and may also be found at www.proteinatlas.org; *see also* Uhlen et al. (2010) Nat. Biotech. 28:1248-50. Table 1 below provides exemplary and non-limiting organ specific markers for which antigen-binding proteins, which may be useful as retargeting ligands, are available and the cells/tissue/organ expressing such markers.

**Table 1: Exemplary tissue specific markers**

| Tissue | Tissue Specific Markers |
|---|---|
| Liver | ATP binding cassette subfamily B; members 11 (ABCB11) |
| | Alanine-glyoxylate aminotransferase (AGXT) |
| | Alcohol dehydrogenase 1A, class I (ADH1A) |
| | Alchohol dehydrogenase 4 (class II) pi polypeptide (ADH4) |
| | Amyloid P component, serum (APCS) |
| | Angiopoietin like 3 (ANGPTL3) |
| | Apolipoprotein; C1, C2 (APOC1, APOC2) |
| | APOC4-APOC2 |
| | Asialoglycoprotein receptor 1 (ASGR1) |
| | Asialoglycoprotein receptor 2 (ASGR2) |
| | Bile acid-CoA: amino acid N-aceyltransferase (BAAT) |
| | Complement C8 beta chain (C8B) |
| | Coagulation factor II, thrombin (F2) |
| | Cytochrome P450 family 1 subfamily A member 2 CYP1A2 |
| | Mannose binding lectin 2 (MBL2) |
| | Soluble carrier organic anion transporter family member 1B3 (SLCO1B3) |
| | Paraoxonase 3 (PON3) |
| | Transferrin receptor 2 (TFR2) |
| | Urocanate hydratase 1 (UROC 1) |

| | |
|---|---|
| Intestine | Fatty acid binding protein 6 (FABP6) |
| Pancreas | CUB and zona pellucida like domains 1 (CUZD1) |
| | Protease, serine 2 (PRSS2) |
| | Protease, serine 3 (PRSS3) |

In some embodiments, the retargeting ligand binds a receptor expressed by a (human) liver cell, e.g., an asialoglycoprotein receptor, e.g., hASGR1. In some embodiments, the retargeting ligand binds a receptor expressed by a (human) brain cell. In some embodiments, the retargeting ligand binds a receptor expressed by a (human) T cell, e.g., CD3, e.g., CD3ε. In some embodiments, the retargeting ligand binds a receptor expressed by a (human) kidney cell. In some embodiments, the retargeting ligand binds a receptor expressed by a (human) muscle cell, e.g., an integrin. In some embodiments, the retargeting ligand binds a receptor expressed by a (human) cancerous cell, e.g., a tumor associated antigen, e.g., E6 and E7. In some embodiments, the retargeting ligand binds human glucagon receptor (hGCGR). In some embodiments, the retargeting ligand binds human ENTPD3.

In some embodiments, the retargeting ligand binds a tumor-associated antigen expressed by a tumor cell. Non-limiting examples of specific tumor-associated antigens include, e.g., adipophilin, AIM-2, ALDH1A1, alpha-actinin-4, alpha-fetoprotein ("AFP"), ARTC1, B-RAF, BAGE-1, BCLX (L), BCR-ABL fusion protein b3a2, beta-catenin, BING-4, CA-125, CALCA, carcinoembryonic antigen ("CEA"), CASP-5, CASP-8, CD274, CD45, Cdc27, CDK12, CDK4, CDKN2A, CEA, CLPP, COA-1, CPSF, CSNK1A1, CTAG1, CTAG2, cyclin D1, Cyclin-A1, dek-can fusion protein, DKK1, EFTUD2, Elongation factor 2, ENAH (hMena), Ep-CAM, EpCAM, EphA3, epithelial tumor antigen ("ETA"), ETV6-AML1 fusion protein, EZH2, E6, E7, FGF5, FLT3-ITD, FN1, G250/MN/CAIX, GAGE-1,2,8, GAGE-3,4,5,6,7, GAS7, glypican-3, GnTV, gp100/Pme117, GPNMB, HAUS3, Hepsin, HER-2/neu, HERV-K-MEL, HLA-A11, HLA-A2, HLA-DOB, hsp70-2, IDO1, IGF2B3, IL13Ralpha2, Intestinal carboxyl esterase, K-ras, Kallikrein 4, KIF20A, KK-LC-1, KKLC1, KM-HN-1, KMHN1 also known as CCDC110, LAGE-1, LDLR-fucosyltransferaseAS fusion protein, Lengsin, M-CSF, MAGE-A1, MAGE-A 10, MAGE-A12, MAGE-A2, MAGE-A3, MAGE-A4, MAGE-A6, MAGE-A9, MAGE-C1, MAGE-C2, malic enzyme, mammaglobin-A, MART2, MATN, MC1R, MCSP, mdm-2, ME1, Melan-A/MART-1, Meloe, Midkine, MMP-2, MMP-7, MUC1, MUC5AC, mucin, MUM-1, MUM-2, MUM-3, Myosin, Myosin class I, N-raw, NA88-A, neo-PAP, NFYC, NY-BR-1, NY-ESO-1/LAGE-2, OA1, OGT, OS-9, P polypeptide, p53, PAP, PAX5, PBF, pml-RARalpha fusion protein, polymorphic epithelial mucin ("PEM"), PPP1R3B, PRAME, PRDX5, PSA, PSMA, PTPRK, RAB38/NY-MEL-1, RAGE-1, RBAF600, RGS5, RhoC, RNF43, RU2AS, SAGE, secernin 1, SIRT2, SNRPD1, SOX10, Sp17, SPA17, SSX-2, SSX-4, STEAP1, survivin, SYT-SSX1 or -SSX2 fusion protein, TAG-1, TAG-2, Telomerase, TGF-betaRII, TPBG, TRAG-3, Triosephosphate isomerase, TRP-1/gp75, TRP-2, TRP2-INT2, tyrosinase, tyrosinase ("TYR"), VEGF, WT1, XAGE-lb/GAGED2a, Kras, NY-ESO1, MAGE-A3, HPV E2, HPV E6, HPV E7, WT-1 antigen (in lymphoma and other solid tumors), ErbB receptors, Melan A [MART1], gp 100, tyrosinase, TRP-1/gp 75, and TRP-2 (in melanoma); MAGE-1 and MAGE-3 (in bladder, head and neck, and non-small cell carcinoma); HPV EG and E7 proteins (in cervical cancer); Mucin [MUC-1] (in breast, pancreas, colon, and prostate cancers); prostate-specific antigen [PSA] (in prostate cancer); carcinoembryonic antigen [CEA] (in colon, breast, and gastrointestinal cancers), and such shared tumor-specific antigens as MAGE-2, MAGE-4, MAGE-6, MAGE-10, MAGE-12, BAGE-1, CAGE-1,2,8, CAGE-3 TO 7, LAGE-1, NY-ESO-1/LAGE-2, NA-88, GnTV, TRP2-INT2. In some embodiments, the tumor-associated antigen is ErbB2/Her2. In some embodiments the, the tumor-associated antigen is E6 and/or E7.

In some embodiments, the retargeting ligand binds to CD markers associated with the immune response, e.g., CD3, CD4, CD8, CD19, CD20, etc. In some embodiments, the CD marker is CD3.

In certain exemplary embodiments, the multispecific binding molecule is a bispecific antibody. Each antigen-binding domain of a bispecific antibody comprises a heavy chain variable domain (HCVR) and a light chain variable domain (LCVR). In the context of a bispecific antigen-binding molecule comprising a first and a second antigen-binding domain (e.g., a bispecific antibody), the CDRs of the first antigen-binding domain may be designated with the prefix "A1" and the CDRs of the second antigen-binding domain may be designated with the prefix "A2". Thus, the CDRs of the first antigen-binding domain may be referred to herein as A1-HCDR1, A1-HCDR2, and A1-HCDR3; and the CDRs of the second antigen-binding domain may be referred to herein as A2-HCDR1, A2-HCDR2, and A2-HCDR3.

The first antigen-binding domain and the second antigen-binding domain may be directly or indirectly connected to one another to form a bispecific antigen-binding molecule of the present invention. Alternatively, the first antigen-binding domain and the second antigen-binding domain may each be connected to a separate multimerizing domain. The association of one multimerizing domain with another multimerizing domain facilitates the association between the two antigen-binding domains, thereby forming a bispecific antigen-binding molecule. As used herein, a "multimerizing domain" is any macromolecule, protein, polypeptide, peptide, or amino acid that has the ability to associate with a second multimerizing domain of the same or similar structure or constitution. For example, a multimerizing domain may be a polypeptide comprising an immunoglobulin CH3 domain. A non-limiting example of a multimerizing component is an Fc portion of an immunoglobulin (comprising a CH2-CH3 domain), e.g., an Fc domain of an IgG selected from the isotypes IgG1, IgG2, IgG3, and IgG4, as well as any allotype within each isotype group.

Bispecific antigen-binding molecules employed in the present invention will typically comprise two multimerizing domains, e.g., two Fc domains that are each individually part of a separate antibody heavy chain. The first and second multimerizing domains may be of the same IgG isotype such as, e.g., IgG1/IgG1, IgG2/IgG2, IgG4/IgG4. Alternatively, the first and second multimerizing domains may be of different IgG isotypes such as, e.g., IgG1/IgG2, IgG1/IgG4, IgG2/IgG4, etc.

In certain embodiments, the multimerizing domain is an Fc fragment or an amino acid sequence of 1 to about 200 amino acids in length containing at least one cysteine residues. In other embodiments, the multimerizing domain is a cysteine residue, or a short cysteine-containing peptide. Other multimerizing domains include peptides or polypeptides comprising or consisting of a leucine zipper, a helix-loop motif, or a coiled-coil motif.

Any bispecific antibody format or technology may be used to make the bispecific antigen-binding molecules employed in the present invention. For example, an antibody or fragment thereof having a first antigen binding specificity can be functionally linked (e.g., by chemical coupling, genetic fusion, noncovalent association or otherwise) to one or more other molecular entities, such as another antibody or antibody fragment having a second antigen-binding specificity to produce a bispecific antigen-binding molecule. Specific exemplary bispecific formats that can be used in the context of the present invention include, without limitation, e.g., scFv-based or diabody bispecific formats, IgG-scFv fusions, dual variable domain (DVD)-Ig, Quadroma, knobs-into-holes, common light chain (e.g., common light chain with knobs-into-holes, etc.), CrossMab, CrossFab, (SEED)body, leucine zipper, Duobody, IgG1/IgG2, dual acting Fab (DAF)-IgG, and Mab2 bispecific formats (see, e.g., Klein et al. 2012, mAbs 4:6, 1-11, and references cited therein, for a review of the foregoing formats; see also Brinkmann and Konterman (2017) mAbs 9:182-212).

The present invention may also employ bispecific antigen-binding molecules comprising a first CH3 domain and a second Ig CH3 domain, wherein the first and second Ig CH3 domains differ from one another by at least one amino acid, and wherein at least one amino acid difference reduces binding of the bispecific antibody to Protein A as compared to a bispecific antibody lacking the amino acid difference. In one embodiment, the first Ig CH3 domain binds Protein A and the second Ig CH3 domain contains a mutation that reduces or abolishes Protein A binding such as an H95R modification (by IMGT exon numbering; H-435-R by EU numbering). The second CH3 may further comprise a Y96F modification (by IMGT; Y436F by EU). Further modifications that may be found within the second CH3 include: D-16-E, L-18-M, N-44-S, K-52-N, V-57-M, and V-82-I (by IMGT; D-356-E, L-358-M, N-384-S, K-392-N, V-397-M, and V-422-I by EU) in the case of IgG1 antibodies; N-44-S, K-52-N, and V-82-I (IMGT; N-384-S, K-392-N, and V-422-I by EU) in the case of IgG2 antibodies; and Q-15-R, N-44-S, K-52-N, V-57-M, R-69-K, E-79-Q, and V-82-I (by IMGT; Q-355-R, N-384-S, K-392-N, V-397-M, R-409-K, E-419-Q, and V-422-I by EU) in the case of IgG4 antibodies; see, e.g., WO 2010/151792.

In certain embodiments, the Fc domain may be chimeric, combining Fc sequences derived from more than one immunoglobulin isotype. For example, a chimeric Fc domain can comprise part or all of a CH2 sequence derived from a human IgG1, human IgG2 or human IgG4 CH2 region, and part or all of a CH3 sequence derived from a human IgG1, human IgG2 or human IgG4. A chimeric Fc domain can also contain a chimeric hinge region. For example, a chimeric hinge may comprise an "upper hinge" sequence, derived from a human IgG1, a human IgG2 or a human IgG4 hinge region, combined with a "lower hinge" sequence, derived from a human IgG1, a human IgG2 or a human IgG4 hinge region. A particular example of a chimeric Fc domain that can be included in any of the antigen-binding molecules set forth herein comprises, from N- to C-terminus: [IgG4 CH1] - [IgG4 upper hinge] - [IgG2 lower hinge] - [IgG4 CH2] - [IgG4 CH3]. Another example of a chimeric Fc domain that can be included in any of the antigen-binding molecules set forth herein comprises, from N- to C-terminus: [IgG1 CH1] - [IgG1 upper hinge] - [IgG2 lower hinge] - [IgG4 CH2] - [IgG1 CH3]. These and other examples of chimeric Fc domains that can be included in any of the antigen-binding molecules of the present invention are described in PCT Application No. WO2014/022540. Chimeric Fc domains having these general structural arrangements, and variants thereof, can have altered Fc receptor binding, which in turn affects Fc effector function.

### Methods of Use and Making

A further embodiment of the recombinant AAV capsid proteins described herein is their use for delivering a nucleotide of interest, e.g., a reporter gene or a therapeutic gene, to a target cell. Hence, the present invention provides a composition of the present invention for use in an *in vivo* method of treatment or diagnosis, the method comprising delivering the nucleotide of interest to a target cell expressing a cell surface protein comprising contacting the target cell with the composition, wherein the multispecific binding molecule comprises a retargeting ligand that binds the cell surface protein. The present invention further provides an *in vitro* or *ex vivo* method of delivering a nucleotide of interest to a target cell expressing a cell surface protein comprising contacting the target cell with the composition of the present invention, wherein the multispecific binding molecule comprises a retargeting ligand that binds the cell surface protein.

Generally, a nucleotide of interest may be a transfer plasmid, which may generally comprise 5' and 3' inverted terminal repeat (ITR) sequences flanking the reporter gene(s) or therapeutic gene(s) (which may be under the control of a viral or non-viral promoter, when encompassed within an AAV vector). In one embodiment, a nucleotide of interest is a transfer plasmid comprising from 5' to 3': a 5' ITR, a promoter, a gene (e.g., a reporter and/or therapeutic gene) and a 3' ITR.

Non-limiting examples of useful promoters include, e.g., cytomegalovirus (CMV)-promoter, the spleen focus forming virus (SFFV)-promoter, the elongation factor 1 alpha (EF1a)-promoter (the 1.2 kb EFla-promoter or the 0.2 kb EFla-promoter), the chimeric EF 1 a/IF4-promoter, and the phospho-glycerate kinase (PGK)-promoter. An internal enhancer may also be present in the viral construct to increase expression of the gene of interest. For example, the CMV enhancer (Karasuyama et al. 1989. J. Exp. Med. 169:13) may be used. In some embodiments, the CMV enhancer can be used in combination with the chicken 13-actin promoter.

A variety of reporter genes (or detectable moieties) can be encapsulated in a multimeric structure comprising the recombinant AAV capsid proteins described herein. Exemplary reporter genes include, for example, β-galactosidase (encoded *lacZ* gene), Green Fluorescent Protein (GFP), enhanced Green Fluorescent Protein (eGFP), MmGFP, blue fluorescent protein (BFP), enhanced blue fluorescent protein (eBFP), mPlum, mCherry, tdTomato, mStrawberry, J-Red, DsRed, mOrange, mKO, mCitrine, Venus, YPet, yellow fluorescent protein (YFP), enhanced yellow fluorescent protein (eYFP), Emerald, CyPet, cyan fluorescent protein (CFP), Cerulean, T-Sapphire, luciferase, alkaline phosphatase, or a combination thereof. The methods described herein demonstrate the construction of targeting vectors that employ the use of a reporter gene that encodes green fluorescent protein, however, persons of skill upon reading this disclosure will understand that non-human animals described herein can be generated in the absence of a reporter gene or with any reporter gene known in the art.

A variety of therapeutic genes can also be encapsulated in the can be encapsulated in a multimeric structure comprising the recombinant AAV capsid proteins described herein, e.g., as part of a transfer vector. Non-limiting examples of a therapeutic gene include those that encode a toxin (e.g., a suicide gene), a therapeutic antibody or fragment thereof, a CRISPR/Cas system or portion(s) thereof, antisense RNA, siRNA, shRNA, etc.

A further embodiment of the present invention is a process for the preparation of a recombinant AAV capsid protein, the method comprising the steps of:
a) expressing a nucleic acid encoding the recombinant AAV capsid protein under suitable conditions, and
b) isolating the expressed capsid protein of step a).

Further embodiments of the present invention is a method for altering the tropism of a virus, the method comprising the steps of: (a) inserting a nucleic acid encoding a heterologous epitope into a nucleic acid sequence encoding an AAV capsid protein to form a nucleotide sequence encoding a genetically modified AAV capsid protein comprising the heterologous epitope and/or (b) culturing a packaging cell in conditions sufficient for the production of AAV vectors, wherein the packaging cell comprises the nucleotide sequence. A further embodiment of the present invention is a method for displaying a heterologous epitope on the surface of an AAV capsid protein, the method comprising the steps of: a) expressing the nucleic acid according to this invention under suitable conditions, and b) isolating the expressed AAV capsid protein of step a).

In some embodiments, the packaging cell further comprises a helper plasmid and/or a transfer plasmid comprising a nucleotide of interest. In some embodiments, the methods further comprise isolating self-complementary adeno-associated viral vectors from culture supernatant. In some embodiments, the methods further comprise lysing the packaging cell and isolating single-stranded adeno-associated viral vectors from the cell lysate. In some embodiments, the methods further comprise (a) clearing cell debris, (b) treating the supernatant containing AAV vectors with DNase I and MgCl2, (c) concentrating viral vectors, (d) purifying the viral vectors, and (e) any combination of (a)-(d).

Packaging cells useful for production of the AAV vectors described herein include, e.g., animal cells permissive for the virus, or cells modified so as to be permissive for the virus; or the packaging cell construct, for example, with the use of a transformation agent such as calcium phosphate. Non-limiting examples of packaging cell lines useful for producing AAV vectors described herein include, e.g., human embryonic kidney 293 (HEK-293) cells (e.g., American Type Culture Collection [ATCC] No. CRL-1573), HEK-293 cells that contain the SV40 Large T-antigen (HEK-293T or 293T), HEK293T/17 cells, human sarcoma cell line HT-1080 (CCL-121), lymphoblast-like cell line Raj i (CCL-86), glioblastoma-astrocytoma epithelial-like cell line U87-MG (HTB-14), T-lymphoma cell line HuT78 (TIB-161), NIH/3T3 cells, Chinese Hamster Ovary cells (CHO) (e.g., ATCC Nos. CRL9618, CCL61, CRL9096), HeLa cells (e.g., ATCC No. CCL-2), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RATI cells, mouse L cells (ATCC No. CCLI.3), HLHepG2 cells, CAP cells, CAP-T cells, and the like.

L929 cells, the FLY viral packaging cell system outlined in Cosset et al (1995) J Virol 69,7430-7436, NS0 (murine myeloma) cells, human amniocytic cells (e.g., CAP, CAP-T), yeast cells (including, but not limited to, S. cerevisiae, Pichia pastoris), plant cells (including, but not limited to, Tobacco NT1, BY-2), insect cells (including but not limited to SF9, S2, SF21, Tni (e.g. High 5)) or bacterial cells (including, but not limited to, E. coli).

For additional packaging cells and systems, packaging techniques and vectors for packaging the nucleic acid genome into the pseudotyped viral vector see, for example, Polo, et al, Proc Natl Acad Sci USA, (1999) 96:4598-4603. Methods of packaging include using packaging cells that permanently express the viral components, or by transiently transfecting cells with plasmids.

Further embodiments include methods of redirecting an AAV virus and/or delivering a reporter or therapeutic gene to a target cell, the method comprising a method for transducing cells in vitro, the method comprising the steps of: contacting the target cell with the combination of an AAV vector comprising a capsid comprising a recombinant AAV capsid displaying a heterologous epitope and a multispecific binding molecule, wherein the multispecific binding molecule comprises (i) an antibody paratope that specifically binds the epitope and (ii) a retargeting ligand that specifically binds a receptor expressed by the target cell. In some embodiments, a composition described herein comprises, or a method described herein combines, a recombinant AAV vector and multispecific binding molecule at a molecule molecule ratio that restores the transduction efficiency of the AAV vector similar to that of a wildtype control AAV vector. In some embodiments, the recombinant AAV vector to multispecific binding molecule (molecule:molecule) ratio ranges from 1:0.5 to 1:100. In some embodiments, the recombinant AAV vector to multispecific binding molecule (molecule:molecule) ratio ranges from 1:4 to 1:20. In some embodiments, the recombinant AAV vector to multispecific binding molecule (molecule:molecule) ratio ranges from 1:8 to 1:15. In some embodiments, the recombinant AAV vector to multispecific binding molecule (molecule:molecule) ratio is 1:4. In some embodiments, the recombinant AAV vector to multispecific binding molecule (molecule:molecule) ratio is 1:8. In some embodiments, the recombinant AAV vector to multispecific binding molecule (molecule:molecule) ratio is 1:15. In some embodiments, the recombinant AAV vector to multispecific binding molecule (molecule:molecule) ratio is 1:20. In some embodiments, the recombinant AAV vector to multispecific binding molecule (molecule:molecule) ratio is less than 1:100. In some embodiments, the recombinant AAV vector to multispecific binding molecule (molecule:molecule) ratio is less than 1:50. In some embodiments, the recombinant AAV vector to multispecific binding molecule (molecule:molecule) ratio is less than 1:20. In some embodiments, the recombinant AAV vector to multispecific binding molecule (molecule:molecule) ratio is less than 1:15. In some embodiments, the recombinant AAV vector to multispecific binding molecule (molecule:molecule) ratio is less than 1:10.

In some embodiments, the target cell is *in vitro.* In other embodiments, the invention provides a composition for use in an *in vivo* method of treatment or diagnosis, the method comprising delivering the nucleotide of interest to a target cell expressing a cell surface protein comprising contacting the target cell with the composition. Hence, in such embodiments the target cell is *in vivo* in a subject, e.g., a human.

### Target Cells

A wide variety of cells may be targeted in order to deliver a nucleotide of interest using a recombinant AAV vector as disclosed herein. The target cells will generally be chosen based upon the nucleotide of interest and the desired effect.

In some embodiments, a nucleotide of interest may be delivered to enable a target cell to produce a protein that makes up for a deficiency in an organism, such as an enzymatic deficiency, or immune deficiency, such as X-linked severe combined immunodeficiency. Thus, in some embodiments, cells that would normally produce the protein in the animal are targeted. In other embodiments, cells in the area in which a protein would be most beneficial are targeted.

In other embodiments, a nucleotide of interest, such as a gene encoding an siRNA, may inhibit expression of a particular gene in a target cell. The nucleotide of interest may, for example, inhibit expression of a gene involved in a pathogen life cycle. Thus, cells susceptible to infection from the pathogen or infected with the pathogen may be targeted. In other embodiments, a nucleotide of interest may inhibit expression of a gene that is responsible for production of a toxin in a target cell.

In other embodiments a nucleotide of interest may encode a toxic protein that kills cells in which it is expressed. In this case, tumor cells or other unwanted cells may be targeted.

In still other embodiments a nucleotide of interest that encodes a protein to be collected, such as a therapeutic protein may be used and cells that are able to produce and secrete the protein are targeted.

Once a particular population of target cells is identified in which expression of a nucleotide of interest is desired, a target receptor is selected that is specifically expressed on that population of target cells. The target receptor may be expressed exclusively on that population of cells or to a greater extent on that population of cells than on other populations of cells. The more specific the expression, the more specifically delivery can be directed to the target cells. Depending on the context, the desired amount of specificity of the marker (and thus of the gene delivery) may vary. For example, for introduction of a toxic gene, a high specificity is most preferred to avoid killing non-targeted cells. For expression of a protein for harvest, or expression of a secreted product where a global impact is desired, less marker specificity may be needed.

As discussed above, the target receptor may be any receptor for which a retargeting ligand can be identified or created. Preferably the target receptor is a peptide or polypeptide, such as a receptor. However, in other embodiments the target receptor may be a carbohydrate or other molecule that can be recognized by a binding partner. If a binding partner, e.g., ligand, for the target receptor is already known, it may be used as the affinity molecule. However, if a binding molecule is not known, antibodies to the target receptor may be generated using standard procedures. The antibodies can then be used as a retargeting ligand as part of the multispecific binding molecule.

Thus, target cells may be chosen based on a variety of factors, including, for example, (1) the particular application (e.g., therapy, expression of a protein to be collected, and conferring disease resistance) and (2) expression of a marker with the desired amount of specificity.

Target cells are not limited in any way and include both germline cells and cell lines and somatic cells and cell lines. Target cells can be stem cells derived from either origin. When the target cells are germline cells, the target cells are preferably selected from the group consisting of single-cell embryos and embryonic stem cells (ES).

### Pharmaceutical compositions, dosage forms and administration

A further embodiment provides a medicament comprising at least one recombinant AAV capsid protein and appropriate multispecific binding molecule according to this invention and/or a nucleic acid according to this invention, preferably at least one multimeric structure according to this invention. Preferably such medicament is useful as a gene transfer vector.

Also disclosed herein are pharmaceutical compositions comprising the AAV vectors described herein and a pharmaceutically acceptable carrier and/or excipient. In addition, disclosed herein are pharmaceutical dosage forms comprising the AAV vector described herein.

As discussed herein, the AAV vectors described herein can be used for various therapeutic applications (in vivo and ex vivo) and as research tools.

Pharmaceutical compositions based on the AAV vectors disclosed herein can be formulated in any conventional manner using one or more physiologically acceptable carriers and/or excipients. The AAV vector may be formulated for administration by, for example, injection, inhalation or insulation (either through the mouth or the nose) or by oral, buccal, parenteral or rectal administration, or by administration directly to a tumor.

The pharmaceutical compositions can be formulated for a variety of modes of administration, including systemic, topical or localized administration. Techniques and formulations can be found in, for example, Remington's Pharmaceutical Sciences, Meade Publishing Co., Easton, Pa. For systemic administration, injection is preferred, including intramuscular, intravenous, intraperitoneal, and subcutaneous. For the purposes of injection, the pharmaceutical compositions can be formulated in liquid solutions, preferably in physiologically compatible buffers, such as Hank's solution or Ringer's solution. In addition, the pharmaceutical compositions may be formulated in solid form and redissolved or suspended immediately prior to use. Lyophilized forms of the pharmaceutical composition are also suitable.

For oral administration, the pharmaceutical compositions may take the form of, for example, tablets or capsules prepared by conventional means with pharmaceutically acceptable excipients such as binding agents (e.g. pregelatinized maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulfate). The tablets can also be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. ationd oil, oily esters, ethyl alcohol or fractionated vegetable oils); and preservatives (e.g. methyl or propyl-p-hydroxybenzoates or sorbic acid). The preparations can also contain buffer salts, flavoring, coloring and sweetening agents as appropriate.

The pharmaceutical compositions can be formulated for parenteral administration by injection, e.g. by bolus injection or continuous infusion. Formulations for injection can be presented in a unit dosage form, e.g. in ampoules or in multi-dose containers, with an optionally added preservative. The pharmaceutical compositions can further be formulated as suspensions, solutions or emulsions in oily or aqueous vehicles, and may contain other agents including suspending, stabilizing and/or dispersing agents.

Additionally, the pharmaceutical compositions can also be formulated as a depot preparation. These long acting formulations can be administered by implantation (e.g. subcutaneously or intramuscularly) or by intramuscular injection. Thus, for example, the compounds may be formulated with suitable polymeric or hydrophobic materials (e.g. as an emulsion in an acceptable oil) or ion exchange resins, or as sparingly soluble derivatives, for example, as a sparingly soluble salt. Other suitable delivery systems include microspheres, which offer the possibility of local noninvasive delivery of drugs over an extended period of time. This technology can include microspheres having a precapillary size, which can be injected via a coronary catheter into any selected part of an organ without causing inflammation or ischemia. The administered therapeutic is then slowly released from the microspheres and absorbed by the surrounding cells present in the selected tissue.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts, and fusidic acid derivatives. In addition, detergents may be used to facilitate permeation. Transmucosal administration can occur using nasal sprays or suppositories. For topical administration, the AAV vector described herein can be formulated into ointments, salves, gels, or creams as generally known in the art. A wash solution can also be used locally to treat an injury or inflammation in order to accelerate healing.

Pharmaceutical forms suitable for injectable use can include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid. It must be stable under the conditions of manufacture and certain storage parameters (e.g. refrigeration and freezing) and must be preserved against the contaminating action of microorganisms, such as bacteria and fungi.

If formulations disclosed herein are used as a therapeutic to boost an immune response in a subject, a therapeutic agent can be formulated into a composition in a neutral or salt form. Pharmaceutically acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

A carrier can also be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required viral vector size in the case of dispersion and by the use of surfactants. The prevention of the action of microorganisms can be brought about by various antibacterial and antifungal agents known in the art. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions can be prepared by incorporating the active compounds or constructs in the required amount in the appropriate solvent with various of the other ingredients enumerated above, as required, followed by filtered sterilization.

Upon formulation, solutions can be administered in a manner compatible with the dosage formulation and in such amount as is therapeutically effective. The formulations are easily administered in a variety of dosage forms, such as the type of injectable solutions described above, but slow release capsules or microparticles and microspheres and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intratumorally, intramuscular, subcutaneous and intraperitoneal administration. In this context, sterile aqueous media that can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion.

The person responsible for administration will, in any event, determine the appropriate dose for the individual subject. For example, a subject may be administered AAV vectors described herein on a daily or weekly basis for a time period or on a monthly, bi-yearly or yearly basis depending on need or exposure to a pathogenic organism or to a condition in the subject (e.g. cancer).

In addition to the compounds formulated for parenteral administration, such as intravenous, intratumorally, intradermal or intramuscular injection, other pharmaceutically acceptable forms include, e.g., tablets or other solids for oral administration; liposomal formulations; time release capsules; biodegradable and any other form currently used.

One may also use intranasal or inhalable solutions or sprays, aerosols or inhalants. Nasal solutions can be aqueous solutions designed to be administered to the nasal passages in drops or sprays. Nasal solutions can be prepared so that they are similar in many respects to nasal secretions. Thus, the aqueous nasal solutions usually are isotonic and slightly buffered to maintain a pH of 5.5 to 7.5. In addition, antimicrobial preservatives, similar to those used in ophthalmic preparations, and appropriate drug stabilizers, if required, may be included in the formulation. Various commercial nasal preparations are known and can include, for example, antibiotics and antihistamines and are used for asthma prophylaxis.

Oral formulations can include excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders. In certain defined embodiments, oral pharmaceutical compositions will include an inert diluent or assimilable edible carrier, or they may be enclosed in hard or soft-shell gelatin capsule, or they may be compressed into tablets, or they may be incorporated directly with the food of the diet. For oral therapeutic administration, the active compounds may be incorporated with excipients and used in the form of ingestible tablets, buccal tablets, troches, capsules, elixirs, suspensions, syrups, wafers, and the like.

The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent methyl and propylparabens as preservatives, a dye and flavoring, such as cherry or orange flavor.

Further embodiments disclosed herein can concern kits for use with methods and compositions. Kits can also include a suitable container, for example, vials, tubes, mini- or microfuge tubes, test tube, flask, bottle, syringe or other container. Where an additional component or agent is provided, the kit can contain one or more additional containers into which this agent or component may be placed. Kits herein will also typically include a means for containing the AAV vectors and any other reagent containers in close confinement for commercial sale. Such containers may include injection or blow-molded plastic containers into which the desired vials are retained. Optionally, one or more additional active agents such as, e.g., anti-inflammatory agents, anti-viral agents, anti-fungal or anti-bacterial agents or anti-tumor agents may be needed for compositions described.

Dose ranges and frequency of administration can vary depending on the nature of the AAV vectors and the medical condition as well as parameters of a specific patient and the route of administration used. In some embodiments, AAV vector compositions can be administered to a subject at a dose ranging from about 1×10⁵ plaque forming units (pfu) to about 1×10¹⁵ pfu, depending on mode of administration, the route of administration, the nature of the disease and condition of the subject. In some cases, the AAV vector compositions can be administered at a dose ranging from about 1×10⁸ pfu to about 1×10¹⁵ pfu, or from about 1×10¹⁰ pfu to about 1×10¹⁵ pfu, or from about 1×10⁸ pfu to about 1×10¹² pfu. A more accurate dose can also depend on the subject in which it is being administered. For example, a lower dose may be required if the subject is juvenile, and a higher dose may be required if the subject is an adult human subject. In certain embodiments, a more accurate dose can depend on the weight of the subject. In certain embodiments, for example, a juvenile human subject can receive from about 1×10⁸ pfu to about 1×10¹⁰ pfu, while an adult human subject can receive a dose from about 1×10¹⁰ pfu to about 1×10¹² pfu.

Compositions disclosed herein may be administered by any means known in the art. For example, compositions may include administration to a subject intravenously, intratumorally, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intrathecally, subcutaneously, subconjunctival, intravesicularly, mucosally, intrapericardially, intraumbilically, intraocularly, orally, locally, by inhalation, by injection, by infusion, by continuous infusion, by localized perfusion, via a catheter, via a lavage, in a cream, or in a lipid composition.

Any method known to one skilled in the art maybe used for large scale production of AAV vectors, packaging cells and vector constructs described herein. For example, master and working seed stocks may be prepared under GMP conditions in qualified primary CEFs or by other methods. Packaging cells may be plated on large surface area flasks, grown to near confluence and AAV vectors purified. Cells may be harvested and AAV vectors released into the culture media isolated and purified, or intracellular AAV vectors released by mechanical disruption (cell debris can be removed by large-pore depth filtration and host cell DNA digested with endonuclease). Virus AAV vectors may be subsequently purified and concentrated by tangential-flow filtration, followed by diafiltration. The resulting concentrated bulk maybe formulated by dilution with a buffer containing stabilizers, filled into vials, and lyophilized. Compositions and formulations may be stored for later use. For use, lyophilized AAV vectors may be reconstituted by addition of diluent.

Certain additional agents used in the combination therapies can be formulated and administered by any means known in the art.

Compositions as disclosed herein can also include adjuvants such as aluminum salts and other mineral adjuvants, tensoactive agents, bacterial derivatives, vehicles and cytokines. Adjuvants can also have antagonizing immunomodulating properties. For example, adjuvants can stimulate Th1 or Th2 immunity. Compositions and methods as disclosed herein can also include adjuvant therapy.

### EXAMPLES

The following examples are provided for illustrative purposes only and are not intended to limit the scope of the invention.

### Example 1: Production of Adeno-associated Viral vectors with a heterologous epitope

AAV capsid proteins are modified to contain one of several heterologous epitopes: FLAG, c-myc, hexahistidine, etc. using PCR to generate a plasmid encoding a recombinant capsid protein. Briefly, the sequence encoding FLAG, c-myc or hexahistidine is inserted in frame after the codon encoding N587 of an AAV2 capsid protein, Q585 of an AAV6 VP1 capsid protein, N590 of an AAV8 VP1 capsid protein, A589 of an AAV9 VP1 capsid protein, or G453 of an AAV9 VP1 capsid protein.

Adeno-associated virus production is performed using a triple transfection method with HEK293 cells (see, e.g., Erik Arden and Joseph M. Metzger, J Biol Methods. 2016; 3(2)). Cells are plated one day prior to PEFpro (Polyplus transfection, New York, NY)-mediated transfection with appropriate vectors:
A helper plasmid, pHelper (Agilent, Cat #240074);
A plasmid encoding the wildtype or modified AAV rep/cap gene (pAAV RC2 (Cell biolabs, Cat# VPK-422), e.g., pAAV RC2/6/9 (Cell Biolabs, Cat# VPK-426), pAAV RC8, pAAV RC2-N587myc, pAAV RC2/6-Q585myc, pAAVRC8-N590myc, pAAV RC9-A589myc, etc.; and
A plasmid encoding a nucleotide of interest and AAV ITR sequences, e.g., pscAAV-CMV-eGFP, pAAV-CMVGFP (Agilent Cat# 240074), pAA V-EF1a-eGFP or pAAV-CAGG-eGFP, etc.

Seventy-two hours after transfection, medium is collected and cells are lysed in buffer [50mM Tris-HCl, 150mM NaCl and 0.5% Sodium Deoxycholate (Sigma, Cat# D6750-100G)]. Next, benzonase (Sigma, St. Louis, MO) is added to both medium and cell lysate to a final concentration of 0.5 U/µl before incubation at 37 °C for 60 minutes. Cell lysate is spun down at 4000rpm for 30min. Cell lysate and medium are combined together and precipitated with PEG 8000 (Teknova Cat# P4340) at a final concentration of 8%. Pellet is resuspended in 400mM NaCl and centrifuged at 10000g for 10min. Viruses in the supernatant are pelleted by ultracentrifugation at 149,000g for 3 hours and tittered by qPCR.

For qPCR to titrate AAV genomes, AAV samples are treated with DNaseI (Thermofisher Scientific, Cat #EN0525) at 37°C for one hour and lysed using DNA extract All Reagents (Thermofisher Scientific Cat# 4403319). Encapsidated viral genomes are quantified using an QuantStudio 3 Real-Time PCR System (Thermofisher Scientific) using primers directed to the AAV2 ITRs. The sequences of the AAV2 ITRs primers are 5'-GGAACCCCTAGTGATGGAGTT-3' (fwd ITR; SEQ ID NO:9) and 5'-CGGCCTCAGTGAGCGA-3' (rev ITR; SEQ ID NO:10) (Aurnhammer *et al.*, 2012), derived the left internal inverted repeat (ITR) sequence from of the AAV and the right internal inverted repeat (ITR) sequence from of the AAV, respectively. The sequence of the AAV2 ITRs probe is 5'-6-FAM-CACTCCCTCTCTGCGCGCTCG-TAMRA-3' (SEQ ID NO:11) (Aurnhammer C., Haase M., Muether N., et al., 2012, Hum. Gene Ther. Methods 23, 18-28). After a 95°C activation step for 10 min, a two-step PCR cycle is performed at 95°C for 15 seconds and 60°C for 30 seconds for 40 cycles. The TaqMan Universal PCR Master Mix (Thermofisher Scientific, Cat #4304437) was used in the qPCR. DNA plasmid (Agilent, Cat #240074) is used as standard to determine absolute titers.

Adeno-associated viral vectors comprising a capsid in which a c-myc epitope were produced. In this example, a c-myc epitope (EQKLISEEDL; SEQ ID NO:6) was inserted between amino acids N587 and R588 of the AAV2 VP1 capsid protein or between amino acids A589 and Q590 of the AAV9 VP1 capsid protein, i.e., the nucleotide sequence encoding the c-myc epitope (GAA CAA AAA CTC ATC TCA GAA GAG GAT CTG; SEQ ID NO:12) was inserted into plasmid pAAV RC2 (Cell Biolabs, Inc., San Diego, CA) or plasmid pAAV RC2/9, and each of the respective modified pAAV RC2-N587Myc pAAV RC9-A589Myc plasmids was used to encode the modified capsid protein for AAV viral vectors with a reduced or abolished tropism.

To create pAAV RC2-N587Myc, a first polymerase chain reaction (PCR) product comprising (from 5' to 3') a BsiW1 restriction site, the nucleotide sequence between positions 3050 and 3773 of pAAV RC2, and a c-myc epitope overhang nucleotide sequence, and a second PCR product comprising (from 5' to 3') a c-myc epitope overhang nucleotide sequence, the nucleotide sequence between positions 3774 to 4370 of pAAV RC2, and a Pme1 restriction site were created using the primers set forth in Table 2. The pAAV RC2-N587Myc plasmid (i.e., a pAAV RC2 plasmid modified to encode the c-myc epitope between amino acids N587 and R588 of the VP1 capsid protein) was created by digesting pAAV RC2 with BsiW1 (New England Biolabs, R0553L) and Pme1 (New England Biolabs, R0560L), and inserting the two PCR products via ligation independent cloning as described in (2012) Methods Mol. Biol. 52:51-9.

To create pAAV RC2/6-Q585Myc, a gblock DNA fragment comprising positions of 3700 and 3940 of pAAV RC2/6 with c-myc-epitope sequence inserted between 3757 and 3758 was ordered from Integrated DNA Technologies (Coralville, Iowa). pAAV RC2/6-Q585Myc plasmid was created by insertion of the gblock fragment into pAAV RC2/6 digested with MscI (New England Biolabs, Cat# R0534L) and AflII (New England Biolabs, Cat #R0520L) via ligation independent cloning as described in (2012) Methods Mol. Biol. 52:51-9.

To create pAAV RC9-A589Myc, a first polymerase chain reaction (PCR) product comprising (from 5' to 3') a BsiW1 restriction site, the nucleotide sequence between positions 3052 and 3779 of pAAV RC9, and a c-myc epitope overhang nucleotide sequence, and a second PCR product comprising (from 5' to 3') a c-myc epitope overhang nucleotide sequence, the nucleotide sequence between positions 3779 to 4404 of pAAV RC9, and a Pme1 restriction site were created using the primers set forth in Table 2.

The pAAV RC9-A589Myc plasmid (i.e., a pAAV RC2/9 plasmid modified to encode the c-myc epitope between amino acids A589 and Q590 of the VP1 capsid protein) was created by digesting pAAV RC9 with BsiW1 (New England Biolabs, R0553L) and Pme1 (New England Biolabs, R0560L), and inserting the two PCR products via ligation independent cloning as described in (2012) Methods Mol. Biol. 52:51-9.

**Table 2**

| PCR Product | Primer name | 5'-Sequence-3' and (SEQ ID NO.) |
|---|---|---|
| 3050-3773 pAAVRC2-cMyc | pAAVRC2BsiWF | GGAGTACCAGCTCCCGTACG¹ (SEQ ID NO:13) |
| | N587mycR | CTC TTC TGA GAT GAG TTT TTG TTC GTTGCCTCTCTGGAGGTTG (SEQ ID NO:14) |
| cMyc-3774-4370 pAAVRC2 | N587mycF | AAA CTC ATC TCA GAA GAG GAT CTG AGACAAGCAGCTACCGCAG (SEQ ID NO:15) |
| | pAAVRC2PmeR | TCCGCCCGCTGTTTAAAC² (SEQ ID NO:16) |
| pAAVRC9-cMyc | pAAVRC9BsiWF | ACTCAGACTATCAGCTCCCGTACG¹ (SEQ ID NO:17) |
| | A589mycR | CTCTTCTGAGATGAGTTTTTGTTCTGCTTGGGC ACTCTGGT (SEQ ID NO:18) |
| cMyc-pAAVRC9 | A589mycF | AAACTCATCTCAGAGAGGATCTGCAGGCGCA GACCGG (SEQ ID NO:19) |
| | pAAVRC9PmeR | CTCCGCCCGCTGTTTAAAC² (SEQ ID NO:20) |
| Underline sequences represent restriction enzyme recognition sites. | | |
| ¹BsiW1 | | |
| ²Pme1 | | |

pscAAV-CMV-eGFP was produced by introducing the GFP fragment into pscAAV MCS vector (Cell Biolabs, Cat# VPK-430) using BamHI and NotI restriction site. pAAV-EF1a-eGFP plasmid and pAAV-CAGG-eGFP was made de novo synthesis from Thermofisher Scientific (Waltham, MA).

### Example 2: Antibody-Mediated Retargeting of AAV viral vectors In Vitro

HepG2 is a human liver cancer cell line, which expresses liver-specific marker asialoglycoprotein receptor 1 (ASGR1). To test whether scAAV2-N587Myc-CMV-hrGFP viral vectors may infect HepG2 cells, e.g., via a bispecific antibody that recognizes both c-myc and ASGR1 (anti-myc-ASGR1 antibody), mixtures comprising different ratios of the number of viral genomes (5e9 viral genomes) to the number of antibody molecules (1:0.5, 1:1, 1:2, 1:4, 1:8, 1:15, 1:20, 1:50 or 1:100) of scAAV2-N587Myc-CMV-eGFP and bispecific anti-myc-ASGR1 antibody were incubated at room temperature for half hour, and then added to HepG2 cells. HepG2 cells were also incubated with wildtype scAAV2-CMV-eGFP viral vectors only, scAAV2-N587Myc-CMV-eGFP viral vectors only, or scAAV2-N587Myc-CMV-eGFP viral vectors with monospecific anti-myc antibody (Regeneron Pharmaceuticals, Inc., Tarrytown, NY) in a 1:8 ratio as controls. Three days after infection, GFP expression by infected HepG2 cells was confirmed by fluorescence activated cell sorting (FACS) (**Figure 1**). GFP expression was also detected in a comparable percentage of HepG2 cells when incubated with wildtype scAAV2-CMV-eGFP and when incubated with scAAV2-N587Myc-CMV-eGFP viral vectors and bispecific anti-myc-ASGR1 antibody at a ratio of 1:8 (44.6% and 44.1%, respectively, **Figures 1A** **and** **1G****, top and bottom panels**). Lower GFP expression was detected by FACS in HepG2 cells incubated with a mixture of scAAV2-N587Myc-CMV-eGFP viral vectors and higher or lower bispecific anti-myc-ASGR1 antibody (Regeneron Pharmaceuticals, Inc., Tarrytown, NY) (**Figure 1C****, bottom panel**). Additionally, GFP was not detected in HepG2 cells infected with scAAV-N587Myc-CMV-eGFP in the absence of anti-myc-ASGR1 antibody, or in HepG2 cells incubated with scAAV2-N587Myc-CMV-eGFP and monospecific anti-myc antibody.

Similarly, 293T-hASGR1 cells, which are 29T3 cells genetically modified to express human (h) ASGR1 on the cell surface, were incubated with mixtures comprising different ratios of viral genomes to the number of antibody molecules (1:0.5, 1:1, 1:2, 1:4, 1:8, 1:15, 1:20, or 1:100) of scAAV2-N587Myc-CMV-eGFP and bispecific anti-myc-ASGR1 antibody. Wildtype 293T cells (that do not express hASGR1) incubated with a mixture of scAAV2-N587Myc-CMV-eGFP and bispecific anti-myc-ASGR1 antibody at a ratio of 1:8, and 293T-hASGR1 cells incubated with (a) wildtype scAAV viral vectors only, (b) scAAV2-N587Myc-CMV-eGFP viral vectors only, or (c) scAAV2-N587Myc-CMV-eGFP viral vectors with an irrelevant bispecific anti-myc-GCGR antibody that recognizes c-myc and glucagon receptor (GCGR) (Regeneron Pharmaceuticals, Inc., Tarrytown, NY) not expressed by 293T cells at a 1:8 ratio, served as controls. Three days after infection, 293T-hASGR1 or 293T cells were stained with human anti-hASGR1antibody (Regeneron Pharmaceuticals, Inc., Tarrytown, NY) followed by APC conjugated goat anti-human antibody (Jackson ImmunoResearch laboratories Inc, Cat# 109-136-098, West Grove, PA) and GFP expression analyzed by FACS. hASGR1 expression was detected on the surface of 293T-hASGR1 cells (**Figures 2Ai-2Ax** and **2Axii**) but not 293T cells (**Figure 2Axi**). GFP positive 293T-hASGR1 cells were detected after incubation with scAAV2-N587Myc-CMV-eGFP and bispecific anti-myc-ASGR1 antibody mixtures with ratios of 1:0.5, 1:1, 1:2, 1:4, 1:8, 1:15, 1:20, and 1:50 at levels (56.9% to 68.3%) comparable to 293T-hASGR1 cells incubated with wildtype scAAV (56.7%) (**Figures 2Ai** and **2Aiii****-2Aix**). A 1:100 ratio of scAAV2-N587Myc-CMV-eGFP and bispecific anti-myc-ASGR1 antibody decreased infectivity (**Figure 2Ax**). Incubation with scAAV2-N587Myc-CMV-eGFP alone or scAAV-N587Myc with an irrelevant bispecific anti-myc-GCGR antibody did not result in GFP expression by 293T-hASGR1 cells (**Figures 2Aii** and **2Axii**).

In a similar experiment, 293T-hASGR1 cells were incubated with cells incubated with unmodified AAV9-CAGG-GFP viral vectors only, AAV9- A589Myc -CAGG-eGFP viral vectors only, or AAV9- A589Myc -CAGG-eGFP viral vectors mixed with bispecific anti-Myc-ASGR1 antibodies at different ratios. Three days after infection, GFP expression was analyzed by FACS. GFP positive 293T-hASGR1 cells were detected after incubation with AAV9-A589Myc-CAGG-eGFP and bispecific anti-myc-ASGR1 antibody mixtures with ratios of 1:1, 1:2, 1:4, 1:8, 1:20, 1:50, and 1:100 at levels (41.1% to 91%) comparable to 293T-hASGR1 cells incubated with wildtype AAV9-CAGG-eGFP (9.72%) (z and Figures2B(iii)-(ix). Incubation with AAV9-A589Myc-CAGG-eGFP alone did not result in GFP expression by 293T-hASGR1 cells (Figure 2B(ii)).

The ability of a bivalent anti-hASGR1 antibody (Regeneron Pharmaceuticals, Inc., Tarrytown, NY) to block infection of 293T-hASGR1 cells was tested. 293T-hASGR1 cells were incubated with the bivalent anti-hASGR1 antibody at different concentrations for 1 hour at room temperature, and subsequently incubated with a mixture comprising a 1:8 ratio of scAAV2-N587Myc-CMV-eGFP and bispecific anti-myc-ASGR1 antibody. Three days after infection, cells were fixed and analyzed by FACS as described above. **Figure 3** provides data showing that entry of scAAV2-N587Myc-CMV-eGFP may be blocked in a dose-dependent manner by bivalent anti-hASGR1 antibody.

To determine whether sequential administration of bispecific antibody and modified AAV could result in antibody-mediated retargeting of the modified AAV, different numbers of bispecific anti-myc-ASGR1 antibody molecules (ranging from 1×10⁹ to 1×10¹² molecules) were added to 2×10⁵ 293T-hASGR1 cells for an hour, after which 1×10⁹ scAAV-N587Myc viral vectors were added. Controls included (1) 293T-hASGR1 cells incubated with wildtype scAAV only, i.e., in the absence of any antibody, (2) 293T-hASGR1 cells sequentially incubated with 1×10¹¹ irrelevant bispecific anti-myc-GCGR antibody molecules and 1×10⁹ scAAV2-N587Myc-CMV-eGFP viral vectors, and (3) 293T cells, which do not express hASGR1, sequentially incubated with 1×10¹¹ bispecific anti-myc-ASGR1 antibody molecules and 1×10⁹ scAAV-N587Myc viral vectors. Two days after infection, GFP expression by infected 293T-hASGR1 cells was visualized by microscopy (**Figure 4**). GFP expression by 293T-hASGR1 cells was observed after incubation with wildtype scAAV only and after sequential incubation with anti-myc-ASGR1 antibody molecules at all concentrations and scAAV2-N587Myc-CMV-eGFP (Figures 4A, 4C-4I)**.** GFP was not detected in 29T3-hASGR1 cells after incubation with scAAV2-N587Myc-CMV-eGFP only (**Figure 4B**), in 293T cells that do not express ASGR1 after sequential incubation with anti-myc-hASGR1 antibody and scAAV2-N587Myc-CMV-eGFP viral vectors (**Figure 4J**), or in 29T3-hASGR1 cells after sequential incubation with irrelevant bispecific anti-myc-GCGR antibody and scAAV2-N587Myc-CMV-eGFP viral vectors (**Figure 4K**).

As described herein, the tropism of self-complementary AAV (scAAV) may be (1) inactivated, e.g., by modification of a capsid protein, e.g., by insertion of a c-myc epitope, and optionally, (2) redirected using bispecific antibodies, e.g., with a bispecific antibody that recognizes the c-myc epitope and a ligand expressed by the target cell. To determine whether the tropism of single-stranded AAV (ssAAV) may be similarly (1) reduced or abolished, and optionally, (2) redirected, 293T-hASGR1 cells were incubated with ssAAV2-N587Myc-CMV-hrGFP viral vectors produced as described in Example 1 in the absence or presence of bispecific anti-myc-hASGR1 antibody at different viral vector:antibody ratios (1:0, 1:1, 1:2, 1:4, 1:8, 1:20, 1:100 or 1:1000). Controls included 293T-hASGR1 cells incubated with wildtype ssAAV, 293T-hASGR1 cells incubated with ssAAV2-N587Myc-CMV-hrGFP viral vectors and irrelevant bispecific anti-myc-GCGR antibody at a viral vector:antibody ratio of 1:8, and 293T cells (which do not express hASGR1) incubated with ssAAV2-N587Myc-CMV-hrGFP viral vectors and bispecific anti-myc-hASGR1 antibody at a viral vector:antibody ratio of 1:8. Three days after infection, cells were fixed and GFP expression detected by FACS (**Figure 5**). GFP expression was detected in 293T-hASGR1 cells incubated with wildtype ssAAV, and with mixtures of ssAAV2-N587Myc-CMV-hrGFP viral vectors and bispecific anti-myc-ASGR1 antibodies at all ratios (**Figures 5A**, 5C-5I). GFP expression was not observed by 29T3 cells incubated with ssAAV2-N587Myc-CMV-hrGFP viral vectors and bispecific anti-myc-ASGR1 antibodies (**Figure 5J**), or by 293T-hASGR1 cells incubated with ssAAV2-N587Myc-CMV-hrGFP viral vectors only, or with ssAAV-N587Myc viral vectors and irrelevant bispecific anti-myc-GCGR bispecific antibody (**Figures 5B****,** **5K**).

Human (h) glucagon receptor (GCGR) is not normally expressed by 293T cells. However, 293T-hGCGR is a stable 293T cell line genetically modified to express hGCGR on the cell surface. To test if the retargeting of scAAV2-N587Myc-CMV-eGFP viral vectors may be mediated by bispecific anti-myc-GCGR antibody through hGCGR receptor, 293T-hGCGR cells were incubated with different mixtures comprising different ratios of scAAV2-N587Myc-CMV-eGFP:bispecific anti-myc-GCGR antibody. The scAAV2-N587Myc-CMV-eGFP viral vectors were mixed with bispecific anti-myc-GCGR antibody at ratios of 1:0.5, 1:1, 1:2, 1:4, 1:8, 1:15, 1:20, 1:50, or 1:100 at room temperature for half hour prior to addition to 293T-hGCGR cells. Three days after infection, GFP expression by infected 293T-hCGCR cells was confirmed by fluorescence activated cell sorting (FACS) (**Figure 6**). A significant percentage of GFP positive cells were detected after incubation with wildtype scAAV (**Figure 6A**) or scAAV2-N587Myc-CMV-eGFP /bispecific anti-myc-GCGR antibody at ratio of 1:0.5, 1:1, 1:2, 1:4, 1:8, 1:15, 1:20, 1:50, and 1:100 (**Figures 6C-6K**). GFP was not detected in 293T-hGCGR cells incubated with scAAV2-N587myc-CMV-eGFP viral vectors alone or scAAV2-N587Myc-CMV-eGFP viral vectors with monospecific anti-myc antibody (**Figures 6B** **and** **6L**, respectively).

Jurkat is a human acute T cell leukemia cell line, which expresses human (h) CD3. To test if the retargeting of AAV6-Q585Myc-EF1a-eGFP viral vectors may be mediated by bispecific anti-myc-CD3 antibody through hCD3 receptor, Jurkat cells were incubated with different mixtures comprising different ratios of AAV6-Q585Myc-EF1a-eGFP:bispecific anti-myc-CD3 antibody. The AAV6-Q585Myc-EF1a-eGFP viral vectors were mixed with bispecific anti-myc-CD3 antibody at ratios of 1:1, 1:5, 1:10, 1:100, 1:1000 at room temperature for half hour prior to addition to Jurkat cells. Three days after infection, GFP expression by infected Jurkat cells was confirmed by fluorescence activated cell sorting (FACS) (**Figure 7**). A significant percentage of GFP positive cells were detected after incubation with wildtype AAV6-EF1a-eGFP (**Figure 7B**) or AAV6-Q585Myc-EFla-eGFP/bispecific anti-myc-CD3 antibody at ratio of 1:1, 1:5, 1:10, and 1:100 **(****Figures 7D-7G**). GFP was not detected in Jurkat cells incubated with AAV6-Q585Myc-EF1a-eGFP viral vectors **(****Figures 7C**).

Described in this Example is the inactivation of the natural tropism of several self-complementary (sc) or single-stranded (ss) AAV serotypes demonstrated by the inability of scAAV2 or ssAAV2 genetically modified with a c-myc epitope inserted between amino acid N587 and R588 of theVP1 capsid protein (scAAV-N587myc or ssAAV-N587myc), AAV6 with a c-myc epitope inserted between Q585 and S586, and AAV9 with to infect cells normally infected by wildtype AAVs (**Figures 1A-1B**, 2A-2B, 3A-3B, 4A-4B, 5A-5B, 6A-6B, and 7). Additionally demonstrated in this Example is the ability of a bispecific antibody that recognizes the c-myc epitope and a second ligand expressed by the target cell (e.g., hASGR1, hGCGR or hCD3) to retarget the tropism of the modified AAV and mediate infection of the target cell by pseudotyped AAV in a ligand-specific manner (**Figures 1-7**). Moreover, this Example demonstrates that simultaneous administration of the genetically modified sc- or ss- AAV and bispecific antibody is not necessary for infection, i.e., sequential administration is sufficient for retargeting of the genetically modified sc- or ss- AAV *in vitro* (**Figure 4**).

### Example 3: Antibody-Mediated Redirection of modified viral vectors In Vivo

### Retargeting viral vectors to the liver with different multispecific binding molecule formats

To determine whether the bispecific anti-myc-ASGR1 antibody could retarget scAAV2-N587myc-CMV-eGFP viral vectors to liver cells expressing hASGR1 *in vivo,* mice genetically modified such that their liver cells express hASGR1 on C57BL/6 background , and control wild-type C57BL/6 mice were injected with 1×10¹¹ (titrated by qPCR) of wild-type scAAV2-CMV-eGFP alone or scAAV2-N587myc-CMV-eGFP viral vectors in combination with bispecific anti-myc-ASGR1 antibody and at 1:8 ratio of viral genome to antibody molecules intravascularly. Controls included mice injected with saline [250mM NaCl] or with scAAV2-N587myc-CMV-eGFP viral vector alone. Ten days post injection, mice were sacrificed and transcardial perfused with 4% PFA. Organs of livers, kidney and heart were collected and dehydrated in 15% sucrose followed by 30% sucrose. Then organs were cryo-sectioned on slides and stained with chicken anti-EGFP antibody (Jackson ImmunoResearch Labs, Inc. West Grove, PA) and Alexa-488 conjugated anti-chicken secondary antibody (Jackson ImmunoResearch Labs, Inc. West Grove, PA). (Figures 8A-8C). GFP positive cells were detected in livers from those transgenic animals modified to express ASGR1 in the liver and injected with wildtype scAAV2-CMV-eGFP or scAAV2-N587myc-CMV-eGFP in combination with bispecific anti-myc-ASGR1 antibody (Figures 8A(i) and 8A(iv)), and in livers from wildtype C57BL/6 mice injected with wildtype scAAV2-CMV-eGFP (Figure 8A(v)). GFP was not detected in any spleen or kidney samples (Figures 8B and 8C), nor in liver, spleen or kidney samples from any animal injected with saline or scAAV2-N587myc-CMV-eGFP viral vectors alone (Figures8A(ii, iii, vi, vii)), nor in liver samples taken from wildtype C57BL/6 animals injected with scAAV2-N587myc-CMV-eGFP in combination with bispecific anti-myc-ASGR1 antibody (Figure 8A(viii)). In summary, the combination of scAAV2-N587myc-CMV-eGFP viral vectors and bispecific anti-myc-ASGR1 antibody infected only those (liver) cells expressing hASGR1, strongly suggesting that the scAAV2-CMV-eGFP viral vector was inactivated by the modification of the capsid protein, e.g., natural tropism of the scAAV viral vector could be reduced to abolished, e.g., with a c-myc epitope, and such viral vectors could be specifically reactivated, e.g., specifically retargeted, e.g., to liver cells *in vivo,* e.g., by bispecific anti-myc-ASGR1 antibodies.

Similarly, to determine whether the bispecific anti-myc-ASGR1 antibody could retarget ssAAV2-N587myc-CAGG-eGFP viral vectors to liver cells expressing hASGR1 *in vivo,* mice genetically modified such that their liver cells express hASGR1 on C57BL/6 background , and control wild-type C57BL/6 mice were injected with 2.18×10¹¹ (titrated by qPCR) of wild-type ssAAV2-CAGG-eGFP alone or ssAAV2-N587myc-CAGG-eGFP viral vectors in combination with bispecific anti-myc-ASGR1 antibody and at 1:4 ratio of viral genome to antibody molecules intravascularly. Controls included mice injected with PBS or with ssAAV2-N587myc-CAGG-eGFP viral vector alone. Four weeks post injection, mice were sacrificed and transcardial perfused with 4% PFA. Organs of livers, kidney and heart were collected and dehydrated in 15% sucrose followed by 30% sucrose. Then organs were cryo-sectioned on slides and stained with chicken anti-EGFP antibody (Jackson ImmunoResearch Labs, Inc. West Grove, PA) and Alexa-488 conjugated anti-chicken secondary antibody (Jackson ImmunoResearch Labs, Inc. West Grove, PA). GFP positive cells were detected in livers from those transgenic animals modified to express ASGR1 in the liver and injected with wildtype ssAAV2-CAGG-eGFP or ssAAV2-N587myc-CAGG-eGFP in combination with bispecific anti-myc-ASGR1antibody (**Figures 9E-9F**, 9P-9R), and in livers from wildtype C57BL/6 mice injected with wildtype ssAAV2-CAGG-eGFP (**Figure 9B-9C**). Surprisingly, the infection efficiency of ssAAV2-N587myc-CAGG-eGFP in combination with bispecific anti-myc-ASGR1 antibody is much higher than WT ssAAV2-CAGG-GFP (**Figures 9E-9F**, 9P-9R). GFP was not detected or barely detected in in liver samples from any animal injected with saline or ssAAV2-N587myc-CAGG-eGFP viral vectors alone (**Figures 9A, 9D**, 9G-9L)), nor in liver samples taken from wildtype C57BL/6 animals injected with ssAAV2-N587myc-CAGG-eGFP in combination with bispecific anti-myc-ASGR1 antibody (**Figure 9M-9O**). In summary, the combination of ssAAV2-N587myc-CAGG-eGFP viral vectors and bispecific anti-myc-ASGR1 antibody infected only those (liver) cells expressing hASGR1, strongly suggesting that the ssAAV2-N587myc-CAGG-eGFP viral vector was inactivated by the modification of the capsid protein, e.g., natural tropism of the scAAV viral vector could be reduced to abolished, e.g., with a c-myc epitope, and such viral vectors could be specifically reactivated, e.g., specifically retargeted, e.g., to liver cells *in vivo,* e.g., by bispecific anti-myc-ASGR1 antibodies.

Further experiments with a pseudotyped AAV9 viral vector was performed with mice genetically modified such that their liver cells express hASGR1 on C57BL/6 background. These mice were injected with 1×10¹¹ (titrated by qPCR) of wild-type AAV9-CAGG-eGFP or AAV9-A589myc-CAGG-eGFP viral particles in combination with bispecific anti-myc-ASGR1 antibody at 1:100 ratio of viral genome to antibody molecules intravascularly. Controls included mice injected with PBS or with AAV9-N587myc-CAGG-eGFP viral particles in combination with irrelevant bispecific anti-myc-hCD3 antibody. Four weeks post injection, mice were sacrificed. Livers were fixed with 10% formalin and sent to HistoWiz. Inc (New York, NY) for GFP staining. GFP positive cells were detected in livers from those animals injected with wild-type AAV9-CAGG-eGFP or AAV9-N587myc-CAGG-eGFP viral particles in combination with bispecific anti-myc-ASGR1 antibody (**Figures 10A and 10D**). GFP was not detected or barely detected in in liver samples from any animal injected with saline or with AAV9-N587myc-CAGG-eGFP viral particles in combination with bispecific anti-myc-hCD3 antibody, respectively (**Figures 10B and 10C**). In summary, similarly to AAV2, the natural tropism of AAV9 may be reduced to abolished by the modification of the capsid protein, e.g., by insertion of a c-myc epitope, and that such modified viral vector, e.g., AAV9-A589myc, may be retargeted to specific cell types using corresponding bispecific anti-epitope-cell specific marker binding protein, e.g, bispecifc anti-myc-ASGR1 antibody to liver cells.

### Anti-myc scFc-Appended to anti-hASGR1 IgG4 knobs-into-hole Fc format

To determine whether various multispecific binding molecule formats could mediate the infection AAV, anti-myc scFv was fused to the C-terminus of one chain of a knobs-into-holes anti-hASGR1 IgG4 antibody (see Figure 11A). Specifically, a gblock encoding an anti-myc scFv (SEQ ID NO:28) flanked by homologous arms to 1459-1478 and 1479-1498 of pRG7078 encoding an anti-hASGR1 stealth knob IgG4 heavy chain was provided by Integrated DNA technologies (Skokie, Illinois). The nucleotide sequences (NA) encoding and the amino acid (AA) sequences of HCVR, LCVR, HCDR1, HCDR2, HCDR3, LCDR1, LCDR2, and LCDR3 of the scFv (SEQ ID NO:37) encoded by SEQ ID NO:28 are provided in Table 3.

**Table 3: SEQ ID NOs associated with the scFv set forth as SEQ ID NO:37**

| | scFv | HCVR | LCVR | HCDR1 | HCDR2 | HCDR3 | LCDR1 | LCDR2 | LCDR3 |
|---|---|---|---|---|---|---|---|---|---|
| AA | 37 | 29 | 30 | 31 | 32 | 33 | 34 | 35 | 36 |
| NA | 38 | 39 | 40 | | | | | | |

The gblock was cloned into pRG7078 encoding an anti-hASGR1 stealth knob IgG4 heavy chain to make a pRG7078 encoding an anti-hASGR1 stealth knob IgG4 anti-myc scFv fusion polypeptide "anti-hASGR1-IgG4-Fc/anti-myc bispecific." The pRG7078 plasmid modified with c-myc, unmodified pRG7078 antihASGR1 IgG4 stealth hole star and a plasmid containing antibody light chain, were transfected into 293T cells, cultured with OPTI-MEM (Cat#31985070, Thermo Fisher). Four days after transfection, medium was collected and the anti-hASGR1-IgG4-Fc/anti-myc bispecific binding molecule was purified using protein A column (cat # 89948, Thermo Fisher).

To create a pseudotyped AAV8 viral vector, pAAV RC8 N590myc, a gblock with a c-myc epitope (SEQ ID NO:22) inserted between the N590 and T591 of a VP1 capsid protein of AAV8 was ordered from Integrated DNA technologies (Skokie, Illinois). pAAV RC8 plasmid (SEQ ID NO:23) was digested with Mlu1 and Sbf1 enzymes and the gblock was cloned into the vector using SLIC to make pAAV RC8 N590myc (SEQ ID NO:24).

293T-hASGR1 cells, which are 293T cells genetically modified to express human (h) ASGR1 on the cell surface, were incubated with either mixtures comprising different ratios of AAV8-N590Myc-CAGG-eGFP to anti-hASGR1-IgG4-Fc/anti-myc bispecific binding molecule molecules (1:0, 1:1, 1:2, 1:4, 1:8, 1:12, 1:15, 1:50, or 1:100) or AAV8-CAGG-eGFP viral genomes. Three days after infection, GFP expression was analyzed by FACS. GFP positive 293T-hASGR1 cells were detected after incubation with mixtures of AAV8-N590Myc-CAGG-eGFP and anti-hASGRI-IgG4-Fc/anti-myc bispecific molecules at ratios of 1:1, 1:2, 1:4, 1:8, 1:12, 1:15, 1:50, or 1:100 (2.12% to 22.2%; **Figures 12D-12K**) comparable to 293T-hASGR1 cells incubated with wildtype AAV8-CAGG-eGFP (46.1%) **(****Figure 12A**). Neither mock-infected cells (Figure 12B) nor incubation with AAV8-N590Myc-CAGG-eGFP alone resulted in GFP expression by 293T hASGR1 cells (Figure 12C).

To determine whether anti-hASGRI-IgG4-Fc/anti-myc bispecific binding proteins could retarget AAV8 N590myc-CAGG-eGFP viral particles to liver cells expressing hASGR1 *in vivo,* mice genetically modified such that their liver cells express hASGR1 on C57BL/6 background were injected intravascularly with 1×10¹¹ (titrated by qPCR) of wild-type AAV8-CAGG-eGFP or AAV8 N590myc-CAGG-eGFP viral particles in combination with anti-hASGR1-IgG4-Fc/anti-myc bispecific binding molecules at a 1:12 ratio of viral genome to binding protein molecules. Control animals were injected with AAV8 N590myc-CAGG-eGFP viral particles in combination with an irrelevant anti-hCD3xanti-myc bispecific binding molecule having a similar format as the anti-hASGR1-IgG4-Fc/anti-myc bispecific binding molecule. Three weeks post injection, mice were sacrificed and transcardial perfused with 4% PFA. Organs of livers, kidney and heart were collected and dehydrated in 15% sucrose followed by 30% sucrose. Then organs were cryo-sectioned on slides and stained with chicken anti-EGFP antibody (Jackson ImmunoResearch Labs, Inc. West Grove, PA) and Alexa 488 conjugated anti-chicken secondary antibody (Jackson ImmunoResearch Labs, Inc. West Grove, PA). GFP positive cells were detected in livers from those animals injected with wildtype AAV8-CAGG-eGFP (**Figures 13A-13C**) or AAV8 N590myc-CAGG-eGFP viral particles in combination with the anti-hASGRI-IgG4-Fc/anti-myc bispecific binding molecules (**Figures 13G-13I**). GFP was not detected or barely detected in in liver samples from any animal injected with AAV8 N590myc-CAGG-eGFP viral particles in combination with the irrelevant anti-hCD3/myc binding protein (**Figures 13D-13F**). In summary, epitope tagged AAV8, e.g., AAV8-N590myc, can be retargeted to a specific cell type using a bispecific binding molecule that specifically binds the epitope and a cell specific marker expressed by the targeted cell type.

### Retargeting viral vectors to the intestine and/or pancreas

293T cells genetically modified to express human ectonucleoside triphosphate diphosphohydrolase 3 (hENTPD3) on the cell surface ("293T-ENTPD3") were incubated with mixtures comprising different ratios of AAV2-N587Myc-CAGG-eGFP or AAV2-CAGG-GFP viral genomes and bispecific binding molecule formed by fusing anti-myc scFv to the C-terminus of one chain of a knobs-into-holes anti-hENTPD3 IgG4 antibody (anti-hENTPD3-IgG4-Fc/anti-myc) at different ratios (1:0, 1:1, 1:2, 1:4, 1:8, 1:20, 1:50, 1:100 or 1:200). Three days after infection, GFP expression was analyzed by FACS. GFP positive 293T-ENTPD3 cells were detected after incubation with mixtures of AAV2-N587Myc-CAGG-eGFP and anti-hENTPD3-IgG4-Fc/anti-myc at levels (1.48% to 16%; **Figures 14C-14J**) comparable to 293T-ENTPD3 cells incubated with wildtype AAV2-CAGG-eGFP (66%) (**Figure 14A**). Incubation with AAV2-N587Myc-CAGG-eGFP alone results in very low level of GFP expression by 293T-ENTPD3 cells (**Figure 14B**).

It was determined that ENTPD3 is expressed in the mucosa of the intestine (data not shown). To determine whether the anti-hENTPD3-IgG4-Fc/anti-myc binding protein could retarget AAV2-N587myc-CAGG-eGFP viral particles to intestinal cells expressing ENTPD3 *in vivo*, C57BL/6 mice were injected intravascularly with 5×10¹¹ (titrated by qPCR) AAV2-N587myc-CAGG-eGFP viral particles in combination with anti-hENTPD3-IgG4-Fc/anti-myc binding proteins at a 1:20 ratio of viral genome to binding protein molecules. Control mice were injected with PBS, wildtype AAV9, or with AAV2-N587myc-CAGG-eGFP viral particles in combination with an irrelevant binding protein molecule. Three weeks post injection, mice were sacrificed. Livers, intestines, and pancreas were fixed with 10% formalin and sent to HistoWiz. Inc (New York, NY) for GFP staining. GFP positive cells were detected in mice injected with wildtype AAV9 (Figure 15B(ii)), but not in livers from mice injected with PBS or AAV2-N587myc-CAGG-eGFP viral particles in combination with irrelevant binding molecules or anti-hENTPD3-IgG4-Fc/anti-myc binding proteins (Figure 15A(iii)-15A(iv), respectively), indicating that AAV2-N587myc viral vectors do not infect ENTPD3-negative liver cells even when co-injected with hENTPD3-IgG4-Fc/anti-myc binding proteins. GFP was detected in intestines of only mice injected with wildtype AAV9 or AAV2-N587myc-CAGG-eGFP viral particles in combination with hENTPD3-IgG4-Fc/anti-myc binding proteins (Figures 15B(i)-15B(iv)). GFP is detected in the pancreatic islets of a mouse injected with AAV2-N587myc-CAGG-eGFP viral particles in combination with anti-hENTPD3-IgG4-Fc/anti-myc binding proteins. (Figure 15C(iv)). WT AAV9 infected both islet cells and non-islets cell of pancreas (Figure 15C(ii)). GFP was not detected in pancreas samples from mice injected with saline or AAV2-N587myc-CAGG-eGFP viral particles in combination with irrelevant binding proteins. (Figures 7C(i) and 7C(iii)). In summary, the natural tropism of AAV can be reduced to abolished by inserting a heterologous epitope and retargeted to the same or other cells using multispecific binding protein that binds the epitope tag and a marker expressed by the retargeted cells or tissues.

This example demonstrates that several different serotypes of adeno-associated viral vector may be genetically modified with a heterologous epitope (e.g., c-myc) as described herein to inactivate infectivity, and a bispecific antibody (regardless of bispecific formats) that recognizes both the heterologous epitope (e.g., c-myc) and a marker expressed by a targeted cell may be used to retarget the viral vector to deliver a nucleotide of interest.

### Example 4: Use of Genetically modified AAV-N587Myc viral vectors to Deliver Therapeutic Cargo to Specific Cells

This example demonstrates the ability of AAV-N587Myc viral vectors to specifically deliver therapeutic cargo, such as one or more suicide genes, a biological therapeutic (e.g., antibody), a CRISPR/Cas gene editing system, shRNA, etc., to a targeted cell type. Specifically, this example describes the delivery of a suicide gene, antibody encoding sequence, or a CRISPR/Cas gene editing system to cells expressing a targeted ligand.

### Delivery of a suicide gene to cells expressing a targeted ligand

To test the ability of scAAV2-N587Myc viral vectors to deliver a suicide gene to a specific cell, a xenograft nude mouse model of HER2⁺ breast cancer as described by Wang et al. ((2010) Cancer Gene Therapy 17:559-570) is used.

*Viral vectors:* scAAV2-N587Myc or ssAAV2-N587Myc viral vectors carrying a reporter EGFP gene (scAAV2-N587Myc-EGFP or ssAAV2-N587MycEGFP) are generated as described in Example 1. scAAV2-N587Myc or ssAAV2-N587Myc viral vectors carrying a suicide gene (SG) are similarly generated. Briefly, 293T17 cells are transfected with (1) pAd Helper and (2) pAAV RC2 (encoding a wild-type capsid) or pAAV RC2-N587Myc vectors (encoding a capsid modified with a c-myc epitope) as described above, and with (3) a pAAV vector carrying a suicide gene, e.g., cytosine deaminase gene, herpes simplex virus thymidine kinase gene, under the control of a promoter, e.g., CMV. ssAAV-N587MycSG or scAAV-N587SG viral vectors are isolated and titrated as described in Example 1.

*Cell Lines:* BT474 breast cancer, SK-BR-3 breast cancer and Calu-3 lung cancer cell lines are HER2 positive human tumor cell lines (Bunn P.A. et al., (2001) Clin Cancer Res. 7:3239-3250; Pegram M, et al. (1999) Oncogene 18:2241-2251; Spiridon CI, et al., (2002) Clin Cancer Res. 8:1720-1730). A-673 rhabdomyosarcoma and HeLa cervical cancer are HER2 negative human tumor cell lines and BEAS-2b is an immortalized bronchial epithelial HER2 negative cell line (Jia LT et al. (2003) Cancer Res. 63:3257-3262; Kern JA, et al., (1993) Am J Respir Cell Mol Biol 9:448-454; Martinez-Ramirez A, et al., (2003) Cancer Genet Cytogenet. 2003; 141:138-142). All of these cell lines are obtained from American Type Culture Collection (ATCC, Manassas, VA) and maintained in medium recommended by the ATCC.

*Mice:* Female nude mice, 6 to 8 weeks of age are obtained and housed under specific pathogen-free conditions. On day 0, mice are simultaneously (1) injected subcutaneously in the right flank with 10⁷ BT474, SK-BR-3, Calu-3, A-673 or HeLa tumor cells and (2) treated intravenously with bispecific anti-myc-HER2 antibody and ss- or scAAV-N587Myc viral vectors carrying a reporter (e.g., EGFP) or suicide gene. Serving as controls are untreated animals (animals injected with tumor cells only), animals injected with wildtype ss- or scAAV viral vectors carrying a reporter or suicide gene, animals injected with bispecific anti-myc-HER2 antibody only, or animals injected with ss- or scAAV-N587Myc viral vectors carrying a reporter or suicide gene only. All animals are treated with an appropriate pro-drug 1 day after injection and treatment. The size of each tumor is measured using calipers 2 times weekly, and tumor volume is calculated as length×width²×0.52. Upon morbidity, tumor ulceration, a tumor diameter of 15 mm, or tumor volume of 1000 mm³, the mice are sacrificed, and the sacrifice date recorded as the date of death. Livers, spleens, kidneys and tumors of animals injected with wild-type ss- or scAAV or ss- or scAAV-N587Myc virus viral vectors carrying a reporter gene are fixed and reporter gene expression visualized.
Although the targeted delivery of suicide inducing genes has been described (Zarogoulidis P., et al. (2013) J. Genet. Syndr. Gene Ther. 4:16849), this example describes the delivery of a suicide gene to a cell expressing the targeted HER2 ligand using the viral vectors described herein comprising a heterologous epitope, e.g., c-myc, and a bispecific antibody that specifically binds the targeted ligand and the heterologous epitope. In additional experiments, a suicide gene is delivered to another cell type expressing one or more other target ligands using a viral vector comprising a heterologous epitope as described herein and a bispecific antibody that specifically binds the heterologous epitope (e.g., c-myc) and the target receptor. Exemplary and non-limiting examples of receptors suitable for targeting include those receptors that mediate endocytosis of the viral vector, e.g., carcino-embryonic antigen (CEA) (Qiu Y, et al. (2012) Cancer Lett. 316:31-38) and vascular endothelial growth factor receptor (VEGFR) (Leng A, et al. (2013) Tumour Biol. 32:1103-1111; Liu T, et al. (2011) Exp Mol Pathol. 91:745-752). Additional receptors that may be targeted include: epidermal growth factor receptor (EGFR) (Heimberger AB, et al. (2009) Expert Opin Biol Ther. 9:1087-1098), cluster of differentiation 44s (CD44s) (Heider KH, et al. (2004) Cancer Immunol Immunother. 53:567-579), cluster of differentiation 133 (CD133 aka AC133) (Zhang SS, et al. (2012) BMC Med. ;10:85), folate receptor (FR) (Duarte S, et al., (2011) J Control Release 149(3):264-72), transferrin receptor (TfR) or cluster differentiation 71 (CD71) (Habashy HO, et al., Breast Cancer Res Treat. 119(2):283-93), mucins (Torres MP, et al., (2012) Curr Pharm Des. 2012; 18(17):2472-81), stage specific embryonic antigen 4 (SSEA-4) (Malecki M., et al., (2012) J Stem Cell Res Ther. 2(5)), tumor resistance antigen 1-60 (TRA-1-60) (Malecki M., et al., (2013) J Stem Cell Res Ther. 3:134).

## Claims

1. A composition comprising
(i) a recombinant adeno-associated virus (AAV) vector comprising
an AAV capsid that encapsulates a nucleotide of interest,
wherein the AAV capsid comprises a recombinant AAV capsid protein modified to comprise a heterologous epitope, wherein the recombinant AAV capsid protein is derived from an AAV capsid gene modified to express the heterologous epitope;
(ii) a multispecific binding molecule comprising
(a) an antibody paratope that specifically binds the heterologous epitope; and
(b) a retargeting ligand that specifically binds a cell surface protein or marker; and optionally
(iii) a pharmaceutically acceptable carrier,
wherein the AAV capsid exhibits:
- reduced to abolished tropism in the absence of the multispecific binding molecule; and
- modified tropism, upon combination with the multispecific binding molecule, compared to a reference AAV capsid, wherein the reference AAV capsid comprises a reference AAV capsid protein that is identical to the recombinant AAV capsid protein but for the lack of the heterologous epitope.

2. The composition of claim 1, wherein the recombinant AAV capsid protein further comprises a mutation in addition to the heterologous epitope.

3. The composition of claim 1 or 2, wherein the heterologous epitope is inserted such that the insertion partially reduces the tropism of the AAV capsid compared to a reference AAV capsid lacking the heterologous epitope and the AAV capsid protein further comprises a mutation (e.g. a substitution, deletion, insertion other than the insertion of the heterologous epitope), in addition to the insertion of the heterologous epitope, with the mutation reducing further and/or abolishing the tropism of the recombinant AAV capsid compared to a reference AAV capsid lacking the mutation.

4. The composition of any one of claims 1 to 3, wherein the modified tropism upon combination with the multispecific binding molecule means that the tropism of the AAV capsid is restored and redirected compared to the tropism of the reference AAV capsid.

5. The composition of any one of the preceding claims, wherein
(i) the heterologous epitope comprises an affinity tag and an optional linker, and/or
(ii) the recombinant AAV capsid protein comprises an amino acid sequence EQKLISEEDL (set forth as SEQ ID NO: 6) flanked by and/or operably linked to at least 5 contiguous amino acids of an AAV capsid protein.

6. The composition of any one of the preceding claims, wherein the heterologous epitope comprises an amino acid sequence EQKLISEEDL (SEQ ID NO:6) or a portion thereof, and wherein the antibody paratope specifically binds the amino acid sequence EQKLISEEDL or a portion thereof.

7. The composition of claim 1, wherein the nucleotide of interest is:
(i) under the control of a promoter selected from the group consisting of a viral promoter, a bacterial promoter, a mammalian promoter, an avian promoter, a fish promoter, an insect promoter, and any combination thereof; optionally wherein the nucleotide of interest is under the control of a non-human promoter; or
(ii) under the control of a non-human promoter and flanked by AAV ITR sequences; or
(iii) a reporter gene, optionally wherein the reporter gene encodes green fluorescent protein, or is selected from the group consisting of a suicide gene, a nucleotide encoding an antibody or fragment thereof, a nucleotide encoding a CRISPR/Cas system or portion(s) thereof, a nucleotide encoding antisense RNA, a nucleotide encoding siRNA, and a combination thereof.

8. The composition of any one of the preceding claims, wherein the multispecific binding molecule is a bispecific binding molecule.

9. The composition of claim 1, wherein the antibody paratope is an Fv domain, optionally wherein the Fv domain is directly fused to a heavy chain constant domain.

10. The composition of claim 1, wherein the retargeting ligand is an antibody or portion thereof, optionally:
wherein the antibody is a bispecific antibody, wherein the antibody paratope and the retargeting ligand each comprise a distinct Fv domain fused to a first and second heavy chain constant domain, optionally wherein the first and second heavy chains bind to Protein A with differential binding affinities or
wherein the retargeting ligand comprises a tetrameric antibody structure comprising two identical immunoglobulin heavy chains and two identical light chains and wherein the antibody paratope that binds the heterologous epitope is appended to the C-terminus or N-terminus of one or both heavy chains and/or to the C-terminus or N-terminus of one or both heavy chains, and optionally wherein the antibody paratope is an scFv, optionally wherein the scFv comprises an amino acid sequence set forth as SEQ ID NO:37.

11. The composition of any one of the preceding claims, wherein the cell surface protein is a receptor; and/or wherein the retargeting ligand binds a receptor expressed on the surface of a mammalian or human cell.

12. The composition of any one of the preceding claims, wherein the AAV is selected from the group consisting of AAV1, AAV2, AAV3, AAV4, AAV5, AAV6, AAV7, AAV8 and AAV9 and/or wherein the heterologous epitope is inserted after and/or replaces a position selected from the group consisting of I-453 of AAV2, I-587 of AAV2, I-585 of AAV6, I-590 of AAV8, I-453 of AAV9, I-589 of AAV9, and any corresponding amino acids of an AAV serotype that infects primates.

13. The composition of any one of the preceding claims, wherein the AAV capsid is a mosaic capsid which comprises a reference AAV capsid protein of the same serotype that does not comprise the heterologous epitope at a certain ratio.

14. The composition of any one of the preceding claims, wherein the recombinant AAV capsid protein is derived from a chimeric AAV capsid gene modified to express the heterologous epitope, wherein the chimeric AAV capsid gene comprises a plurality of nucleic acid sequences, wherein each of the plurality of nucleic acid sequences encodes a portion of a capsid protein of a different AAV serotype, and wherein the plurality of nucleic acid sequences together encodes a chimeric AAV capsid protein.

15. The composition of any one of the preceding claims for use in an *in vivo* method of treatment or diagnosis, the method comprising delivering the nucleotide of interest to a target cell expressing a cell surface protein comprising contacting the target cell with the composition, wherein the multispecific binding molecule comprises a retargeting ligand that binds the cell surface protein.

16. The composition for use according to claim 15, wherein:
(a) the delivery is to a human subject; or
(b) the target cell is a human cell; or
(c) the target cell is a human neuronal cell; or
(d) the target cell is a human muscle cell; or
(e) the target cell is selected from the group consisting of a liver cell, a brain cell, a T cell, a kidney cell, an intestinal cell, a pancreas cell, a cancerous cell, and a cell infected with heterologous pathogen.

17. The composition for use according to claim 15 or claim 16 wherein:
(a) the target cell is a human liver cell optionally wherein the retargeting ligand binds human asialoglycoprotein receptor 1 (hASGR1) expressed by the human liver cell; or
(b) the retargeting ligand binds GABA receptor expressed by the human neuronal cell; or
(c) the retargeting ligand binds transferrin receptor expressed by the human neuronal cell;
(d) the target cell is a human T cell, optionally wherein the retargeting ligand binds CD3 expressed by the human T cell; or
(e) the target cell is a human T cell, optionally wherein the retargeting ligand binds CD3ε expressed by the human T cell; or
(f) the target cell is a human hematopoietic cell, optionally wherein the retargeting ligand binds CD34; or
(g) the target cell is a human cancer cell, optionally wherein the retargeting ligand binds a tumor associated antigen; or
(h) the target cell is a human cancer cell, optionally wherein the retargeting ligand binds a tumor associated antigen which is E6, E7 or Her2; or
(i) the cell surface protein is human glucagon receptor (hGCGR); or
(j) the target cell is an intestinal cell or is a pancreas cell; optionally wherein the receptor is ENTPD3.

18. The composition for use in the method of claim 15 or 16, wherein:
(i) the retargeting ligand binds CD20; or
(ii) the retargeting ligand binds human glucagon receptor; or
(iii) the retargeting ligand specifically binds CD63 or human ectonucleoside triphosphate diphosphohydrolase 3 (hENTPD3).

19. The composition of claim 5(i) wherein the affinity tag comprises c-myc (EQKLISEEDL; SEQ ID NO:6).

20. The composition of claim 6 wherein the antibody paratope specifically binds c-myc, which antibody paratope comprises an HCVR, LCVR, HCDR1, HCDR2, HCDR3, LCDR1, LCDR2 and/or LCDR3 sequence of the scFv encoded by the nucleic acid sequence set forth as SEQ ID NO:28.

21. An *in vitro* or *ex vivo* method of delivering a nucleotide of interest to a target cell expressing a cell surface protein comprising contacting the target cell with the composition of any one of claims 1 to 14, wherein the multispecific binding molecule comprises a retargeting ligand that binds the cell surface protein.

## Patentansprüche

1. Zusammensetzung, umfassend
(i) einen rekombinanten Adeno-assoziierten Virus (AAV) -Vektor, umfassend
ein AAV-Kapsid, das ein Nukleotid von Interesse ummantelt,
wobei das AAV-Kapsid ein rekombinantes AAV-Kapsid-Protein umfasst, das modifiziert ist, um ein heterologes Epitop zu umfassen, wobei das rekombinante AAV-Kapsid-Protein aus einem AAV-Kapsid-Gen abgeleitet ist, das modifiziert ist, um das heterologe Epitop zu exprimieren;
(ii) ein multispezifisches Bindemolekül, umfassend
(a) ein Antikörper-Paratop, das das heterologe Epitop spezifisch bindet; und
(b) einen Retargeting-Liganden, der ein Zelloberflächenprotein oder einen Marker spezifisch bindet; und optional
(iii) einen pharmazeutisch annehmbaren Träger,
wobei das AAV-Kapsid Folgendes aufweist:
- verringerten oder beseitigten Tropismus in Abwesenheit des multispezifischen Bindemoleküls; und
- modifizierten Tropismus bei Kombination mit dem multispezifischen Bindemolekül im Vergleich zu einem Referenz-AAV-Kapsid, wobei das Referenz-AAV-Kapsid ein Referenz-AAV-Kapsid-Protein umfasst, das identisch zu dem rekombinanten AAV-Kapsid-Protein ist, außer dem fehlenden heterologen Epitop.

2. Zusammensetzung nach Anspruch 1, wobei das rekombinante AAV-Kapsid-Protein ferner eine Mutation zusätzlich zu dem heterologen Epitop umfasst.

3. Zusammensetzung nach Anspruch 1 oder 2, wobei das heterologe Epitop eingefügt wird, sodass das Einfügen teilweise den Tropismus des AAV-Kapsids verringert im Vergleich zu einem Referenz-AAV-Kapsid, dem das heterologe Epitop fehlt, und das AAV-Kapsid ferner eine Mutation (z. B. eine Substitution, Deletion, Einfügung außer der Einfügung des heterologen Epitops) umfasst, zusätzlich zu der Einfügung des heterologen Epitops, wobei die Mutation den Tropismus des rekombinanten AAV-Kapsids ferner verringert und/oder beseitigt im Vergleich zu einem Referenz-AAV-Kapsid, dem die Mutation fehlt.

4. Zusammensetzung nach einem der Ansprüche 1 bis 3, wobei der modifizierte Tropismus bei Kombination mit dem multispezifischen Bindemolekül bedeutet, dass der Tropismus des AAV-Kapsids im Vergleich zu dem Tropismus des Referenz-AAV-Kapsids wiederhergestellt und umgeleitet wird.

5. Zusammensetzung nach einem der vorhergehenden Ansprüche, wobei
(i) das heterologe Epitop ein Affinitäts-Tag und einen optionalen Linker umfasst, und/oder
(ii) das rekombinante AAV-Kapsid-Protein eine Aminosäuresequenz EQKLISEEDL (dargelegt als SEQ ID NO: 6), flankiert von und/oder operabel verknüpft mit mindestens 5 zusammenhängenden Aminosäuren eines AAV-Kapsid-Proteins.

6. Zusammensetzung nach einem der vorhergehenden Ansprüche, wobei das heterologe Epitop eine Aminosäuresequenz EQKLISEEDL (SEQ ID NO: 6) oder einen Abschnitt davon umfasst, und wobei das Antikörper-Paratop die Aminosäuresequenz EQKLISEEDL oder einen Abschnitt davon spezifisch bindet.

7. Zusammensetzung nach Anspruch 1, wobei das Nukleotid von Interesse Folgendes ist:
(i) unter der Kontrolle eines Promotors, ausgewählt aus der Gruppe bestehend aus einem viralen Promotor, einem bakteriellen Promotor, einem Säugetier-Promotor, einem Vogel-Promotor, einem Fisch-Promotor, einem Insekten-Promotor und jeder Kombination davon; wobei optional das Nukleotid von Interesse unter der Kontrolle eines nicht-menschlichen Promotors steht; oder
(ii) unter der Kontrolle eines nicht-menschlichen Promotors und flankiert von AAV-ITR-Sequenzen; oder
(iii) ein Reportergen, wobei optional das Reportergen grün fluoreszierendes Protein kodiert oder ausgewählt ist aus der Gruppe bestehend aus einem Suizidgen, einem Nukleotid, das einen Antikörper oder ein Fragment davon kodiert, einem Nukleotid, das CRISPR/Cas-System oder Abschnitt(e) davon kodiert, einem Nukleotid, das Antisense-RNA kodiert, einem Nukleotid, das siRNA kodiert und einer Kombination davon.

8. Zusammensetzung nach einem der vorhergehenden Ansprüche, wobei das multispezifische Bindemolekül ein bispezifisches Bindemolekül ist.

9. Zusammensetzung nach Anspruch 1, wobei das Antikörper-Paratop eine Fv-Domäne ist, wobei optional die Fv-Domäne direkt an eine konstante Domäne der schweren Kette fusioniert ist.

10. Zusammensetzung nach Anspruch 1, wobei der Retargeting-Ligand ein Antikörper oder ein Abschnitt davon ist, optional:
wobei der Antikörper ein bispezifischer Antikörper ist, wobei das Antikörper-Paratop und der Retargeting-Ligand jeweils eine unterschiedliche Fv-Domäne umfassen, die an eine erste und zweite konstante Domäne der schweren Kette fusioniert ist, wobei optional die erste und zweite schwere Kette an Protein A mit differentiellen Bindungsaffinitäten binden, oder
wobei der Retargeting-Ligand eine tetramere Antikörperstruktur umfasst, umfassend zwei identische schwere Immunglobulinketten und zwei identische leichte Ketten, und wobei das Antikörper-Paratop, das das heterologe Epitop bindet, an den C-Terminus oder N-Terminus von einer oder beiden schweren Ketten und/oder an den C-Terminus oder N-Terminus von einer oder beiden schweren Ketten angehängt ist, und wobei optional das Antikörper-Paratop ein scFv ist, wobei optional das scFv eine Aminosäuresequenz umfasst, die als SEQ ID NO: 37 dargelegt ist.

11. Zusammensetzung nach einem der vorhergehenden Ansprüche, wobei das Zelloberflächenprotein ein Rezeptor ist; und/oder wobei der Retargeting-Ligand einen Rezeptor, der an der Oberfläche einer Säugetier- oder menschlichen Zelle exprimiert ist, bindet.

12. Zusammensetzung nach einem der vorhergehenden Ansprüche, wobei das AAV ausgewählt ist aus der Gruppe bestehend aus AAV1, AAV2, AAV3, AAV4, AAV5, AAV6, AAV7, AAV8 und AAV9 und/oder wobei das heterologe Epitop nach einer Position eingefügt ist und/oder diese ersetzt, ausgewählt aus der Gruppe bestehend aus 1-453 von AAV2, I-587 von AAV2, I-585 von AAV6, I-590 von AAV8, I-453 von AAV9, I-589 von AAV9 und jeglichen entsprechenden Aminosäuren eines AAV-Serotyps, der Primaten infiziert.

13. Zusammensetzung nach einem der vorhergehenden Ansprüche, wobei das AAV-Kapsid ein Mosaik-Kapsid ist, das ein Referenz-AAV-Kapsid-Protein desselben Serotyps umfasst, das das heterologe Epitop in einem bestimmten Verhältnis nicht umfasst.

14. Zusammensetzung nach einem der vorhergehenden Ansprüche, wobei das rekombinante AAV-Kapsid-Protein aus einem chimären AAV-Kapsid-Gen abgeleitet ist, das modifiziert ist, um das heterologe Epitop zu exprimieren, wobei das chimäre AAV-Kapsid-Gen eine Vielzahl von Nukleinsäuresequenzen umfasst, wobei jede der Vielzahl von Nukleinsäuresequenzen einen Abschnitt eines Kapsid-Proteins eines unterschiedlichen AAV-Serotyps kodiert, und wobei die Vielzahl von Nukleinsäuresequenzen zusammen ein chimäres AAV-Kapsid-Protein kodiert.

15. Zusammensetzung nach einem der vorhergehenden Ansprüche zur Verwendung in einem In-vivo-Verfahren der Behandlung oder Diagnose, wobei das Verfahren das Zuführen des Nukleotids von Interesse zu einer Zielzelle umfasst, die ein Zelloberflächenprotein exprimiert, umfassend das Kontaktieren der Zielzelle mit der Zusammensetzung, wobei das multispezifische Bindungsmolekül einen Retargeting-Liganden umfasst, der das Zelloberflächenprotein bindet.

16. Zusammensetzung zur Verwendung nach Anspruch 15, wobei:
(a) das Zuführen an ein menschliches Subjekt erfolgt; oder
(b) die Zielzelle eine menschliche Zelle ist; oder
(c) die Zielzelle eine menschliche neuronale Zelle ist; oder
(d) die Zielzelle eine menschliche Muskelzelle ist; oder
(e) die Zielzelle ausgewählt ist aus der Gruppe bestehend aus einer Leberzelle, einer Gehirnzelle, einer T-Zelle, einer Nierenzelle, einer Darmzelle, einer Bauchspeicheldrüsenzelle, einer Krebszelle und einer Zelle, die mit heterologem Pathogen infiziert ist.

17. Zusammensetzung zur Verwendung nach Anspruch 15 oder Anspruch 16, wobei:
(a) die Zielzelle eine menschliche Leberzelle ist, wobei optional der Retargeting-Ligand menschlichen Asialoglycoproteinrezeptor 1 (hASGR1) bindet, exprimiert durch die menschliche Leberzelle; oder
(b) der Retargeting-Ligand GABA-Rezeptor bindet, exprimiert durch die menschliche neuronale Zelle; oder
(c) der Retargeting-Ligand Transferrin-Rezeptor bindet, exprimiert durch die menschliche neuronale Zelle;
(d) die Zielzelle eine menschliche T-Zelle ist, wobei optional der Retargeting-Ligand CD3 bindet, exprimiert durch die menschliche T-Zelle; oder
(e) die Zielzelle eine menschliche T-Zelle ist, wobei optional der Retargeting-Ligand CD3ε bindet, exprimiert durch die menschliche T-Zelle; oder
(f) die Zielzelle eine menschliche hämatopoetische Zelle ist, wobei optional der Retargeting-Ligand CD34 bindet; oder
(g) die Zielzelle eine menschliche Krebszelle ist, wobei optional der Retargeting-Ligand ein Tumor-assoziiertes Antigen bindet; oder
(h) die Zielzelle eine menschliche Krebszelle ist, wobei optional der Retargeting-Ligand ein Tumor-assoziiertes Antigen bindet, das E6, E7 oder Her2 ist; oder
(i) das Zelloberflächenprotein menschlicher Glukagonrezeptor (hGCGR) ist; oder
(j) die Zielzelle eine Darmzelle oder eine Bauchspeicheldrüsenzelle ist; wobei optional der Rezeptor ENTPD3 ist.

18. Zusammensetzung zur Verwendung in dem Verfahren nach Anspruch 15 oder 16, wobei:
(i) der Retargeting-Ligand CD20 bindet; oder
(ii) der Retargeting-Ligand menschlichen Glukagonrezeptor bindet; oder
(iii) der Retargeting-Ligand CD63 oder menschliche Ektonukleosidtriphosphatdiphosphohydrolase 3 (hENTPD3) spezifisch bindet.

19. Zusammensetzung nach Anspruch 5(i), wobei der Affinitäts-Tag c-myc (EQKLISEEDL; SEQ ID NO: 6) umfasst.

20. Zusammensetzung nach Anspruch 6, wobei das Antikörper-Paratop c-myc spezifisch bindet, wobei das Antikörper-Paratop eine HCVR-, LCVR-, HCDR1-, HCDR2-, HCDR3-, LCDR1-, LCDR2- und/oder LCDR3-Sequenz des scFv umfasst, kodiert durch die Nukleinsäuresequenz, die als SEQ ID NO: 28 dargelegt ist.

21. *In-vitro-* oder *Ex-vivo*-Verfahren des Zuführens eines Nukleotids von Interesse zu einer Zielzelle, die ein Zelloberflächenprotein exprimiert, umfassend das Kontaktieren der Zielzelle mit der Zusammensetzung nach einem der Ansprüche 1 bis 14, wobei das multispezifische Bindemolekül einen Retargeting-Liganden umfasst, der das Zelloberflächenprotein bindet.

## Revendications

1. Composition comprenant
(i) un vecteur de virus adéno-associé (AAV) recombinant comprenant
une capside AAV qui encapsule un nucléotide d'intérêt,
dans laquelle la capside AAV comprend une protéine de capside AAV recombinante modifiée pour comprendre un épitope hétérologue, dans laquelle la protéine de capside AAV recombinante est dérivée d'un gène de capside AAV modifié pour exprimer l'épitope hétérologue ;
(ii) une molécule de liaison multispécifique comprenant
(a) un paratope d'anticorps qui se lie spécifiquement à l'épitope hétérologue ; et
(b) un ligand de reciblage qui se lie spécifiquement à une protéine ou un marqueur de surface cellulaire ; et éventuellement
(iii) un support pharmaceutiquement acceptable,
dans laquelle la capside AAV présente :
- un tropisme réduit à aboli en l'absence de la molécule de liaison multispécifique ; et
- un tropisme modifié, lors d'une combinaison avec la molécule de liaison multispécifique, en comparaison avec une capside AAV de référence, dans laquelle la capside AAV de référence comprend une protéine de capside AAV de référence qui est identique à la protéine de capside AAV recombinante, à l'exception de l'absence de l'épitope hétérologue.

2. Composition selon la revendication 1, dans laquelle la protéine de capside AAV recombinante comprend en outre une mutation en plus de l'épitope hétérologue.

3. Composition selon la revendication 1 ou 2, dans laquelle l'épitope hétérologue est inséré de telle sorte que l'insertion réduit partiellement le tropisme de la capside AAV en comparaison avec une capside AAV de référence dépourvue de l'épitope hétérologue et la protéine de capside AAV comprend en outre une mutation (par exemple, une substitution, une délétion, une insertion autre que l'insertion de l'épitope hétérologue), en plus de l'insertion de l'épitope hétérologue, la mutation réduisant davantage et/ou abolissant le tropisme de la capside AAV recombinante en comparaison avec une capside AAV de référence dépourvue de la mutation.

4. Composition selon l'une quelconque des revendications 1 à 3, dans laquelle le tropisme modifié lors d'une combinaison avec la molécule de liaison multispécifique signifie que le tropisme de la capside AAV est restauré et réorienté en comparaison avec le tropisme de la capside AAV de référence.

5. Composition selon l'une quelconque des revendications précédentes, dans laquelle
(i) l'épitope hétérologue comprend une étiquette d'affinité et un lieur éventuel, et/ou
(ii) la protéine de capside AAV recombinante comprend une séquence d'acides aminés EQKLISEEDL (présentée comme SEQ ID N° : 6) flanquée par au moins 5 acides aminés contigus d'une protéine de capside AAV et/ou liée fonctionnellement à ceux-ci.

6. Composition selon l'une quelconque des revendications précédentes, dans laquelle l'épitope hétérologue comprend une séquence d'acides aminés EQKLISEEDL (SEQ ID N° : 6) ou une portion de celle-ci, et dans laquelle le paratope d'anticorps se lie spécifiquement à la séquence d'acides aminés EQKLISEEDL ou à une portion de celle-ci.

7. Composition selon la revendication 1, dans laquelle le nucléotide d'intérêt est :
(i) sous la commande d'un promoteur sélectionné dans le groupe constitué d'un promoteur viral, d'un promoteur bactérien, d'un promoteur mammifère, d'un promoteur aviaire, d'un promoteur de poisson, d'un promoteur d'insecte, et de toute combinaison de ceux-ci ; éventuellement dans laquelle le nucléotide d'intérêt est sous la commande d'un promoteur non humain ; ou
(ii) sous la commande d'un promoteur non humain et flanqué par des séquences ITR d'AAV ; ou
(iii) un gène rapporteur, éventuellement dans laquelle le gène rapporteur code pour la protéine fluorescente verte, ou est sélectionné dans le groupe constitué d'un gène suicide, d'un nucléotide codant pour un anticorps ou un fragment de celui-ci, d'un nucléotide codant pour un système CRISPR/Cas ou une(des) portion(s) de celui-ci, d'un nucléotide codant pour un ARN antisens, d'un nucléotide codant pour un ARNsi, et d'une combinaison de ceux-ci.

8. Composition selon l'une quelconque des revendications précédentes, dans laquelle la molécule de liaison multispécifique est une molécule de liaison bispécifique.

9. Composition selon la revendication 1, dans laquelle le paratope d'anticorps est un domaine Fv, éventuellement dans laquelle le domaine Fv est directement fusionné à un domaine constant de chaîne lourde.

10. Composition selon la revendication 1, dans laquelle le ligand de reciblage est un anticorps ou une portion de celui-ci, éventuellement :
dans laquelle l'anticorps est un anticorps bispécifique, dans lequel le paratope d'anticorps et le ligand de reciblage comprennent chacun un domaine Fv distinct fusionné à un premier et un deuxième domaine constant de chaîne lourde, éventuellement dans laquelle les première et deuxième chaînes lourdes se lient à la protéine A avec des affinités de liaison différentielles ou
dans laquelle le ligand de reciblage comprend une structure d'anticorps tétramère comprenant deux chaînes lourdes d'immunoglobuline identiques et deux chaînes légères identiques et dans laquelle le paratope d'anticorps qui se lie à l'épitope hétérologue est annexé à l'extrémité C-terminale ou à l'extrémité N-terminale de l'une ou des deux chaînes lourdes et/ou à l'extrémité C-terminale ou à l'extrémité N-terminale de l'une ou des deux chaînes lourdes, et éventuellement dans laquelle le paratope d'anticorps est un scFv, éventuellement dans laquelle le scFv comprend une séquence d'acides aminés présentée comme SEQ ID N° : 37.

11. Composition selon l'une quelconque des revendications précédentes, dans laquelle la protéine de surface cellulaire est un récepteur ; et/ou dans laquelle le ligand de reciblage se lie à un récepteur exprimé sur la surface d'une cellule mammifère ou humaine.

12. Composition selon l'une quelconque des revendications précédentes, dans laquelle l'AAV est sélectionné dans le groupe constitué de AAV1, AAV2, AAV3, AAV4, AAV5, AAV6, AAV7, AAV8 et AAV9 et/ou dans laquelle l'épitope hétérologue est inséré après et/ou remplace une position sélectionnée dans le groupe constitué de 1-453 de AAV2, 1-587 de AAV2, 1-585 de AAV6, 1-590 de AAV8, 1-453 de AAV9, 1-589 de AAV9, et tout acide aminé correspondant d'un sérotype d'AAV qui infecte les primates.

13. Composition selon l'une des revendications précédentes, dans laquelle la capside AAV est une capside mosaïque qui comprend une protéine de capside AAV de référence du même sérotype qui ne comprend pas l'épitope hétérologue dans un certain rapport.

14. Composition selon l'une quelconque des revendications précédentes, dans laquelle la protéine de capside AAV recombinante est dérivée d'un gène de capside AAV chimérique modifié pour exprimer l'épitope hétérologue, dans laquelle le gène de capside AAV chimérique comprend une pluralité de séquences d'acides nucléiques, dans laquelle chacune de la pluralité de séquences d'acides nucléiques code pour une portion d'une protéine de capside d'un sérotype AAV différent, et dans laquelle la pluralité de séquences d'acides nucléiques code ensemble pour une protéine de capside AAV chimérique.

15. Composition selon l'une quelconque des revendications précédentes pour une utilisation dans un procédé de traitement ou de diagnostic *in vivo,* le procédé comprenant l'administration du nucléotide d'intérêt à une cellule cible exprimant une protéine de surface cellulaire comprenant la mise en contact de la cellule cible avec la composition, dans laquelle la molécule de liaison multispécifique comprend un ligand de reciblage qui se lie à la protéine de surface cellulaire.

16. Composition pour une utilisation selon la revendication 15, dans laquelle :
(a) l'administration se fait à un sujet humain ; ou
(b) la cellule cible est une cellule humaine ; ou
(c) la cellule cible est une cellule neuronale humaine ; ou
(d) la cellule cible est une cellule musculaire humaine ; ou
(e) la cellule cible est sélectionnée dans le groupe constitué d'une cellule hépatique, d'une cellule cérébrale, d'une cellule T, d'une cellule rénale, d'une cellule intestinale, d'une cellule pancréatique, d'une cellule cancéreuse, et d'une cellule infectée par un agent pathogène hétérologue.

17. Composition pour une utilisation selon la revendication 15 ou la revendication 16 dans laquelle :
(a) la cellule cible est une cellule hépatique humaine, éventuellement dans laquelle le ligand de reciblage se lie au récepteur 1 d'asialoglycoprotéine humaine (hASGR1) exprimé par la cellule hépatique humaine ; ou
(b) le ligand de reciblage se lie au récepteur GABA exprimé par la cellule neuronale humaine ; ou
(c) le ligand de reciblage se lie au récepteur transferrine exprimé par la cellule neuronale humaine ;
(d) la cellule cible est une cellule T humaine, éventuellement dans laquelle le ligand de reciblage se lie au CD3 exprimé par la cellule T humaine ; ou
(e) la cellule cible est une cellule T humaine, éventuellement dans laquelle le ligand de reciblage se lie au CD3ε exprimé par la cellule T humaine ; ou
(f) la cellule cible est une cellule hématopoïétique humaine, éventuellement dans laquelle le ligand de reciblage se lie au CD34 ; ou
(g) la cellule cible est une cellule cancéreuse humaine, éventuellement dans laquelle le ligand de reciblage se lie à un antigène associé à une tumeur ; ou
(h) la cellule cible est une cellule cancéreuse humaine, éventuellement dans laquelle le ligand de reciblage se lie à un antigène associé à une tumeur qui est E6, E7 ou Her2 ; ou
(i) la protéine de surface cellulaire est un récepteur de glucagon humain (hGCGR) ; ou
(j) la cellule cible est une cellule intestinale ou une cellule pancréatique ; éventuellement dans laquelle le récepteur est ENTPD3.

18. Composition pour une utilisation dans le procédé de la revendication 15 ou 16, dans laquelle :
(i) le ligand de reciblage se lie au CD20 ; ou
(ii) le ligand de reciblage se lie au récepteur glucagon humain ; ou
(iii) le ligand de reciblage se lie spécifiquement au CD63 ou à l'ectonucléoside triphosphate diphosphohydrolase 3 humaine (hENTPD3).

19. Composition selon la revendication 5(i) dans laquelle l'étiquette d'affinité comprend c-myc (EQKLISEEDL ; SEQ ID N° : 6).

20. Composition selon la revendication 6 dans laquelle le paratope d'anticorps se lie spécifiquement à c-myc, lequel paratope d'anticorps comprend une séquence HCVR, LCVR, HCDR1, HCDR2, HCDR3, LCDR1, LCDR2 et/ou LCDR3 du scFv codé par la séquence d'acide nucléique présentée comme SEQ ID N° : 28.

21. Procédé *in vitro* ou *ex vivo* pour administrer un nucléotide d'intérêt à une cellule cible exprimant une protéine de surface cellulaire comprenant la mise en contact de la cellule cible avec la composition de l'une quelconque des revendications 1 à 14, dans lequel la molécule de liaison multispécifique comprend un ligand de reciblage qui se lie à la protéine de surface cellulaire.
